(19) Europäisches Patentamt
European Patent Office
Office européen des brevets

(11) EP 0 741 139 A1

(12) EUROPÄISCHE PATENTANMELDUNG

(43) Veröffentlichungstag: **06.11.1996 Patentblatt 1996/45**

(51) Int. Cl.[6]: **C07H 13/08**, C07H 13/10, C07H 15/18, A61K 31/70

(21) Anmeldenummer: **96106537.2**

(22) Anmeldetag: **25.04.1996**

(84) Benannte Vertragsstaaten:
**AT BE CH DE DK ES FI FR GB GR IE IT LI LU NL PT SE**

(30) Priorität: **05.05.1995 CH 1311/95**

(71) Anmelder: **F. HOFFMANN-LA ROCHE AG**
**4070 Basel (CH)**

(72) Erfinder:
- **Chucholowski, Alexander**
  **79639 Grenzach-Wyhlen (DE)**
- **Pech, Michael**
  **79258 Hartheim (DE)**
- **Fingerle, Jürgen**
  **79618 Rheinfelden (DE)**
- **Rouge, Marianne**
  **4058 Basel (CH)**
- **Iberg, Niggi**
  **4059 basel (CH)**
- **Schmid, Gérard**
  **4468 Kienberg (CH)**
- **Märki, Hans Peter**
  **4059 Basel (CH)**
- **Tschopp, Thomas**
  **4107 Ettingen (CH)**
- **Müller, Rita**
  **4051 Basel (CH)**
- **Wessel, Hans Peter**
  **79423 Heitersheim (DE)**

(74) Vertreter: **Grossner, Lutz, Dr. et al**
**F.Hoffmann-La Roche AG**
**Patent Department (PLP),**
**124 Grenzacherstrasse**
**4070 Basel (CH)**

(54) **Neue Schwefelsäureester von Aminozuckern**

(57) Die vorliegende Erfindung betrifft neue Schwefelsäureester von Aminozuckern der allgemeinen Formel Ia, Ib, Ic

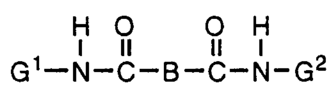

Ia

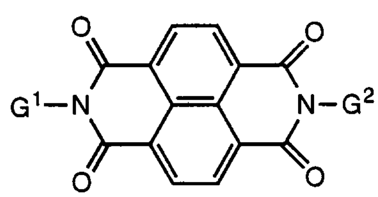

Ib

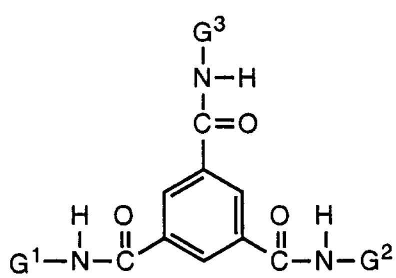

Ic

worin

EP 0 741 139 A1

B niederes Alkylen oder ein gegebenenfalls substituiertes aromatisches Ringsystem;
$G^1$, $G^2$ und $G^3$ unabhängig voneinander je einen Rest eines Glycopyranosids, einer Glycopyranose oder eines Derivates davon, wobei mindestens eine Hydroxygruppe der Reste $G^1$, $G^2$ oder $G^3$ mit Schwefelsäure verestert ist, bedeuten und pharmazeutisch anwendbare Salze davon.

Die Verbindungen eignen sich als therapeutische Wirkstoffe zur Behandlung von Krankheiten, die durch exzessive oder destruktive Proliferation von glatten Muskelzellen gekennzeichnet ist.

**Beschreibung**

Die vorliegende Erfindung betrifft neue Schwefelsäureester von Aminozuckern der allgemeinen Formel Ia, Ib, Ic

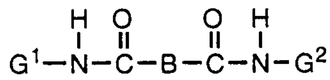

Ia

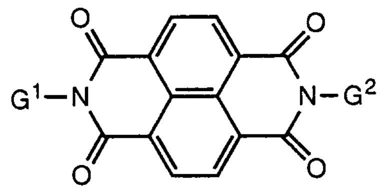

Ib

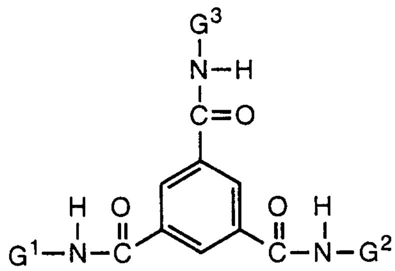

Ic

worin

B niederes Alkylen oder ein gegebenenfalls substituiertes aromatisches Ringsystem;
$G^1$, $G^2$ und $G^3$ unabhängig voneinander je einen Rest eines Glycopyranosids, einer Glycopyranose oder eines Derivates davon, wobei mindestens eine Hydroxygruppe der Reste $G^1$, $G^2$ oder $G^3$ mit Schwefelsäure verestert ist, bedeuten und pharmazeutisch anwendbare Salze davon.

Die Erfindung betrifft weiterhin pharmazeutische Präparate, enthaltend eine Verbindung der allgemeinen Formel Ia-Ic oder Salze davon; die Verwendung der Verbindungen der allgemeinen Formel Ia-Ic und deren Salze als Heilmittel, insbesondere zur Behandlung und/oder Prophylaxe von Krankheiten, die durch exzessive oder destruktive Proliferation von glatten Muskelzellen gekennzeichnet sind, sowie von arteriosklerotischen Gefässwandveränderungen, z.B. zur Verhinderung von Restenose nach koronarer oder peripherer Angioplastie oder nach Bypass-Operationen bzw. zur Herstellung von Heilmitteln für die genannten Indikationen; sowie ein Verfahren zur Herstellung der Verbindungen der allgemeinen Formel Ia-Ic und deren Salze.

Gemeinsames Merkmal der erfindungsgemässen Verbindungen ist, dass sie zwei bzw. drei von einer Glycopyranose abgeleitete Reste haben, die über eine Amid-bzw. Imidbrücke mit einem Zentralelement verknüpft sind.

Vorzugsweise sind die Verbindungen der allgemeinen Formel Ia-Ic symmetrische Verbindungen, wobei die Reste $G^1$· $G^2$ und, falls vorhanden, $G^3$ identisch sind.

Beispiele für das aromatische Ringsystem in Formel Ia sind Phenylen, Naphthylen oder ein Rest der Formel

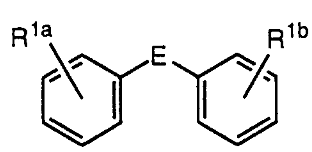

worin

E eine Kohlenstoff-Kohlenstoff-Bindung, -O-,-CO, $-CH_2-$, $-CH_2CH_2-$, -CH=CH-, -C≡C- und $R^{1a}$ und $R^{1b}$ Wasserstoff oder Halogen bedeuten.

Bevorzugte aromatische Ringsysteme in Formel Ia sind Phenylen, Biphenylen, Naphthylen, Stilbenylen oder Biphenylenmethyl.

Beispiele für Glycopyranosid- oder Glycopyranose Reste oder Derivate davon ( $G^1$, $G^2$, $G^3$ ) in Formel Ia, Ib, Ic sind Reste der Formel a-f

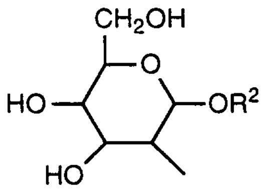
a)

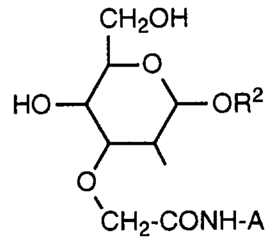
b)

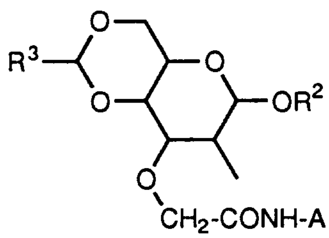
c)

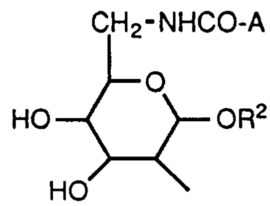
d)

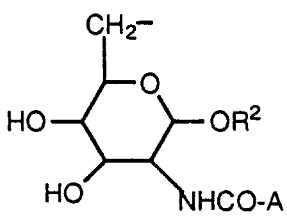
e)

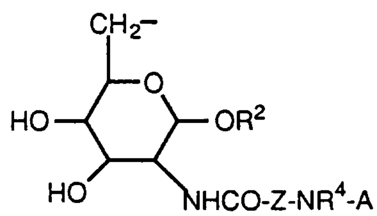
f)

wobei mindestens je eine Hydroxygruppe der Glycopyranose - oder Glycopyranosidreste a-f mit Schwefelsäure verestert ist, und
worin

$R^2$ Wasserstoff, niederes Alkyl oder Benzyl;
$R^3$ Wasserstoff, niederes Alkyl oder Phenyl;
$R^4$ Wasserstoff oder gegebenenfalls substituiertes niederes Alkyl;
Z einen gegebenenfalls substituierten Phenylenrest;
A den um die 1-Hydroxygruppe verminderten Rest eines Zuckeralkohols oder eines Derivats davon, den tris-(Hydroxymethyl)-methylrest, einen Glycopyranosid- oder Glycopyranose-Rest der oben angegebenen Formel a oder einen Rest der Formel g oder h bedeuten,

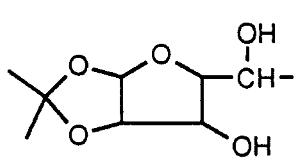
g)

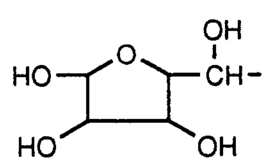
h)

wobei mindestens je eine Hydroxygruppe des Restes A mit Schwefelsäure verestert ist.

Der Rest $R^2$ ist vorzugsweise der Benzylrest.

Wenn der durch $R^4$ bezeichnete Rest substituiert ist, so ist er zweckmässigerweise durch eine OH-Gruppe substituiert, die mit Schwefelsäure verestert sein kann.

Substituenten des mit Z bezeichneten Restes sind zweckmässigerweise die Nitro-Gruppe oder die Acetylaminogruppe.

Beispiele von Zuckeralkoholen, von denen sich der Rest A ableitet, sind Hexitole, wie Glucitol, Galaktitol, Mannitol und Gulitol ; Pentitole, wie Arabinitol, Ribitol und Xylitol, Tetritole wie Threitol und Erythritol oder der Glycerolrest. Derivate solcher Zuckeralkohole können einfach oder mehrfach desoxygenierte Zuckeralkohole sein.

Diese Zuckeralkohole können in der D- oder L-Form oder als Razemate vorliegen, wobei die natürlich vorkommende Form bzw. die Form, die dem zugrundeliegenden, natürlich vorkommenden Zucker entspricht, bevorzugt ist.

Die Ausdrücke "niederes Alkyl" und "niederes Alkylen" umfassen geradkettige oder verzweigte gesättigte Koblenwasserstoffreste mit bis zu 7, vorzugsweise bis zu 4 Kohlenstoffatomen, wie Methyl, Ethyl, n-Propyl, Isopropyl, n-Butyl, Isobutyl, tert.Butyl u.dgl.,bzw. Methylen, Ethylen und dgl.

Der Ausdruck "Halogen" umfasst Fluor, Chlor, Brom und Jod, wovon Chlor bevorzugt ist.

Phenylenreste sind 1,3- oder 1,4-Phenylenreste.

Naphthylenreste sind 1,4- oder 2,6-Naphthylenreste.

Beispiele von Salzen von Verbindungen der allgemeinen Formel Ia-Ic sind Alkalimetallsalze, wie Na- oder K-Salze, Ammoniumsalze und Salze von tertiären Aminen, wie Triethylamin, oder Pyridinium- oder Imidazoliumsalze, oder quaternäre Ammoniumsalze, wie Dodecyltrimethylammonium-, Ethylpyridinium- und Benzethoniumsalze; sowie Erdalkalimetallsalze, wie Ca- oder Mg-Salze.

Bevorzugte Verbindungen der allgemeinen Formel Ia-Ic sind:

(Biphenyl-4,4'-dicarbonsäure)-bis-[[benzyl-3-O-[(benzyl-2-desoxy-2,3,4-tri-O-sulfo-a-D-glucopyranosid)-2-ylcarbamoylmethyl]-4,6-(R)-O-benzyliden-2-desoxy-a-D-glucopyranosid-2-yl]-amid] Hexanatriumsalz ( Beispiel 11 ).

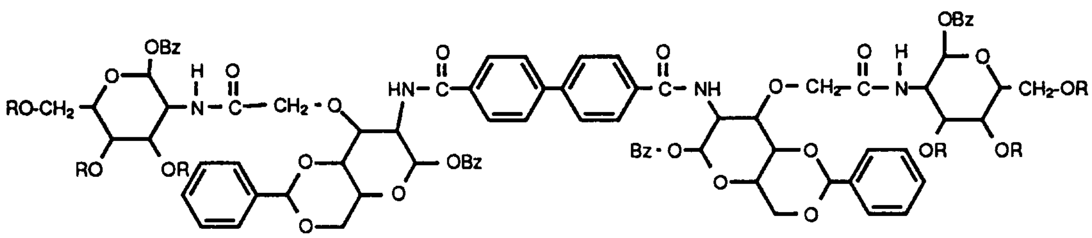

Bz= Benzyl R=$SO_3Na$

Biphenyl-4,4'-dicarbonsäure-bis-[[benzyl-2,6-didesoxy-3,4-di-O-sulfo-6-(2,3,4,5,6-penta-O-sulfo-D-gluconoylamino)-a-D-glucopyranosid-2-yl]-amid] Tetradekanatriumsalz ( Beispiel 14 ).

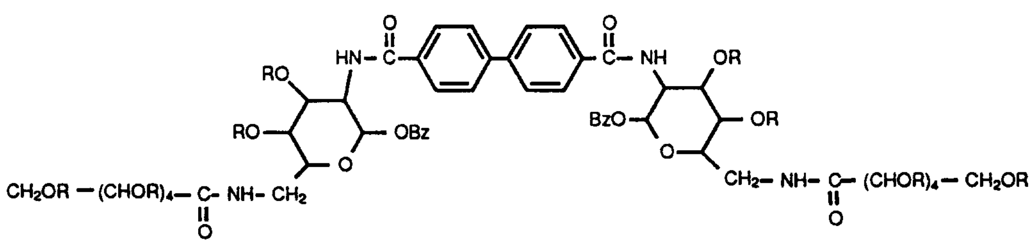

Bz= Benzyl R=$SO_3Na$

Biphenyl-4,4'-dicarbonsäure-bis-[[(Z)-benzyl-2,6-didesoxy-3,4-di-O-sulfo-6-(3-desoxy-2,4,5,6-tetra-O-sulfo-D-erythro-hex-2-enonoylamino)-a-D-glucopyranosid-2-yl]-amid] Dodekanatriumsalz ( Beispiel 15 ).

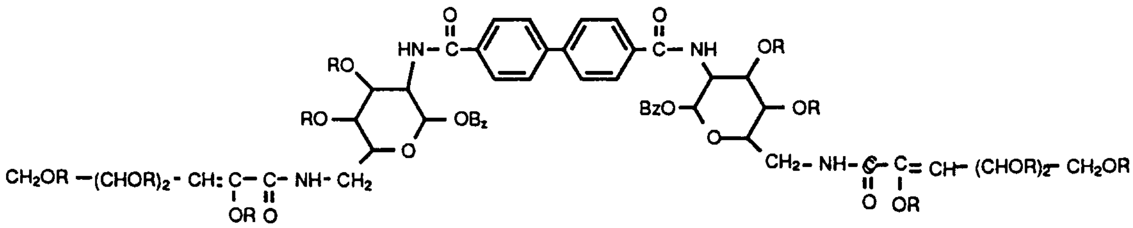

Bz= Benzyl R=$SO_3Na$

(Z)-Stilben-4,4'-dicarbonsäure-bis-[[benzyl-2,6-didesoxy-6-(2,3,4,5,6-penta-O-sulfo-D-gluconoylamino)-3,4-di-O-sulfo-a-D-glucopyranosid-2-yl]-amid] Tetradekanatriumsalz (Beispiel 23).

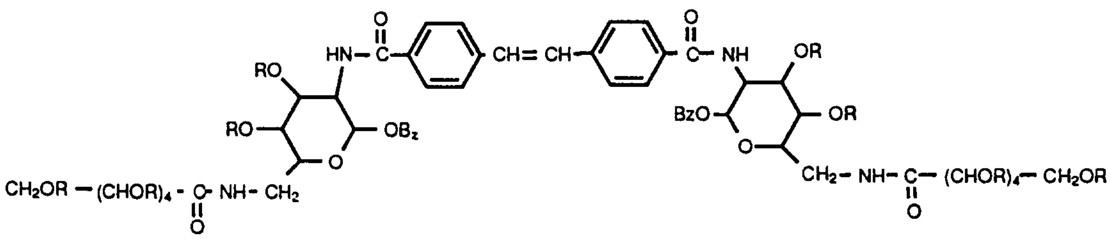

Bz= Benzyl R=$SO_3Na$

Isophthalsäure-bis-[[benzyl-2,6-didesoxy-6-(2,3,4,5,6-penta-O-sulfo-D-gluconoylamino)-3,4-di-O-sulfo-a-D-glucopyranosid-2-yl]-amid] Tetradekanatriumsalz ( Beispiel 24 ).

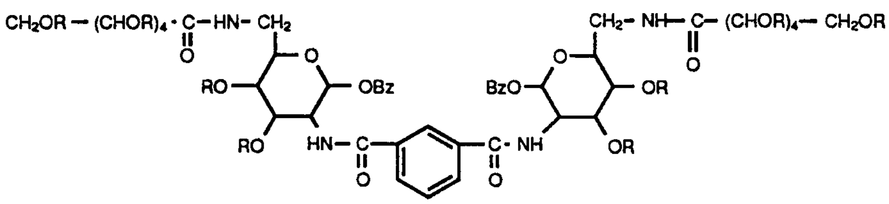

Bz= Benzyl R=$SO_3Na$

Benzol-1,3,5-tricarbonsäure-tris-[[benzyl-2,6-didesoxy-2-[4-(2,3,4,5,6-penta-O-sulfo-D-glucit-1-ylamino)-3-nitrobenzoylamino]-3,4-di-O-sulfo-a-D-glucopyranosid-6-yl]-amid] Henikosanatriumsalz ( Beispiel 30).

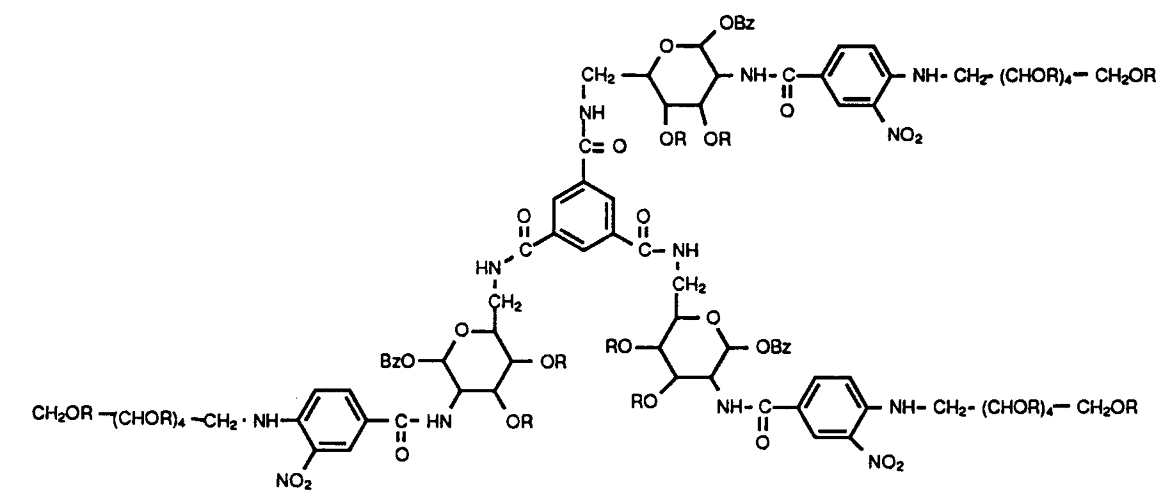

Bz= Benzyl R=$SO_3Na$

(E)-2-Chior-stilben-4,4'-dicarbonsäure-bis-[[benzyl-2,6-didesoxy-2-[4-(2,3,4,5,6-penta-O-sulfo-D-glucit-1-ylamino)-3-nitro-benzoylamino]-3,4-di-O-sulfo-a-D-glucopyranosid-6-yl]-amid] Tetradekanatriumsalz ( Beispiel 32 ).

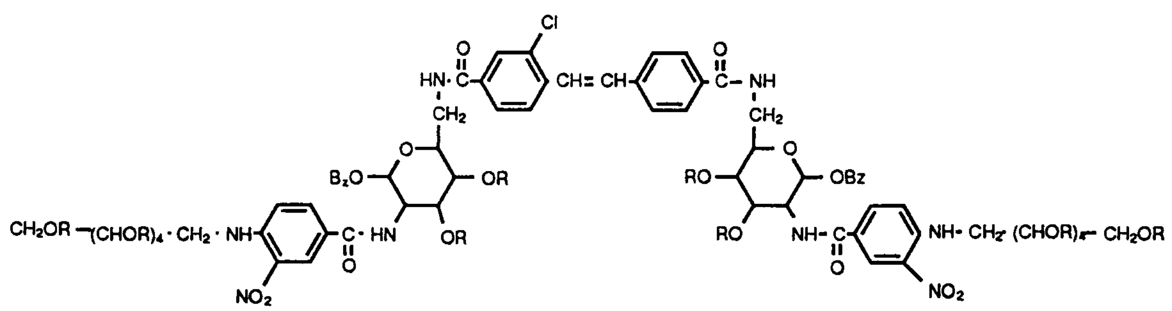

Bz= Benzyl R=$SO_3Na$

Isophthalsäure-bis-[[benzyl-2,6-didesoxy-2-[4-(2,3,4,5,6-penta-O-sulfo-D-glucit-1-ylamino)-3-nitro-benzoylamino]-3,4-di-O-sulfo-a-D-glucopyranosid-6-yl]-amid] Tetradekanatriumsalz ( Beispiel 33 ).

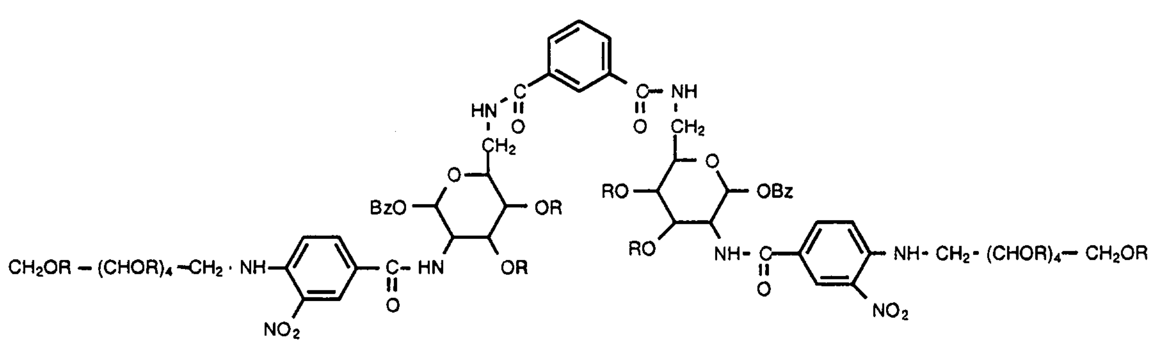

Bz= Benzyl R=$SO_3Na$

2,7-Bis-[benzyl-2,6-didesoxy-2-[3-nitro-4-(2,3,4,5,6-penta-O-sulfo-D-glucit-1-ylamino)-benzoylamino]-3,4-di-O-sulfo-a-D-glucopyranosid-6-yl]-1,2,3,6,7,8-hexahydro-benzo[lmn][3,8]phenanthrolin-1,3,6,8-tetraon Tetradekanatriumsalz ( Beispiel 34).

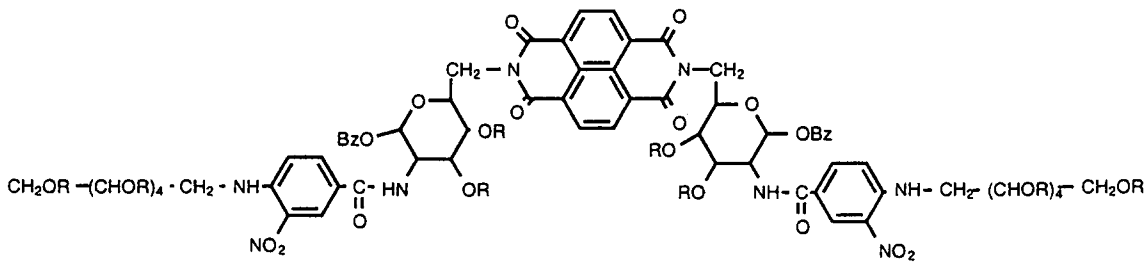

Bz= Benzyl R=$SO_3Na$

Isophthalsäure-bis-[[benzyl-2,6-didesoxy-2-[4-[methyl-(2,3,4,5,6-penta-O-sulfo-D-glucit-1-yl)-amino]-3-nitro-benzoylamino]-3,4-di-O-sulfo-a-D-glucopyranosid-6-yl]-amid] Tetradekanatriumsalz ( Beispiel 35 ).

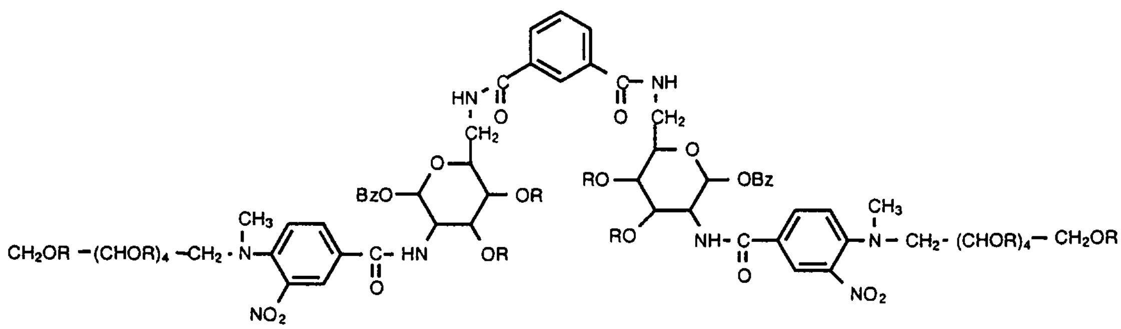

Bz= Benzyl R=$SO_3Na$

Isophthalsäure-bis-[[benzyl-2,6-didesoxy-2-[4-[(2-hydroxysulfonyloxyethyl)-2,3,4,5,6-penta-O-sulfo-D-glucit-1-yl)-amino]-3-nitro-benzoylamino]-3,4-di-O-sulfo-a-D-glucopyranosid-6-yl]-amid] Hexadekanatriumsalz (Beispiel 36 ).

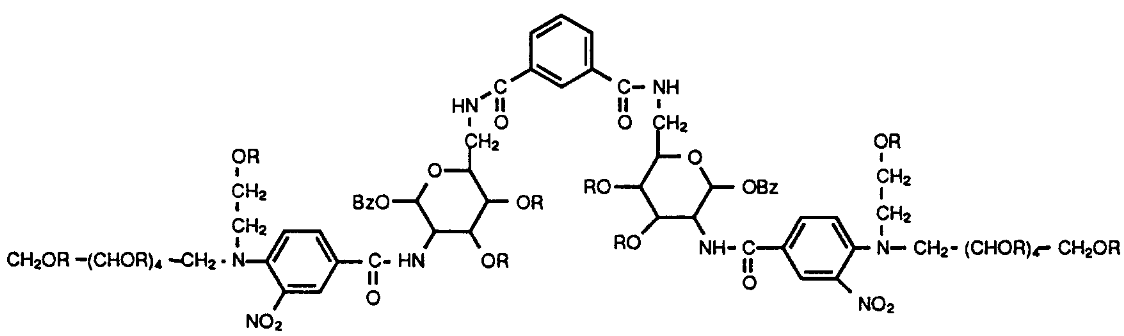

Bz= Benzyl R=$SO_3Na$

Isophthalsäure-bis-[[benzyl-2-[3-acetylamino-4-[methyl-(2,3,4,5,6-penta-O-sulfo-D-glucit-1-yl)-amino]-benzoylamino]-2,6-didesoxy-3,4-di-O-sulfo-a-D-glucopyranosid-6-yl]-amid] Tetradekanatriumsalz ( Beispiel 37).

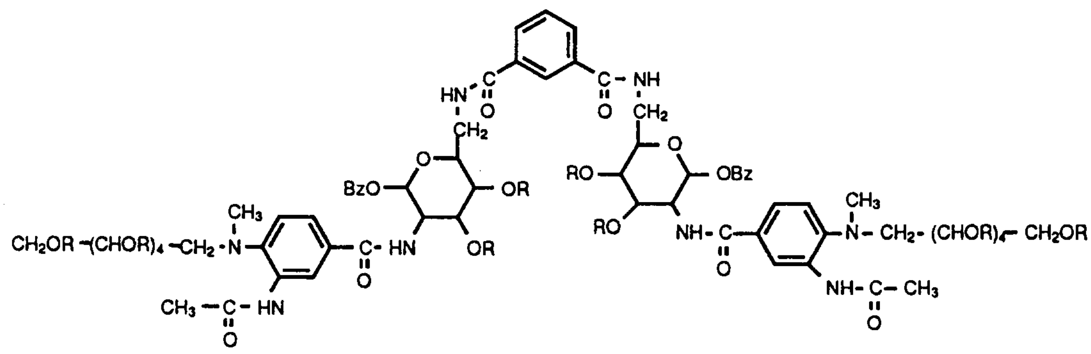

Bz= Benzyl R=$SO_3Na$

Isophthalsäure-bis-[[benzyl-2-[3-acetylamino-4-[(2,3,4,5,6-penta-O-sulfo-D-glucit-1-yl)-(2-sulfonyloxy-ethyl)-amino]-benzoylamino]-2,6-didesoxy-3,4-di-O-sulfo-a-D-glucopyranosid-6-yl]-amid] Hexadekanatriumsalz ( Beipiel 38 ).

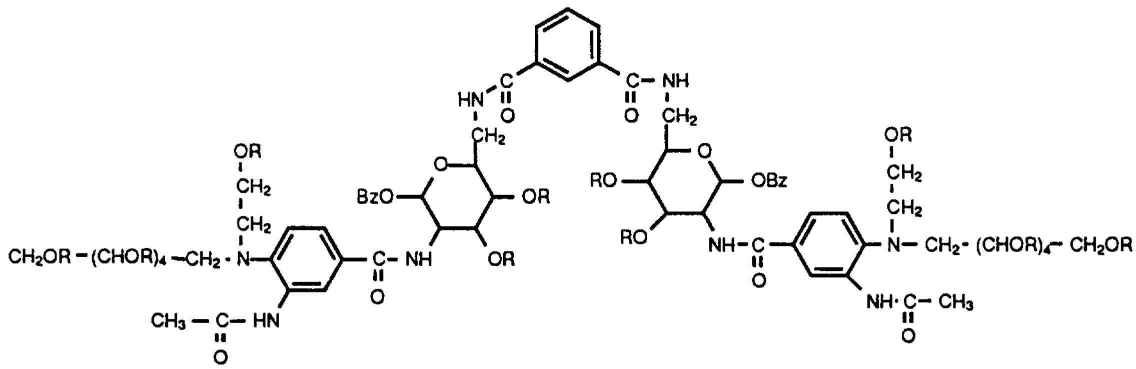

Bz= Benzyl R=$SO_3Na$

Ein Beispiel für eine unsymmetrische Verbindung, die einen Glycopyranosidrest der Formel d und einen Glycopyranosidrest der Formel f enthält ist N-[Benzyl-2,6-didesoxy-2-[4-(2,3,4,5,6-penta-O-sulfo-D-glucit-1-ylamino)-3-nitro-benzoylamino]-3,4-di-O-sulfo-a-D-glucopyranosid-6-yl]-N'-[(benzyl-2,6-didesoxy-6-(2,3,4,5,6-penta-O-sulfo-D-gluconylamino)-3,4-di-O-sulfo-a-D-glucopyranosid-2-yl]-isophthalamid Tetradekanatriumsalz (Beispiel 39).

Die eingangs definierten Verbindungen können erfindungsgemäss dadurch hergestellt werden, dass man eine entsprechende, nicht sulfatierte Verbindung mit einem Sulfatierungsmittel umsetzt.

Die erfindungsgemässe Sulfatierung kann mittels Methoden, die für die Sulfatierung von Hydroxygruppen an sich bekannt sind, vorgenommen werden. Beispiele von Sulfatierungsmitteln sind $SO_3$ - Komplexe wie $SO_3$ • Pyridin, $SO_3$ • Trimethylamin, $SO_3$ • Dioxan und $SO_3$ • Dimethylformamid. Weitere Beispiele von Sulfatierungsmitteln sind Chlorsulfonsäure, Gemische von Chlorsulfonsäure und Schwefelsäure; und Piperidin-N-Sulfat.

Die Umsetzung erfolgt zweckmässigerweise in einem geeigneten Lösungsmittel, insbesondere einem polaren Lösungsmittel, z.B. Dimethylformamid, Dimethylsulfoxid oder Hexamethylphosphortriamid. Die Reaktion kann bei Raumtemperatur oder bei erhöhter Temperatur, z.B. bei 20-70°C, durchgeführt werden, wobei durch Variation von Reaktionsdauer und -temperatur der Sulfatierungsgrad beeinflusst werden kann. Der jeweils erreichte Sulfatierungsgrad kann durch HPLC abgeschätzt werden. In einer bevorzugten Ausführungsform werden durch geeignete Wahl von Reaktionsdauer und -temperatur alle oder praktisch alle freien Hydroxygruppen sulfatiert. Die Aufarbeitung des Reaktiongemisches bzw. die Isolierung des Reaktionprodukts der Formel Ia-Ic kann nach an sich bekannten Methoden erfolgen, z.B. durch Gelfiltration oder Ultrafiltration. Zweckmässig wird das Reaktionsgemisch vor der Aufarbeitung mit einer zur Salzbildung mit den Sulfonsäuregruppen in der Verbindung der Formel Ia-Ic befähigten, hinreichend basischen Verbindung, z.B. mit einem Alkalimetallazetat, wie Natriumazetat, versetzt und die Verbindung der Formel Ia-Ic in Salzform, z.B. als Natriumsalz, isoliert.

Die Ausgangsstoffe für das erfindungsgemässe Verfahren, d.h. die den Verbindungen der Formel Ia-Ic entsprechenden, nicht sulfatierten Verbindungen, können wie in den nachstehenden Beispielen beschrieben oder in Analogie

dazu hergestellt werden. Die Ausgangsverbindungen für Substanzen der Formel Ia-Ic können allgemein wie folgt hergestellt werden:

Als Aminozucker können entweder kommerziell erhältliche Aminozucker wie Glucosamin oder Galaktosamin verwendet werden, oder eine Aminofunktion kann nach an sich bekannten Methoden eingeführt werden. So kann eine Hydroxylgruppe einer Pyranose durch Umwandlung in ein Sulfonat oder ein Halogenid aktiviert werden und dann zum Amin direkt oder über die intermediäre Einführung eines Azids umgesetzt werden. Die anomere Position der Pyranose wird zweckmässigerweise durch Umwandlung in ein Pyranosid geschützt, was durch Fischer-Glykosidierung oder andere Methoden der Glykosidsynthese erfolgen kann.

Die freie Aminofunktion des Aminozuckers wird mit einem zentralen Alkyl- oder Arylbaustein umgesetzt. Ist die Verknüpfungsreaktion die Bildung einer Amidbindung, so kann diese dadurch erfolgen, dass der Aminozucker mit dem zentralen Baustein in Form eines Esters, Lactons, eines Säurechlorides oder einer nach bekannten Methoden der Peptidchemie aktivierten Säurefunktion (gemischtes Anhydrid oder Aktivester) zur Reaktion gebracht wird. Durch Verwendung von Anhydriden des zentralen Bausteins können analog Imide erhalten werden.

Die Aminozucker können nach weiterer Funktionalisierung erweitert werden. Eine Möglichkeit dazu besteht in der Einführung einer weiteren Aminofunktion, die wie oben beschrieben nach an sich bekannten Verfahren erreicht werden kann. Eine andere Möglichkeit liegt in der Einführung einer Säurefunktionalität; dies kann durch Alkylierung einer vorhandenen Hydroxylgruppe mit einem Säureequivalent erreicht werden. Die so erhaltenen Säurederivate von Aminozuckern können nach Aktivierung wie oben beschrieben mit geeigneten zyklischen oder offenkettigen Derivaten von Aminozuckern oder Aminoglycitolen umgesetzt werden. Andererseits können die um eine Aminofunktion erweiterten Aminozucker unter Bildung von Amiden mit hydroxylierten Lactonen umgesetzt werden.

Die erfindungsgemässen Verbindungen hemmen die Migration und Proliferation von glatten Muskelzellen der Gefässwand. Sie können also zur Behandlung und/oder Prophylaxe von arteriosklerotischen Gefässwandveränderungen, insbesondere zur Verhinderung von Restenose nach koronarer oder peripherer Angioplastie oder nach Bypass-Operationen, verwendet werden. Im Prinzip können diese Substanzen zur Behandlung und/oder Prophylaxe von allen Erkrankungen, bei denen Migration oder Proliferation von glatten Muskelzellen eine Rolle spielen, eingesetzt werden.

Im Gegensatz zu Heparin haben diese Verbindungen aber keine $AT_{III}$-Aktivität (Antithrombin III) und somit keine inhibierende Wirkung auf die Gerinnungsfaktoren IIa und Xa. Demzufolge ist ihre blutgerinnungshemmende Aktivität sehr viel geringer als die von Heparin, und damit ist das Blutungsrisiko bei der Therapie mit diesen Substanzen minimal.

Da Heparin-bindende Proteine bei verschiedenen Krankheiten eine wichtige Rolle spielen, können Heparin-ähnliche Substanzen wie die erfindungsgemässen Verbindungen zusätzlich auch zur Behandlung dieser Krankheiten eingesetzt werden: z.B. wird die Aufnahme von verschiedenen Viren (Herpes, HIV) durch solche Substanzen inhibiert, die arterielle Thrombose (vWF, Plättchenadhäsion) wird durch solche Substanzen gehemmt, die Aktivierung des Komplementsystem (z.B. bei Reperfusion) kann gemindert werden, und verschiedene Wachstumsfaktoren oder Cytokine (z.B. bFGF in Tumoren) können inhibiert werden.

Die pharmakologischen Aktivitäten der erfindungsgemässen Verbindungen können in den nachstehend beschriebenen Versuchsanordnungen gezeigt werden:

<u>Antiproliferative Aktivität</u>

Die antiproliferative Aktivität einer Substanz wird als $r_i$-Wert ausgedrückt, der einen Vergleichswert zu der entsprechenden Aktivität von Heparin darstellt und der in Zellkulturen wie folgt ermittelt wurde: Glatte Muskelzellen der Ratte wurden mit einer Dichte von $8x10^3$ Zellen/Loch auf Zellkulturplatten ausgebracht (Medium: DMEM mit 10% FCS. Kultivation bei 37°C und 5% $CO_2$). Nach 4 Stunden wurde die Zahl der adhärierten Zellen bestimmt und die zu testenden Substanzen (100 $\mu$g/ml, gelöst in $H_2O$) zugegeben. Als Kontrolle dienten a) Zellen, denen nichts zugegeben wurde, und b) Zellen, die mit Heparin (100 $\mu$g/ml) versetzt wurden. Anschliessend wurden die Zellen für 48 h inkubiert, und danach wurde wieder die Zellzahl bestimmt.

Aus diesen Werten wurde die Inhibition **i** des Zellwachstums, d.h. die Erniedrigung der Wachstumsrate der Zellen in Prozenten im Vergleich zur Kontrolle ausgerechnet.

$$i = 100 - \frac{\mu_{\text{Substanz}}}{\mu_{\text{Kontrolle}}} \cdot 100$$

wobei die Wachstumsrate μ errechnet wurde als

$$\mu = \frac{\Delta \ln Z}{\Delta t_{[d]}} = \ln \frac{Z_{(t_2)}}{Z_{(t_1)}} * \frac{1}{\Delta t_{[d]}} \quad [d^{-1}]$$

wobei Z die Anzahl der Zellen und d die Zeit in Tagen ist.

Schliesslich wurde $r_i$ - die relative inhibitorische Aktivität - welche the Aktivität von einer Substanz (bei 100 μg/ml) im Vergleich zur Aktivität von Heparin bei gleicher Konzentration im gleichen Experiment ausdrückt, errechnet:

$$r_i = \frac{i_{Substanz}}{i_{Heparin}}$$

Blutgerinnungshemmung

Die blutgerinnungshemmende Wirkung wurde wie folgt ermittelt:

Hemmung von Thrombin oder Faktor Xa im Chromogen Substrat Test (Teien et al., Thrombosis Research 10, 399-410 (1977)): Die Bestimmung erfolgte im Cobas-Bio Centrifugal Automatic Spectrophotometer. Die verwendete Pufferlösung bestand aus 50 mM Tris-Puffer, 180 mM NaCl, 7.5 mM EDTA-$Na_2$, 1% PEG 6000 und 0.02% Tween-80, pH 8.4. Die Testlösung bestand aus 50 μl Puffer, 30 μl Antithrombin III (1U/ml, Kabi-Diagnostica) und 20 μl Plasma, das verschiedene Konzentrationen der Versuchsverbindungen enthielt. Im Analyseautomaten wurden 30 μl Probenlösung und 20 μl Wasser mit 180 μl Thrombin (1U/ml, Thrombin Reagent Roche Basel) in die Testküvette gegeben. Nach 240 Sekunden Inkubation bei 37°C wurden 60 μl S-2238 (H-D-Phe-Pip-Arg-NH.pNA, Kabi Diagnostica, Möndal, Schweden, 0.75 mM in Wasser) und 20 μl Wasser zugesetzt. Die Freisetzung von pNA (p-Nitro-anilin) wurde während 60 Sekunden bei 405 nm in 10-Sekunden-Intervallen im Vergleich zu Wasser als Blindwert verfolgt. Die Hemmwirkung wird als $IC_{50}$, d.h. als Konzentration [μg/ml] angegeben, bei der die amidolytische Aktivität von Thrombin im Vergleich zum Plasma-Kontrollwert um 50% vermindert wird.

In gleicher Weise wurde die Hemmung von Faktor Xa gemessen, wobei eine Lösung von Faktor Xa (2.8 nkat/ml und 2 mM S-2222 (Bz-CO-Ile-Glu-Arg-NH.pNA, Kabi Diagnostica) in Wasser anstelle von Thrombin bzw. S-2238 verwendet wurde.

In der nachstehenden Tabelle sind die in den vorstehend beschriebenen Versuchsanordnungen mit einer repräsentativen Anzahl Verbindungen der Formel Ia - Ic erhaltenen Aktivitätsdaten angeführt:

| Beispiel | Antiproliferative Aktivität ri | Antikoagulative Aktivität $IC_{50}$ [µg/ml] | |
|---|---|---|---|
| | | Thrombin | Faktor Xa |
| 11 | 1,7 | > 1000 | > 1000 |
| 14 | 1,2 | > 1000 | > 1000 |
| 15 | 1,8 | > 1000 | > 1000 |
| 23 | 1,8 | > 1000 | > 1000 |
| 30 | 1,6 | > 1000 | > 1000 |
| 32 | 1,4 | > 1000 | > 1000 |
| 33 | 1,5 | > 1000 | > 1000 |
| 34 | 3,9 | > 1000 | > 1000 |
| 35 | 2,5 | > 1000 | > 1000 |
| 36 | 2,1 | > 1000 | > 1000 |
| 37 | 1,9 | > 1000 | > 1000 |
| 38 | 1,8 | > 1000 | > 1000 |
| Heparin | 1,0 | 1,9 | 2,7 |

In vivo Assay zur Bestimmung der antiproliferativen Aktivität der erfindungsgemässen Verbindungen in verletzten Carotiden der Ratte

Die linken Carotiden von männlichen Wistar Kyoto Ratten (300 - 400g) wurden nach erfolgter Narkose mit einem 2F Embolektomiekatheter verletzt, indem der Katheter im aufgepumpten Zustand dreimal durch das Gefäß gezogen wurde. Nach der Wundversorgung wurden die Tiere mit Standardfutter und Wasser ad libidum paarweise gehalten.

Die Verbindungen wurden in Konzentrationen von 0.3 - 1mg/kg/h i.v. appliziert. Hierzu wurde den Tieren während der Narkose unter die Rückenhaut eine osmotische Minipumpe implantiert, die mit der Vena jugularis verbunden wurde. So konnten die Verbindungen während der gesamten Versuchsdauer von 14 Tagen konstant verabreicht werden.

Nach 14 Tagen hatte sich proliferatives Gewebe gebildet (Neointima), dessen Größe sich morphometrisch an histologischen Querschnitten bestimmen ließ. Hierzu wurden die Ratten getötet und mit Glutaraldehyd perfusionsfixiert.

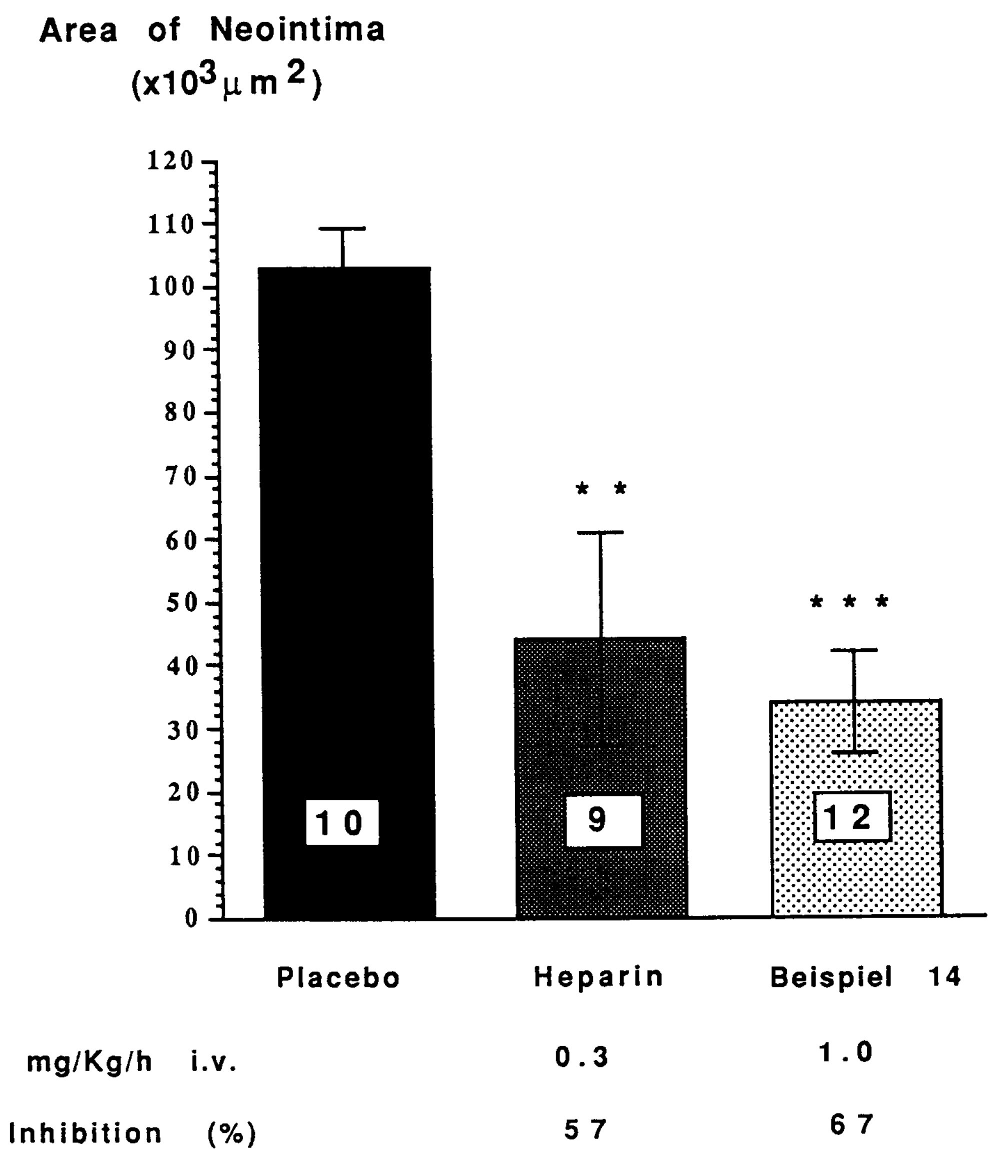


Die Querschnittsfläche der Neointima in Ratten Carotiden 14 Tage nach Ballonisierung war nach i.v. Applikation von Beispiel 14 (1 mg/kg/h) signifikant reduziert ($p<0.001$, t-test; die Anzahl der Tiere n wie in der Abbildung angegeben; Mittelwerte ± SEM).

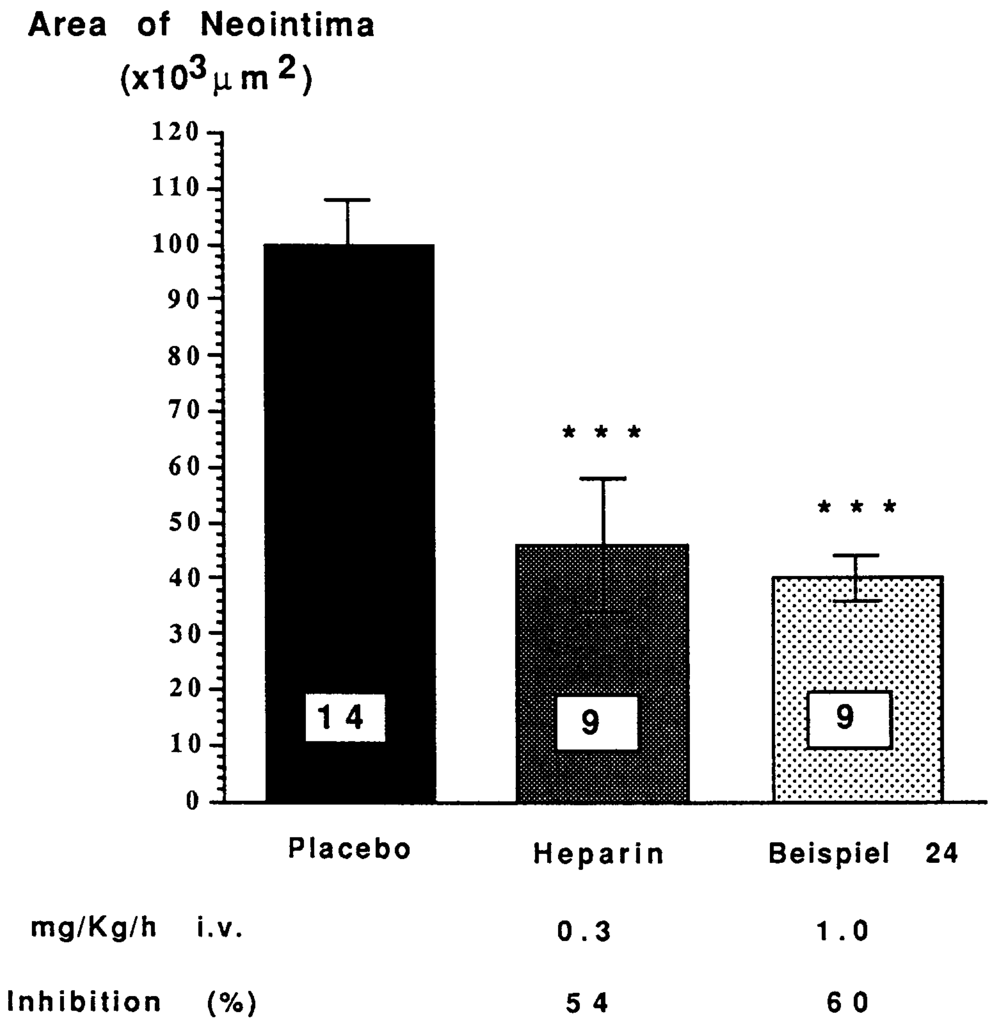


Die Querschnittsfläche der Neointima in Rattencarotiden 14 Tage nach Ballonisierung war nach i.v. Applikation von Beispiel 24 (1 mg/kg/h) signifikant reduziert ($p<0.001$, t-Test; n wie in der Abbildung angegeben; Mittelwerte ± SEM).

Die Versuchsresultate zeigen, dass die erfindungsgemässen Verbindungen eine antiproliferative Wirkung aufweisen, die derjenigen von Heparin entspricht (oder nahekommt) oder grösser ist, im Gegensatz zu Heparin jedoch keine oder eine viel geringere anti-Koagulationswirkung zeigen.

Die Arzneimittel auf der Basis der erfindungsgemässen Verbindungen können enteral, z.B. oral in Form von Tabletten, Lacktabletten, Dragées, Hart- und Weichgelatinekapseln, Lösungen, Emulsionen oder Suspensionen, oder rektal, z.B. in Form von Suppositorien, verabreicht werden. Die Verabreichung erfolgt jedoch vorzugsweise parenteral, z.B. in Form von Injektionslösungen.

Zur Herstellung von Tabletten, Lacktabletten, Dragées und Hartgelatinekapseln kann der Wirkstoff mit pharmazeutisch inerten, anorganischen oder organischen Excipientien vermischt werden. Als solche Excipientien kann man für Tabletten, Dragées und Hartgelatinekapseln z.B. Lactose, Maisstärke oder Derivate davon, Talk, Stearinsäure oder deren Salze, verwenden. Für Weichgelatinekapseln eignen sich als Excipientien z.B. vegetabile Oele, Wachse, Fette, halbfeste und flüssige Polyole; je nach Beschaffenheit des Wirkstoffes sind jedoch bei Weichgelatinekapseln überhaupt keine Excipientien erforderlich. Zur Herstellung von Lösungen und Sirupen eignen sich als Excipientien z.B. Wasser, Polyole, Saccharose, Invertzucker und Glukose, für Injektionslösungen eignen sich z.B. Wasser, Alkohole, Polyole, Glycerin und vegetabile Oele, und für Suppositorien eignen sich z.B. natürliche oder gehärtete Oele, Wachse, Fette und halbflüssige oder flüssige Polyole.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Bei enteraler Verabreichung kann die Resorption des Wirkstoffs mit Hilfe von Liposomen erhöht werden.

Die Dosierung des Wirkstoffes kann innerhalb weiter Grenzen variieren und ist natürlich in jedem einzelnen Fall den individuellen Gegebenheiten anzupassen. Im allgemeinen dürfte bei parenteraler Verabreichung eine Dosis von etwa 0,1 bis 100 mg/kg, vorzugsweise von etwa 1,5 bis 15 mg/kg, pro Tag für den Erwachsenen angemessen sein, wobei aber die soeben angegebene obere Grenze auch überschritten werden kann, wenn sich dies als angezeigt erweisen sollte.

Die Erfindung wird durch nachstehende Beispiele weiter erläutert.

Beispiel 1

A. Eine Lösung von 193 mg Malonsäure und 1,11 g O-(1,2-Dihydro-2-oxo-pyridyl)-1,1,3,3-tetramethyluronium - tetrafluoroborat in 5 ml Dimethylformamid wurde bei 0°C mit 0,77 ml Triethylamin versetzt. Nach 90 Minuten bei Raumtemperatur wurde eine Lösung von 1,0 g Benzyl-2-amino-2-desoxy-a-D-glucopyranosid (Meyer zu Reckendorf, Chem. Ber. **107,** 869 (1974)) in 10 ml Dimethylformamid zugegeben und 18 Stunden bei 95°C gerührt. Nach Einengen wurde der Rückstand mit Essigester/ Methanol/ Wasser über Kieselgel chromatographiert und ergab N,N'-Bis-(benzyl-2-desoxy-a-D-glucopyranosid-2-yl)-malonamid, MS: *m/z* 669,4 ($[M + H]^+$).

B. Eine Lösung von 360 mg N,N'-Bis-(benzyl-2-desoxy-a-D-glucopyranosid-2-yl)-malonamid in 2,5 ml Dimethylformamid wurde mit 991 mg Schwefeltrioxid-Trimethylaminkomplex versetzt und 18 Stunden bei 70°C gerührt. Dann wurde mit 11,7 ml 10 %iger wässriger Natriumacetatlösung versetzt und eingedampft, nochmals in Wasser aufgenommen und eingedampft. Der Rückstand wurde durch Chromatographie über Sephadex LH 20 und C25 ($Na^+$) gereinigt und ergab N,N'-Bis-(benzyl-2-desoxy-3,4,6-tri-O-sulfo-a-D-glucopyranosid-2-yl)-malonamid Hexanatriumsalz, $[\alpha]_D^{20}$ +96,5° (*c* 0,2; Wasser), MS: *m/z* 1218 (rekonstruiertes M).

Beispiel 2

A. Eine Suspension von 332 mg Terephthalsäure in 5 ml Tetrahydrofuran und 5 ml Acetonitril wurde bei 0°C mit 0,62 ml Triethylamin und 0,624 ml Chlorameisensäureisobutylester versetzt. Zu der entstandenen Lösung wurde eine Lösung von 1,35 g Benzyl-2-amino-2-desoxy-a-D-glucopyranosid in 4 ml Wasser und 2 ml Acetonitril gegeben. Nach 2 Stunden Rühren bei Raumtemperatur wurde eingeengt. Der Rückstand wurde mit 40 ml Pyridin und 20 ml Acetanhydrid versetzt und nach 18 Stunden bei Raumtemperatur eingeengt. Der Rückstand wurde mit Essigester/ Hexan über Kieselgel chromatographiert und ergab N,N'-Bis-(benzyl-3,4,6-tri-O-acetyl-2-desoxy-a-D-glucopyranosid-2-yl)-terephthalamid, $[\alpha]_D^{20}$ +144,0° (*c* 0,2; Dioxan), MS: *m/z* 921,7 ($[M + H]^+$).

B. Eine Lösung von 1,2 g N,N'-Bis-(benzyl-3,4,6-tri-O-acetyl-2-desoxy-a-D-glucopyranosid-2-yl)-terephthalamid in 120 ml Methanol wurde mit 9 ml einer 0,3 m Natriummethanolatlösung 3 Stunden bei Raumtemperatur gerührt. Der entstandene Niederschlag wurde mit Methanol neutral gewaschen, getrocknet und ergab N,N'-Bis-(benzyl-2-desoxy-a-D-glucopyranosid-2-yl)-terephthalamid, MS: *m/z* 669,4 ($[M + H]^+$).

C Sulfatierung von N,N'-Bis-(benzyl-2-desoxy-a-D-glucopyranosid-2-yl)-terephthalamid wie in Bsp. 1.B. beschrieben ergab N,N'-Bis-(benzyl-2-desoxy-3,4,6-tri-O-sulfo-a-D-glucopyranosid-2-yl)-terephthalamid Hexanatriumsalz, $[\alpha]_D^{20}$ +52,5° (*c* 0,2; Wasser), MS: *m/z* 1280 (rekonstruiertes M).

Beispiel 3

A. Benzyl-2-amino-2-desoxy-a-D-glucopyranosid wurde mit 4,4'-Biphenyldicarbonsäure wie unter Bsp. 2.A. beschrieben umgesetzt und ergab Biphenyl-4,4'-dicarbonsäure-bis-[(benzyl-3,4,6-tri-O-acetyl-2-desoxy-a-D-glucopyranosid-2-yl)-amid], $[\alpha]_D^{20}$ +177,0° (*c* 0,2; Dioxan), MS: *m/z* 997,8 ($[M + H]^+$).

B. Biphenyl-4,4'-dicarbonsäure-bis-[(benzyl-3,4,6-tri-O-acetyl-2-desoxy-a-D-glucopyranosid-2-yl)-amid] wurde deacetyliert wie in Bsp. 2.B. beschrieben und ergab Biphenyl-4,4'-dicarbonsäure-bis-[(benzyl-2-desoxy-a-D-glucopyranosid-2-yl)-amid], MS: *m/z* 743,6 ($[M - H]^-$).

C. Eine Suspension von 750 mg Biphenyl-4,4'-dicarbonsäure-bis-[(benzyl-2-desoxy-a-D-glucopyranosid-2-yl)-amid] in 20 ml Dimethylformamid wurde mit 2,7 g Schwefeltrioxid-Trimethylaminkomplex versetzt und 3 Tage bei 65°C gerührt. Dann wurde mit 40 ml 10 %iger wässriger Natriumacetatlösung versetzt und eingedampft und nochmals in Wasser aufgenommen und eingedampft. Der Rückstand wurde durch Chromatographie über Sephadex LH 20 und C25 ($Na^+$) gereinigt und ergab Biphenyl-4,4'-dicarbonsäure-bis-[(benzyl-2-desoxy-3,4,6-tri-O-sulfo-a-D-glucopyranosid-2-yl)-amid] Hexanatriumsalz, $[\alpha]_D^{20}$ +105,0° (*c* 0,2; Wasser), MS: *m/z* 1356 (rekonstruiertes M).

Beispiel 4

A. Eine Suspension von 750 mg Biphenyl-4,4'-dicarbonsäure-bis-[(benzyl-2-desoxy-a-D-glucopyranosid-2-yl)-amid (vgl. Bsp. 3.B.) in 20 ml Dimethylformamid wurde mit 1,68 g Schwefeltrioxid-Trimethylaminkomplex versetzt und 18 Stunden bei 70°C gerührt. Dann wurde mit 19,9 ml 10 %iger wässriger Natriumacetatlösung versetzt und

eingedampft und nochmals in Wasser aufgenommen und eingedampft. Der Rückstand wurde durch Chromatographie über Sephadex LH 20 und C25 ($Na^+$) gereinigt und ergab Biphenyl-4,4'-dicarbonsäure-bis-[(benzyl-2-desoxy-3,6-di-O-sulfo-a-D-glucopyranosid-2-yl)-amid] Tetranatriumsalz, $[\alpha]_D^{20}$ +102,5° (*c* 0,2; Wasser), MS: *m/z* 1152 (rekonstruiertes M).

Beispiel 5

A. Benzyl-2-amino-2-desoxy-a-D-glucopyranosid wurde mit Naphthalin-1,4-dicarbonsäure wie unter Bsp. 2.A. beschrieben umgesetzt und ergab Naphthalin-1,4-dicarbonsäure-bis-[(benzyl-3,4,6-tri-O-acetyl-2-desoxy-a-D-glucopyranosid-2-yl)-amid], $[\alpha]_D^{20}$ D +153,5° (*c* 0,2; Dioxan), MS: *m/z* 971,6 ($[M + H]^+$).
B. Naphthalin-1,4-dicarbonsäure-bis-[(benzyl-3,4,6-tri-O-acetyl-2-desoxy-a-D-glucopyranosid-2-yl)-amid] wurde deacetyliert wie in Bsp. 2.B. beschrieben und ergab Naphthalin-1,4-dicarbonsäure-bis-[(benzyl-2-desoxy-a-D-glucopyranosid-2-yl)-amid], MS: *m/z* 719,4 ($[M + H]^+$).
C Sulfatierung von Naphthalin-1,4-dicarbonsäure-bis-[(benzyl-2-desoxy-a-D-glucopyranosid-2-yl)-amid] wie in Bsp. 1.B. beschrieben ergab Naphthalin-1,4-dicarbonsäure-bis-[(benzyl-2-desoxy-3,4,6-tri-O-sulfo-a-D-glucopyranosid-2-yl-amid] Hexanatriumsalz, $[\alpha]_D^{20}$ +133,5° (*c* 0,2; Wasser), MS: *m/z* 1330 (rekonstruiertes M).

Beispiel 6

A. Benzyl-2-amino-2-desoxy-a-D-glucopyranosid wurde mit Naphthalin-2,6-dicarbonsäure wie unter Bsp. 2.A. beschrieben umgesetzt und ergab (Naphthalin-2,6-dicarbonsäure-bis-[(benzyl-3,4,6-tri-O-acetyl-2-desoxy-a-D-glucopyranosid-2-yl)-amid], $[\alpha]_D^{20}$ +175,0° (*c* 0,2; Dioxan), MS: *m/z* 971,4 ($[M + H]^+$).
B. Eine Lösung von 1,0 g (Naphthalin-2,6-dicarbonsäure-bis-[(benzyl-3,4,6-tri-O-acetyl-2-desoxy-a-D-glucopyranosid-2-yl)-amid] in 20 ml Methanol und 20 ml Dioxan wurde mit 5 ml einer 0,3 m Natriummethanolatlösung 18 Stunden bei Raumtemperatur gerührt. Der entstandene Niederschlag wurde mit Methanol, Dioxan und Ether gewaschen, getrocknet und ergab Naphthalin-2,6-dicarbonsäure-bis-[(benzyl-2-desoxy-a-D-glucopyranosid-2-yl)-amid], MS: *m/z* 717,2 ($[M - H]^-$).
C Sulfatierung von Naphthalin-2,6-dicarbonsäure-bis-[(benzyl-2-desoxy-a-D-glucopyranosid-2-yl)-amid] wie in Bsp. 1.B. beschrieben ergab Naphthalin-2,6-dicarbonsäure-bis-[(benzyl-2-desoxy-3,4,6-tri-O-sulfo-a-D-glucopyranosid- 2-yl)-amid] Hexanatriumsalz, $[\alpha]_D^{20}$ +117,0° (*c* 0,2; Wasser), MS: *m/z* 1330 (rekonstruiertes M).

Beispiel 7

A. Benzyl-2-amino-2-desoxy-a-D-glucopyranosid wurde mit (E)-Stilben-4,4'-dicarbonsäure wie unter Bsp. 2.A. beschrieben umgesetzt und ergab (E)-Stilben-4,4'-dicarbonsäure-bis-[(benzyl-3,4,6-tri-O-acetyl-2-desoxy-a-D-glucopyranosid-2-yl)-amid], $[\alpha]_D^{20}$ +195,0° (*c* 0,2; Dioxan), MS: *m/z* 1023,4 ($[M + H]^+$).
B. (E)-Stilben-4,4'-dicarbonsäure-bis-[(benzyl-3,4,6-tri-O-acetyl-2-desoxy-a-D-glucopyranosid-2-yl)-amid] wurde deacetyliert wie in Bsp. 6.B. beschrieben und ergab (E)-Stilben-4,4'-dicarbonsäure-bis-[(benzyl-3,4,6-tri-O-acetyl-2-desoxy-a-D-glucopyranosid-2-yl)-amid], MS: *m/z* 769,2 ($[M - H]^-$).
C Sulfatierung von (E)-Stilben-4,4'-dicarbonsäure-bis-[(benzyl-3,4,6-tri-O-acetyl-2-desoxy-a-D-glucopyranosid-2-yl)-amid] wie in Bsp. 1.B. beschrieben ergab (E)-Stilben-4,4'-dicarbonsäure-bis-[(benzyl-2-desoxy-3,4,6-tri-O-sulfo-a-D-glucopyranosid-2-yl)-amid] Hexanatriumsalz, $[\alpha]_D^{20}$ +131,0° (*c* 0,2; Wasser), MS: *m/z* 1382 (rekonstruiertes M).

Beispiel 8

A. Eine Lösung von 300 mg N,N'-Bis-(benzyl-2-desoxy-3,4,6-tri-O-sulfo-a-D-glucopyranosid-2-yl)-terephthalamid Hexanatriumsalz (vgl. Bsp. 2.C.) in 10 ml Wasser wurde 16 Stunden bei Raumtemperatur in Gegenwart von Palladium auf Kohle hydriert. Nach Filtration über Filterhilfe wurde das Filtrat eingeengt, durch Chromatographie über Sephadex LH 20 gereinigt und ergab N,N'-Bis-(2-desoxy-3,4,6-tri-O-sulfo-D-glucopyranos-2-yl)-terephthalamid Hexanatriumsalz, MS: *m/z* 1100 (rekonstruiertes M).

Beispiel 9

A. Eine Lösung von 30 g Benzyl-2-benzyloxycarbonylamino-2-desoxy-a-D-glucopyranosid (Heyns und Paulsen, Chem. Ber. **88**, 188 (1955)) in 116 ml Pyridin wurde bei 0°C mit einer Lösung von 19,85 g p-Tolylsulfonylchlorid in 30 ml Dichlormethan versetzt und 4 Stunden bei Raumtemperatur gerührt. Dann wurde auf eiskalte 2 n Schwefelsäure gegossen und mit Dichlormethan extrahiert. Die organischen Phasen wurden mit wässriger Natriumhydrogenkarbonatlösung gewaschen, über Magnesiumsulfat getrocknet und eingeengt. Der Rückstand wurde mit

Hexan/ Essigester über Kieselgel chromatographiert und ergab Benzyl-2-benzyloxycarbonyl-amino-2-desoxy-6-O-(p-tolylsulfonyl)-a-D-glucopyranosid, $[\alpha]_D^{20}$ +101,8° (*c* 0,5; Dioxan), MS: *m/z* 580 ([M + Na]$^+$).

B. Eine Lösung von 32,14 g Benzyl-2-benzyloxycarbonylamino-2-desoxy-6-O-(p-tolylsulfonyl)-a-D-glucopyranosid in 75 ml Dimethylsulfoxid wurde bei Raumtemperatur mit 7,5 g Natriumazid versetzt und 3 Stunden bei 90°C gerührt. Dann wurde auf Eis/ Wasser gegossen. Ausgefallene Kristalle wurden abgenutscht, mit Wasser gewaschen und getrocknet. Es wurde Benzyl-6-azido-2-benzyloxycarbonylamino-2,6-didesoxy-a-D-glucopyranosid erhalten, $[\alpha]_D^{20}$ +119,6° (*c* 0,5; Dioxan), MS: *m/z* 428 ([M + H]$^+$).

C. Eine Lösung von 1,1 g Benzyl-6-azido-2-benzyloxycarbonylamino-2,6-didesoxy-a-D-glucopyranosid in 7,5 ml Tetrahydrofuran und 69 ml Wasser wurde bei Raumtemperatur mit 674 mg Triphenylphosphin versetzt und 24 Stunden gerührt. Dann wurde zu dem dicken Brei 1 ml Wasser gegeben, 30 Minuten weitergerührt und eingeengt. Der Rückstand wurde aus Methanol kristallisiert und ergab Benzyl-6-amino-2-benzyloxycarbonylamino-2,6-didesoxy-a-D-glucopyranosid, $[\alpha]_D^{20}$ +124,2° (*c* 0,6; Aceton), MS: *m/z* 403 ([M + H]$^+$).

D. Eine Lösung von 1,07 g 1,2-O-Isopropyliden-a-D-glucofuranurono-6,3-lacton (Weidmann, Ann. **679**, 178 (1964)) in 10 ml Tetrahydrofuran wurde bei 10°C mit einer Suspension von 2,0 g Benzyl-6-amino-2-benzyloxycarbonylamino-2,6-didesoxy-a-D-glucopyranosid in 30 ml Tetrahydrofuran versetzt und 3,5 Stunden bei Raumtemperatur gerührt. Nach Zugabe von 0,5 g 1,2-O-Isopropyliden-a-D-glucofuranurono-6,3-lacton wurde nochmals 7 Stunden bei 50 °C gerührt und dann eingeengt. Der Rückstand wurde bei 0°C in 15 ml Pyridin gelöst, mit 2,64 ml Benzoylchlorid versetzt und 18 Stunden bei Raumtemperatur gehalten. Die Reaktionslösung wurde auf Eis/ Wasser gegossen und mit Essigester extrahiert. Die organischen Phasen wurden mit Wasser gewaschen, über Natriumsulfat getrocknet und nach Einengen mit Toluol/ Essigester über Kieselgel chromatographiert. Die Produktfraktionen ergaben Benzyl-3,4-di-O-benzoyl-6-[(3,5-di-O-benzoyl-1,2-O-isopropyliden-a-D-glucofuranuronyl)-amino]-2-benzyloxycarbonylamino-2,6-didesoxy-a-D-glucopyranosid, $[\alpha]_D^{20}$ +14,0° (*c* 0,5; Dioxan), MS: *m/z* 1035,6 ([M + H]$^+$).

E. Eine Lösung von 0,75 g Benzyl-3,4-di-O-benzoyl-6-[(3,5-di-O-benzoyl-1,2-O-isopropyliden-a-D-glucofuranuronyl)-amino]-2-benzyloxycarbonylamino-2,6-didesoxy-a-D-glucopyranosid in 10 ml Methanol wurde in Gegenwart von Palladium auf Kohle (10 %) bei Raumtemperatur hydriert. Nach 5 Stunden wurde vom Katalysator abfiltriert, eingeengt und mit Hexan/ Essigester über Kieselgel chromatographiert. Die Produktfraktionen ergaben Benzyl-2-amino-3,4-di-O-benzoyl-6-(3,5-di-O-benzoyl-1,2-O-isopropyliden-a-D-glucofuranuronyl)-amino-2,6-didesoxy-a-D-glucopyranosid, MS: *m/z* 901,5 ([M + H]$^+$).

F. Eine Lösung von 218 mg Biphenyl-4,4'-dicarbonsäure in 15 ml Tetrahydrofuran und 15 ml Acetonitril wurde bei -10°C mit 0,279 ml Triethylamin und 0,232 ml Chlorameisensäureisobutylester versetzt und 30 Minuten bei dieser Temperatur gerührt. Dann wurde eine Lösung von 1,62 g Benzyl-2-amino-3,4-di-O-benzoyl-6-[(3,5-di-O-benzoyl-1,2-O-isopropyliden-a-D-glucofuranuronyl)-amino]-2,6-didesoxy-a-D-glucopyranosid in 10 ml Tetrahydrofuran und 10 ml Acetonitril zugegeben und 5 Stunden bei Raumtemperatur gerührt. Nach dem Einengen wurde der Rückstand mit Dichlormethan/ Essigester über Kieselgel chromatographiert und ergab Biphenyl-4,4'-dicarbonsäure-bis-[[benzyl-3,4-di-O-benzoyl-6-[(3,5-di-O-benzoyl-1,2-O-isopropyliden-a-D-glucofuranuronyl)-amino]-2,6-didesoxy-a-D-glucopyranosid-2-yl]-amid], $[\alpha]_D^{20}$ +80,0° (*c* 0,2; Dioxan), MS: *m/z* 2029,6 ([M + Na]$^+$).

G. Eine Lösung von 450 mg Biphenyl-4,4'-dicarbonsäure-bis-[[benzyl-3,4-di-O-benzoyl-6-[(3,5-di-O-benzoyl-1,2-O-isopropyliden-a-D-glucofuranuronyl)-amino]-2,6-didesoxy-a-D-glucopyranosid-2-yl]-amid] in 8 ml Methanol und 4 ml Dioxan wurde mit 0,5 ml einer 0,3 m Natriummethanolatlösung 4 Stunden bei Raumtemperatur gerührt. Der entstandene Niederschlag wurde in Methanol gelöst. Die Lösung wurde mit saurem Ionenaustauscher (Amberlite IR 120 H$^+$) neutral gestellt und eingeengt. Kristallisation aus Methanol ergab Biphenyl-4,4'-dicarbonsäure-bis-[[benzyl-2,6-didesoxy-6-(1,2-O-isopropyliden-a-D-glucofuranuronyl)-amino]-a-D-glucopyranosid-2-yl]-amid], $[\alpha]_D^{20}$ +76,0° (*c* 0,2; Dimethylformamid), MS: *m/z* 1175,4 ([M + H]$^+$).

H. Eine Lösung von 260 mg Biphenyl-4,4'-dicarbonsäure-bis-[[benzyl-2,6-didesoxy-6-(1,2-O-isopropyliden-a-D-glucofuranuronyl)-amino]-a-D-glucopyranosid-2-yl]-amid] in 5 ml Dimethylformamid wurde mit 493 mg Schwefeltrioxid-Trimethylaminkomplex versetzt und 24 Stunden bei 70°C gerührt. Dann wurde mit 5,8 ml 10 %iger wässriger Natriumacetatlösung versetzt und eingedampft. Der Rückstand wurde durch Chromatographie über Sephadex LH 20 und C25 (Na$^+$) gereinigt und ergab Biphenyl-4,4'-dicarbonsäure-bis-[[benzyl-2,6-didesoxy-6-[(1,2-O-isopropyliden-3,5-di-O-sulfo-a-D-glucofuranuronyl)-amino]-3,4-di-O-sulfo-a-D-glucopyranosid-2-yl]-amid] Oktanatriumsalz, $[\alpha]_D^{20}$ +61,5° (*c* 0,2; Wasser), MS: *m/z* 1991 (rekonstruiertes M).

<u>Beispiel 10</u>

A. Eine Lösung von 2,0 g Benzyl-6-amino-2-benzyloxycarbonylamino-2,6-didesoxy-a-D-glucopyranosid (vgl. Bsp. 9.C.) in 30 ml Tetrahydrofuran wurde bei 8 °C mit einer Lösung von 1,2 g Methyl-b-D-glucofuranosidurono-6,3-lacton (Osman et al., J. Am. Chem Soc. **73**, 2726 (1951)) in 20 ml Tetrahydrofuran und 10 ml Acetonitril versetzt und unter Argon 3,5 Stunden bei 55°C gerührt. Nach Zugabe von total 1,8 g Methyl-b-D-glucofuranosidurono-6,3-lacton wurde insgesamt 4 Tage bei 55 °C gerührt und dann eingeengt. Der Rückstand wurde bei 0°C in 30 ml Pyridin gelöst, mit 6,88 ml Benzoylchlorid versetzt und 30 Minuten bei Raumtemperatur gehalten. Die Reaktionslösung

wurde mit Eis/ Wasser versetzt und mit Essigester extrahiert. Die organischen Phasen wurden mit Wasser gewaschen, über Natriumsulfat getrocknet und nach Einengen mit Hexan/Essigester über Kieselgel chromatographiert. Die Produktfraktionen ergaben Benzyl-3,4-di-O-benzoyl-2-benzyloxycarbonylamino-2,6-didesoxy-6-[(methyl-2,3,5-tri-O-benzoyl-b-D-glucofuranosiduronyl)-amino]-a-D-glucopyranosid, $[\alpha]^{20}_{D}$ +27,5° (*c* 0,15; Dioxan), MS: *m/z* 1113 ($[M + H]^+$).

B. Eine Lösung von 2,6 g Benzyl-3,4-di-O-benzoyl-2-benzyloxycarbonylamino-2,6-didesoxy-6-[(methyl-2,3,5-tri-O-benzoyl-b-D-glucofuranosiduronyl)-amino]-a-D-glucopyranosid in 20 ml Tetrahydrofuran und 2,5 ml Wasser wurde in Gegenwart von Palladium auf Kohle (10 %) bei Raumtemperatur hydriert. Nach 4 Stunden wurde über Filterhilfe vom Katalysator abfiltriert, eingeengt und mit Hexan/ Essigester/ 2 % Triethylamin über Kieselgel chromatographiert. Die Produktfraktionen ergaben Benzyl-2-amino-3,4-di-O-benzoyl-2,6-didesoxy-6-[(methyl-2,3,5-tri-O-benzoyl-b-D-glucofuranosid-uronyl)-amino]-a-D-glucopyranosid, $[\alpha]^{20}_{D}$ +37,5° (*c* 0,15; Dioxan), MS: *m/z* 979,5 ($[M + H]^+$).

C. Reaktion von Benzyl-2-amino-3,4-di-O-benzoyl-2,6-didesoxy-6-[(methyl-2,3,5-tri-O-benzoyl-b-D-glucofuranosiduronyl)-amino]-a-D-glucopyranosid mit 4,4'-Biphenyldicarbonsäure wie in Bsp. 9.F. beschrieben ergab Biphenyl-4,4'-dicarbonsäure-bis-[[benzyl-2,6-didesoxy-3,4-di-O-benzoyl-6-[(methyl-2,3,5-tri-O-benzoyl-b-D-glucofuranosiduronyl)-amino]-a-D-glucopyranosid-2-yl]-amid], $[\alpha]^{20}_{D}$ +100,0° (*c* 0,2; Dioxan), MS: *m/z* 2169,5 ($[M + Na]^+$).

D. Biphenyl-4,4'-dicarbonsäure-bis-[[benzyl-2,6-didesoxy-3,4-di-O-benzoyl-6-[(methyl-2,3,5-tri-O-benzoyl-b-D-glucofuranosiduronyl)-amino]-a-D-glucopyranosid-2-yl]-amid] wurde debenzoyliert wie in Bsp. 9.G. beschrieben und ergab Biphenyl-4,4'-dicarbonsäure-bis-[[benzyl-2,6-didesoxy-6-[(methyl-b-D-glucofuranosiduronyl)-amino]-a-Dglucopyranosid-2-yl]-amid], $[\alpha]^{20}_{D}$ +48,0° (*c* 0,2; Dioxan), MS: *m/z* 1124,4 ($[M + H]^+$).

E. Sulfatierung von Biphenyl-4,4'-dicarbonsäure-bis-[[benzyl-2,6-didesoxy-6-[(methyl-b-D-glucofuranosiduronyl)-amino]-a-D-glucopyranosid-2-yl]-amid] wie in Bsp. 9.H. beschrieben ergab Biphenyl-4,4'-dicarbonsäure-bis-[[benzyl-2,6-didesoxy-6-[(methyl-2,3,5-tri-O-sulfo-b-D-glucofuranosiduronyl)-amino]-3,4-di-O-sulfo-a-D-glucopyranosid-2-yl]-amid] Dekanatriumsalz, $[\alpha]^{20}_{D}$ +56,0° (*c* 0,2; Wasser), MS: *m/z* 2143 (rekonstruiertes M).

Beispiel 11

A. Eine Suspension von 20,0 g Benzyl-4,6-(R)-O-benzyliden-2-benzyloxycarbonylamino-2-desoxy-a-D-glucopyranosid (Wyss und Kiss, Helv. Chim. Acta **58**, 1833 (1975)) in 200 ml Dioxan wurde bei Raumtemperatur mit 6,0 g pulverisiertem Kaliumhydroxid versetzt. Innerhalb 10 Minuten wurden 68 ml Bromessigsäure-tert.-butylester zugetropft. Nach 2 Stunden Rühren bei 55 °C wurde das Reaktionsgemisch auf Eis/ wässrige Natriumhydrogenkarbonatlösung gegossen und mit Essigester extrahiert. Die organischen Phasen wurden mit Wasser gewaschen, über Natriumsulfat getrocknet, eingeengt und aus Essigester/ Hexan kristallisiert. Es wurde Benzyl-4,6-(R)-O-benzyliden-2-benzyloxycarbonylamino-3-O-tert.-butoxycarbonylmethyl-2-desoxy-a-D-glucopyranosid erhalten, $[\alpha]^{20}_{D}$ +89,6° (*c* 0,5; Dioxan), MS: *m/z* 628,5 ($[M + Na]^+$).

B. Zu einer Lösung von 5,97 g Kalium-tert.-butylat in 112 ml Ether und 0,25 ml Wasser wurde bei 0°C eine Lösung von 3,73 g Benzyl-4,6-(R)-O-benzyliden-2-benzyloxycarbonylamino-3-O-tert.-butoxycarbonylmethyl-2-desoxy-a-D-glucopyranosid in 78 ml Ether getropft und dann während 18 Stunden unter Rückfluss erhitzt. Nach Abkühlen wurde mit 1 n HCl angesäuert (pH = 5), mit Eis/ Wasser versetzt und mit Dichlormethan extrahiert. Die organischen Phasen wurden mit Wasser gewaschen, über Magnesiumsulfat getrocknet, eingeengt und aus Tetrahydrofuran/ Hexan kristallisiert. Es wurde Benzyl-4,6-(R)-O-benzyliden-2-benzyloxycarbonylamino-3-O-carboxymethyl-2-desoxy-a-D-glucopyranosid erhalten, $[\alpha]^{20}_{D}$ +103,2° (*c* 0,5; Dioxan), MS: *m/z* 548,2 ($[M - H]^-$).

C. Eine Lösung von 4,45 g Benzyl-4,6-(R)-O-benzyliden-2-benzyloxycarbonylamino-3-O-carboxymethyl-2-desoxy-a-D-glucopyranosid in 60 ml Tetrahydrofuran und 60 ml Acetonitril wurde bei -10°C mit 2,49 ml Triethylamin und 2,1 ml Chlorameisensäureisobutylester versetzt und 15 Minuten bei dieser Temperatur gerührt. Dann wurde eine Lösung von 1,62 g Benzyl-2-amino-2-desoxy-a-D-glucopyranosid in 10 ml Tetrahydrofuran und 10 ml Acetonitril zugegeben und 3 Stunden bei Raumtemperatur gerührt. Das Reaktionsgemisch wurde eingeengt und mit 10 ml Acetanhydrid in 20 ml Pyridin 18 Stunden bei Raumtemperatur acetyliert. Dann wurde eingeengt und mittels Chromatographie mit Essigester/ Hexan über Kieselgel gereinigt. Es wurde Benzyl-3-O-[(benzyl-3,4,6-tri-O-acetyl-2-desoxy-a-D-glucopyranosid)-2-ylcarbamoylmethyl]-4,6-(R)-O-benzyliden-2-benzyloxycarbonylamino-2-desoxy-a-D-glucopyranosid erhalten, $[\alpha]^{20}_{D}$ +107,4° (*c* 0,5; Dioxan), MS: *m/z* 927,6 ($[M + H]^+$).

D. Eine Lösung von 4,5 g Benzyl-3-O-[(benzyl-3,4,6-tri-O-acetyl-2-desoxy-a-D-glucopyranosid)-2-ylcarbamoylmethyl]-4,6-(R)-O-benzyliden-2-benzyloxycarbonylamino-2-desoxy-a-D-glucopyranosid in 45 ml Tetrahydrofuran wurde in Gegenwart von Palladium auf Kohle (10 %) bei Raumtemperatur hydriert. Nach 5 Stunden wurde über Filterhilfe vom Katalysator abfiltriert und eingeengt. So wurde Benzyl-2-amino-3-O-[(benzyl-3,4,6-tri-O-acetyl-2-desoxy-a-D-glucopyranosid)-2-ylcarbamoylmethyl]-4,6-(R)-O-benzyliden-2-desoxy-a-D-glucopyranosid erhalten, $[\alpha]^{20}_{D}$ +146,0° (*c* 0,2; Dioxan), MS: *m/z* 793,3 ($[M + H]^+$).

E. Reaktion von Benzyl-2-amino-3-O-[(benzyl-3,4,6-tri-O-acetyl-2-desoxy-a-D-glucopyranosid)-2-ylcarbamoylmethyl]-4,6-(R)-O-benzyliden-2-desoxy-a-D-glucopyranosid mit Biphenyl-4,4'-dicarbonsäure wie in Bsp. 9.F.

beschrieben ergab Biphenyl-4,4'-dicarbonsäure-bis-[[benzyl-3-O-[(benzyl-3,4,6-tri-O-acetyl-2-desoxy-a-D-glucopyranosid)-2-ylcarbamoylmethyl]-4,6-(R)-O-benzyliden-2-desoxy-a-D-glucopyranosid-2-yl]-amid], $[\alpha]_D^{20}$ +114,0° (*c* 0,2; Chloroform).

F. Eine Lösung von 650 mg Biphenyl-4,4'-dicarbonsäure-bis-[[benzyl-3-O-[(benzyl-3,4,6-tri-O-acetyl-2-desoxy-a-D-glucopyranosid)-2-ylcarbamoyl-methyl]-4,6-(R)-O-benzyliden-2-desoxy-a-D-glucopyranosid-2-yl]-amid] in 15 ml Methanol wurde mit 1,0 ml einer 0,3 m Natriummethanolatlösung 4 Stunden bei Raumtemperatur gerührt. Der entstandene Niederschlag wurde mit Methanol gewaschen und getrocknet und ergab Biphenyl-4,4'-dicarbonsäure-bis-[[benzyl-3-O-[(benzyl-2-desoxy-a-D-glucopyranosid)-2-ylcarbamoylmethyl]-4,6-(R)-O-benzyliden-2-desoxy-a-D-glucopyranosid-2-yl]-amid], $[\alpha]_D^{20}$ +136,0° (*c* 0,2; Dimethylsulfoxid).

G. Sulfatierung von Biphenyl-4,4'-dicarbonsäure-bis-[[benzyl-3-O-[(benzyl-2-desoxy-a-D-glucopyranosid)-2-ylcarbamoylmethyl]-4,6-(R)-O-benzyliden-2-desoxy-a-D-glucopyranosid-2-yl]-amid] wie in Bsp. 9.H. beschrieben ergab (Biphenyl-4,4'-dicarbonsäure)-bis-[[benzyl-3-O-[(benzyl-2-desoxy-2,3,4-tri-O-sulfo-a-D-glucopyranosid)-2-ylcarbamoylmethyl]-4,6-(R)-O-benzyliden-2-desoxy-a-D-glucopyranosid-2-yl]-amid] Hexanatriumsalz, $[\alpha]_D^{20}$ +68,0° (*c* 0,2; Dimethylsulfoxid).

Beispiel 12

A. Eine Lösung von 300 mg Biphenyl-4,4'-dicarbonsäure-bis-[[benzyl-3-O-[(benzyl-3,4,6-tri-O-acetyl-2-desoxy-a-D-glucopyranosid)-2-ylcarbamoyl-methyl]-4,6-(R)-O-benzyliden-2-desoxy-a-D-glucopyranosid-2-yl]-amid] in 10 ml Chloroform und 10 ml Dioxan wurde in Gegenwart von Palladium auf Kohle (10 %) bei Raumtemperatur hydriert. Nach 3 Tagen wurde über Filterhilfe vom Katalysator abfiltriert und eingeengt. Chromatographie mit Essigester/ Methanol/ Wasser über Kieselgel ergab Biphenyl-4,4'-dicarbonsäure-bis-[[benzyl-3-O-[(benzyl-3,4,6-tri-O-acetyl-2-desoxy-a-D-glucopyranosid-2-ylcarbamoylmethyl)-2-desoxy-a-D-glucopyranosid-2-yl]-amid], $[\alpha]_D^{20}$ +118,5° (*c* 0,2; Chioroform), MS: *m/z* 1615,5 ($[M + H]^+$).

B. Eine Lösung von Biphenyl-4,4'-dicarbonsäure-bis-[[benzyl-3-O-[(benzyl-3,4,6-tri-O-acetyl-2-desoxy-a-D-glucopyranosid-2-ylcarbamoylmethyl)-2-desoxy-a-D-glucopyranosid-2-yl]-amid] in Methanol/ Dioxan 2:1 wurde deacetyliert wie in Bsp. 11.F. beschrieben und ergab Biphenyl-4,4'-dicarbonsäure-bis-[[benzyl-3-O-(benzyl-2-desoxy-a-D-glucopyranosid-2-ylcarbamoylmethyl)-2-desoxy-a-D-glucopyranosid-2-yl]-amid], MS: *m/z* 1364,6 ($[M + H]^+$).

C. Sulfatierung von Biphenyl-4,4'-dicarbonsäure-bis-[[benzyl-3-O-(benzyl-2-desoxy-a-D-glucopyranosid-2-ylcarbamoylmethyl)-2-desoxy-a-D-glucopyranosid-2-yl]-amid] wie in Bsp. 9.H. beschrieben ergab Biphenyl-4,4'-dicarbonsäure-bis-[[benzyl-3-O-(benzyl-2-desoxy-3,4,6-tri-O-sulfo-a-D-glucopyranosid-2-ylcarbamoylmethyl)-2-desoxy-4,6-di-O-sulfo-a-D-glucopyranosid-2-yl]-amid] Dekanatriumsalz, $[\alpha]_D^{20}$ +73,5° (*c* 0,2; Wasser), MS: *m/z* 2384 (rekonstruiertes M).

Beispiel 13

A. Eine Lösung von 3,0 g Benzyl-4,6-(R)-O-benzyliden-2-benzyloxycarbonylamino-3-O-carboxymethyl-2-desoxy-a-D-glucopyranosid (vgl. Bsp. 11.B.) in 20 ml Tetrahydrofuran wurde mit 65 mg N-Hydroxysuccinimid und danach mit einer Lösung von 1,09 g Glucamin in 10 mi Wasser bei Raumtemperatur versetzt. Dann wurde eine Lösung von 1,24 g Dicyclohexylcarbodiimid in 5 ml Tetrahydrofuran zugesetzt und 18 Stunden gerührt. Das Reaktionsgemisch wurde eingeengt und der Rückstand mit 25 ml Acetanhydrid in 50 ml Pyridin während 18 Stunden acetyliert. Das Reaktionsgemisch wurde wiederum eingeengt, mit Essigester/ Toluol über Kieselgel chromatographiert und ergab Benzyl-3-O-(2,3,4,5,6-penta-O-acetyl-D-glucit-1-ylcarbamoylmethyl)-4,6-(R)-O-benzyliden-2-benzyloxycarbonylamino-2-desoxy-a-D-glucopyranosid, $[\alpha]_D^{20}$ +28,5° (*c* 0,2; Dioxan), MS: *m/z* 923,2 ($[M + H]^+$).

B. Eine Lösung von 250 mg Benzyl-3-O-(2,3,4,5,6-penta-O-acetyl-D-glucit-1-ylcarbamoylmethyl)-4,6-(R)-O-benzyliden-2-benzyloxycarbonylamino-2-desoxy-a-D-glucopyranosid in 20 ml Dioxan und 2 ml Wasser wurde in Gegenwart von Palladium auf Kohle (10 %) bei Raumtemperatur hydriert. Nach 2 Stunden wurde über Filterhilfe vom Katalysator abfiltriert und eingeengt. Es wurde nach Chromatographie mit Essigester/ Methanol/Wasser/ Triethylamin über Kieselgel Benzyl-2-amino-3-O-(2,3,4,5,6-penta-O-acetyl-D-glucit-1-ylcarbamoylmethyl)-4,6-(R)-O-benzyliden-2-desoxy-a-D-glucopyranosid erhalten, MS: *m/z* 789,3 ($[M + H]^+$).

C. Reaktion von Benzyl-2-amino-3-O-(2,3,4,5,6-penta-O-acetyl-D-glucit-1-ylcarbamoylmethyl)-4,6-(R)-O-benzyliden-2-desoxy-a-D-glucopyranosid mit Terephthalsäure wie in Bsp. 9.F. beschrieben ergab N,N'-Bis-[benzyl-3-O-(2,3,4,5,6-penta-O-acetyl-D-glucit-1-ylcarbamoylmethyl)-4,6-(R)-O-benzyliden-2-desoxy-a-D-glucopyranosid-2-yl]-terephthalamid, MS: *m/z* 873,6 ($[M + H + K]^{2+}/2$).

D. Eine Lösung von 300 mg N,N'-Bis-[benzyl-3-O-(2,3,4,5,6-penta-O-acetyl-D-glucit-1-ylcarbamoylmethyl)-4,6-(R)-O-benzyliden-2-desoxy-a-D-glucopyranosid-2-yl]-terephthalamid in 10 ml Methanol und 5 ml Dichlormethan wurde mit 2 ml einer 0,3 m Natriummethanolatlösung 18 Stunden bei Raumtemperatur gerührt. Der entstandene Niederschlag wurde durch Zentrifugation abgetrennt, mit Methanol gewaschen, getrocknet und ergab Terephthalsäure-bis-[[benzyl-4,6-O-benzyliden-2-desoxy-3-O-(D-glucit-1-ylcarbamoylmethyl)-a-D-glucopyranosid-2-yl]-amid]. Dieses Produkt wurde sulfatiert wie in Bsp. 9.H. beschrieben und ergab 1,4-Bis-[benzyl-4,6-(R)-O-benzyliden-2-desoxy-3-O-(2,3,4,5,6-penta-O-sulfo-D-glucit-1-ylcarbamoylmethyl)-a-D-glucopyranosid-2-yl]-terephthalamid Dekanatriumsalz, $[\alpha]_D^{20}$ +35,5° (*c* 0,2; Wasser), MS: *m/z* 2308 (rekonstruiertes M).

Beispiel 14

A. Eine Suspension von 3,0 g Benzyl-6-amino-2-benzyloxycarbonylamino-2,6-didesoxy-a-D-glucopyranosid (vgl. Bsp. 9.C.) und 1,33 g D-Gluconsäure-γ-lacton in 60 ml Dioxan wurde bei 80°C in Lösung gebracht und für weitere 18 Stunden bei dieser Temperatur gehalten. Das ausgefallene Produkt wurde abgenutscht, mit Dioxan gewaschen, getrocknet und ergab D-Gluconsäure(benzyl-2-benzyloxycarbonylamino-2,6-didesoxy-a-D-glucopyranosid-6-yl)-amid, $[\alpha]_D^{20}$ +120,0° (*c* 0,2; Dioxan), MS: *m/z* 603,2 ($[M + Na]^+$).

B. Eine Lösung von 1,4 g D-Gluconsäure-(benzyl-2-benzyloxycarbonylamino-2,6-didesoxy-a-D-glucopyranosid-6-yl)-amid in 5 ml Pyridin wurde mit 2 ml Acetanhydrid während 18 Stunden bei Raumtemperatur acetyliert, eingeengt und ergab 2,3,4,5,6-Penta-O-acetyl-D-gluconsäure(benzyl-3,4-di-O-acetyl-2-benzyloxycarbonylamino-2,6-didesoxy-a-D-glucopyranosid-6-yl)-amid, $[\alpha]_D^{20}$ +84,0° (*c* 0,2; Dioxan), MS: *m/z* 897,3 ($[M + Na]^+$).

C. 2,3,4,5,6-Penta-O-acetyl-D-gluconsäure-(benzyl-3,4-di-O-acetyl-2-benzyloxycarbonylamino-2,6-didesoxy-a-D-glucopyranosid-6-yl)-amid wurde hydriert wie unter Bsp. 13.B. beschrieben. Das Reaktionsprodukt wurde aus Essigester/ Ether kristallisiert und ergab 2,3,4,5,6-Penta-O-acetyl-D-gluconsäure-(benzyl-3,4-di-O-acetyl-2-amino-2,6-didesoxy-a-D-glucopyranosid-6-yl)-amid, $[\alpha]_D^{20}$ +96,0° (*c* 0,2; Dioxan), MS: *m/z* 741,1 ($[M + H]^+$).

D. Reaktion von 2,3,4,5,6-Penta-O-acetyl-D-gluconsäure-(benzyl-3,4-di-O-acetyl-2-amino-2,6-didesoxy-a-D-glucopyranosid-6-yl)-amid mit 4,4'-Biphenyldicarbonsäure wie in Bsp. 9.F. beschrieben ergab Biphenyl-4,4'-dicarbonsäure-bis-[[benzyl-3,4-di-O-acetyl-2,6-didesoxy-6-(2,3,4,5,6-penta-O-acetyl-D-gluconoylamino)-a-D-glucopyranosid-2-yl] -amid], $[\alpha]_D^{20}$ +123,0° (*c* 0,2; Dioxan), MS: *m/z* 1687,5 ($[M + H]^+$).

E. Biphenyl-4,4'-dicarbonsäure-bis-[[benzyl-3,4-di-O-acetyl-2,6-didesoxy-6-(2,3,4,5,6-penta-O-acetyl-D-gluconoylamino)-a-D-glucopyranosid-2-yl]-amid] wurde deacetyliert wie in Bsp. 11.F. beschrieben und ergab Biphenyl-4,4'-dicarbonsäure-bis-[(benzyl-2,6-didesoxy-6-D-gluconoylamino-a-D-glucopyranosid-2-yl)-amid], MS: *m/z* 1099,5 ($[M + H]^+$).

F. Sulfatierung von Biphenyl-4,4'-dicarbonsäure-bis-[(benzyl-2,6-didesoxy-6-D-gluconoylamino-a-D-glucopyranosid-2-yl)-amid] wie in Bsp. 9.H. beschrieben ergab Biphenyl-4,4'-dicarbonsäure-bis-[[benzyl-2,6-didesoxy-3,4-di-O-sulfo-6-(2,3,4,5,6-penta-O-sulfo-D-gluconoylamino)-a-D-glucopyranosid-2-yl]-amid] Tetradekanatriumsalz, $[\alpha]_D^{20}$ +73,0° (*c* 0,2; Wasser), MS: *m/z* 2527 (rekonstruiertes M).

Beispiel 15

Eine Lösung von 200 mg Biphenyl-4,4'-dicarbonsäure-bis-[[benzyl-2,6-didesoxy-3,4-di-O-sulfo-6-(2,3,4,5,6-penta-O-sulfo-D-gluconoylamino)-a-D-glucopyranosid-2-yl]-amid] Tetradekanatriumsalz (vgl. Bsp. 14.F.) in 12 ml 2 n Natriumhydroxid wurde 8 Stunden bei Raumtemperatur belassen, mit saurem Ionenaustauscher (Amberlite IR 120 $H^+$) neutralisiert und eingeengt. Der Rückstand wurde mit Wasser über Sephadex C25 ($Na^+$) chromatographiert und ergab Biphenyl-4,4'-dicarbonsäure-bis- [[(Z)-benzyl-2,6-didesoxy-3,4-di-O-sulfo-6-(3-desoxy-2,4,5,6-tetra-O-sulfo-D-erythro-hex-2-enonoylamino)-a-D-glucopyranosid-2-yl]-amid] Dodekanatriumsalz, $[\alpha]_D^{20}$ +67,5° (*c* 0,2; Wasser), MS: *m/z* 2288 (rekonstruiertes M).

Beispiel 16

A. Benzyl-6-amino-2-benzyloxycarbonylamino-2,6-didesoxy-a-D-glucopyranosid (vgl. Bsp. 9.C.) und D-Ribonsäure-γ-lacton wurden umgesetzt wie in Bsp. 14.A. beschrieben und ergab D-Ribonsäure-(benzyl-2-benzyloxycarbonylamino-2,6-didesoxy-a-D-glucopyranosid-6-yl)-amid, $[\alpha]_D^{20}$ +81,5° (*c* 0,2; Dioxan), MS: *m/z* 551,4 ($[M + H]^+$).

B. D-Ribonsäure-(benzyl-2-benzyloxycarbonylamino-2,6-didesoxy-a-D-glucopyranosid-6-yl)-amid wurde acetyliert wie in Bsp. 14.B. beschrieben und ergab 2,3,4,5-Tetra-O-acetyl-D-ribonsäure-(benzyl-3,4-di-O-acetyl-2-benzyloxycarbonylamino-2,6-didesoxy-a-D-glucopyranosid-6-yl)-amid, $[\alpha]_D^{20}$ +78,0° (*c* 0,2; Dioxan), MS: *m/z* 803,2 ($[M + H]^+$).

C. 2,3,4,5-Tetra-O-acetyl-D-ribonsäure-(benzyl-3,4-di-O-acetyl-2-benzyloxycarbonylamino-2,6-didesoxy-a-D-glucopyranosid-6-yl)-amid wurde hydriert wie unter Bsp. 13.B. beschrieben und ergab 2,3,4,5-Tetra-O-acetyl-D-ribonsäure-(benzyl-3,4-di-O-acetyl-2-amino-2,6-didesoxy-a-D-glucopyranosid-6-yl)-amid, MS: *m/z* 669,2 ($[M + H]^+$).

D. Eine Suspension von 417 mg Biphenyl-4,4'-dicarbonsäure in 10 ml Tetrahydrofuran und 10 ml Acetonitril wurde bei Raumtemperatur mit 0,397 ml N-Methylmorpholin und 604 mg 2-Chlor-2,4-dimethoxy-1,3,5-triazin versetzt. Nach 30 Minuten wurde eine Lösung von 2,3,4,5-Tetra-O-acetyl-D-ribonsäure-(benzyl-3,4-di-O-acetyl-2-amino-2,6-didesoxy-a-D-glucopyranosid-6-yl)-amid in 7,5 ml Tetrahydrofuran und 7,5 ml Acetonitril zugegeben und 2 Stunden gerührt. Dann wurde von ungelösten Bestandteilen abfiltriert und eingeengt. Man erhielt Biphenyl-4,4'-dicarbonsäure-bis-[[benzyl-3,4-di-O-acetyl-2,6-didesoxy-6-(2,3,4,5,6-penta-O-acetyl-D-ribonoylamino)-a-D-glucopyranosid-2-yl]-amid] , $[\alpha]_D^{20}$ +127,0° (*c* 0,2; Dioxan), MS: *m/z* 1562,5 ($[M + NH_4]^+$).

E. Biphenyl-4,4'-dicarbonsäure-bis-[[benzyl-3,4-di-O-acetyl-2,6-didesoxy-6-(2,3,4,5,6-penta-O-acetyl-D-ribonoylamino)-a-D-glucopyranosid-2-yl]-amid] wurde deacetyliert wie in Bsp. 11.F. beschrieben und ergab Biphenyl-4,4'-dicarbonsäure-bis-[(benzyl-2,6-didesoxy-6-D-ribonoylamino-a-D-glucopyranosid-2-yl)-amid], MS: *m/z* 1057,2 ($[M + Na]^+$).

F. Sulfatierung von Biphenyl-4,4'-dicarbonsäure-bis-[(benzyl-2,6-didesoxy-6-D-ribonoylamino-a-D-glucopyranosid-2-yl)-amid] wie in Bsp. 9.H. beschrieben ergab Biphenyl-4,4'-dicarbonsäure-bis-[[benzyl-2,6-didesoxy-3,4-di-O-sulfo-6-(2,3,4,5-tetra-O-sulfo-D-ribonoylamino)-a-D-glucopyranosid-2-yl]-amid] Dodekanatriumsalz, $[\alpha]_D^{20}$ +91,0° (*c* 0,2; Wasser), MS: *m/z* 2264 (rekonstruiertes M).

Beispiel 17

A. Benzyl-6-amino-2-benzyloxycarbonylamino-2,6-didesoxy-a-D-glucopyranosid (vgl. Bsp. 9.C.) und 2-(R)-Hydroxy-3,3-dimethyl-γ-butyrolacton wurden umgesetzt wie in Bsp. 14.A. beschrieben. Nach Zugabe von 30 % des Lactons wurde nochmals 1 Tag bei 80°C gerührt, das Rohprodukt wurde acetyliert wie in Bsp. 14.B. beschrieben und ergab (R)-2,4-Diacetoxy-3,3-dimethyl-buttersäure-(benzyl-3,4-di-O-acetyl-2-benzyloxycarbonylamino-2,6-didesoxy-a-D-glucopyranosid-6-yl)-amid, $[\alpha]_D^{20}$ +99,0° (*c* 0,2; Dioxan), MS: *m/z* 701,2 ($[M + H]^+$).

B. (R)-2,4-Diacetoxy-3,3-dimethyl-buttersäure-(benzyl-3,4-di-O-acetyl-2-benzyloxycarbonylamino-2,6-didesoxy-a-D-glucopyranosid-6-yl)-amid wurde hydriert wie unter Bsp. 13.B. beschrieben und ergab (R)-2,4-Diacetoxy-3,3-dimethyl-buttersäure-(benzyl-3,4-di-O-acetyl-2-amino-2,6-didesoxy-a-D-glucopyranosid-6-yl)-amid, MS: *m/z* 657,5 ($[M + H]^+$).

C. Reaktion von (R)-2,4-Diacetoxy-3,3-dimethylbuttersäure-(benzyl-3,4-di-O-acetyl-2-amino-2,6-didesoxy-a-D-glucopyranosid-6-yl)-amid mit Biphenyl-4,4'-dicarbonsäure wie in Bsp. 16.D. beschrieben ergab Biphenyl-4,4'-dicarbonsäure-bis-[[benzyl-3,4-di-O-acetyl-6-((R)-2,4-diacetoxy-3,3-dimethyl-butyrylamino)-2,6-didesoxy-a-D-giucopyranosid-2-yl]-amid], $[\alpha]_D^{20}$ +140,0° (*c* 0,2; Dioxan), MS: *m/z* 1361,5 ($[M + Na]^+$).

D. Biphenyl-4,4'-dicarbonsäure-bis-[[benzyl-3,4-di-O-acetyl-6-((R)-2,4-diacetoxy-3,3-dimethyl-butyrylamino)-2,6-didesoxy-a-D-glucopyranosid-2-yl]-amid] wurde deacetyliert wie in Bsp. 11.F. beschrieben und ergab Biphenyl-4,4'-dicarbonsäure-bis-[[benzyl-2,6-didesoxy-6-((R)-2,4-dihydroxy-3,3-dimethyl-butyrylamino)-a-D-glucopyranosid-2-yl]-amid], $[\alpha]_D^{20}$ +119,0° (*c* 0,2; Dioxan), MS: *m/z* 1025,8 ($[M + Na]^+$).

E. Sulfatierung von Biphenyl-4,4'-dicarbonsäure-bis-[[benzyl-2,6-didesoxy-6-((R)-2,4-dihydroxy-3,3-dimethyl-butyrylamino)-a-D-glucopyranosid-2-yl]-amid] wie in Bsp. 9.H. beschrieben ergab Biphenyl-4,4'-dicarbonsäure-bis-[[benzyl-2,6-didesoxy-3,4-di-O-sulfo-6-((R)-2,4-bis-hydroxysulfonyloxy-3,3-dimethyl-butyrylamino)-a-D-glucopyranosid-2-yl]-amid] Oktanatriumsalz, $[\alpha]_D^{20}$ +106,5° (*c* 0,2; Wasser), MS: *m/z* 1820 (rekonstruiertes M).

Beispiel 18

A. Benzyl-6-amino-2-benzyloxycarbonylamino-2,6-didesoxy-a-D-glucopyranosid (vgl. Bsp. 9.C.) und (S)-g-Hydroxymethyl-γ-butyrolacton wurden umgesetzt wie in Bsp. 14.A. beschrieben. Nach Zugabe von 30 % des Lactons wurde nochmals 1 Tag bei 80°C gerührt, das Rohprodukt wurde aus Essigester/ Methylenchlorid kristallisiert und ergab (S)-4,5-Dihydroxypentansäure-(benzyl-2-benzyloxycarbonylamino-2,6-didesoxy-a-D-glucopyranosid-6-yl)-amid, $[\alpha]_D^{20}$ +73,0° (*c* 0,2; Dioxan), MS: *m/z* 519,5 ($[M + H]^+$).

B. (S)-4,5-Dihydroxy-pentansäure-(benzyl-2-benzyloxycarbonylamino-2,6-didesoxy-a-D-glucopyranosid-6-yl)-amid wurde acetyliert wie in Bsp. 14.B. beschrieben und ergab (S)-4,5-Diacetoxy-pentansäure-(benzyl-3,4-di-O-acetyl-2-benzyloxycarbonylamino-2,6-didesoxy-a-D-glucopyranosid-6-yl)-amid, $[\alpha]_D^{20}$ +89,5° (*c* 0,2; Dioxan), MS: *m/z* 687,2 ($[M + H]^+$).

C. (S)-4,5-Diacetoxy-pentansäure-(benzyl-3,4-di-O-acetyl-2-benzyloxycarbonylamino-2,6-didesoxy-a-D-glucopyranosid-6-yl)-amid wurde hydriert wie unter Bsp. 13.B. beschrieben und ergab (S)-4,5-Diacetoxy-pentansäure-(benzyl-3,4-di-O-acetyl-2-amino-2,6-didesoxy-a-D-glucopyranosid-6-yl)-amid, MS: *m/z* 553,4 ($[M + H]^+$).

D. Reaktion von (S)-4,5-Diacetoxy-pentansäure-(benzyl-3,4-di-O-acetyl-2-amino-2,6-didesoxy-a-D-glucopyranosid-6-yl)-amid mit Biphenyl-4,4'-dicarbonsäure wie in Bsp. 16.D. beschrieben ergab Biphenyl-4,4'-dicarbonsäure-bis-[[benzyl-3,4-di-O-acetyl-6-[(S)-4,5-diacetoxy- pentanoylamino]-2,6-didesoxy-a-D-glucopyranosid-2-yl]-amid], $[\alpha]_D^{20}$ +132,0° (*c* 0,2; Dioxan), MS: *m/z* 1361,5 ($[M + Na]^+$).

E. Biphenyl-4,4'-dicarbonsäure-bis-[[benzyl-3,4-di-O-acetyl-6-[(S)-4,5-diacetoxy-pentanoylamino]-2,6-didesoxy-a-D-glucopyranosid-2-yl]-amid] wurde deacetyliert wie in Bsp. 11.F. beschrieben und ergab Biphenyl-4,4'-dicarbonsäure-bis-[[benzyl-2,6-didesoxy-6-[(S)-4,5-dihydroxy-pentanoylamino]-a-D-glucopyranosid-2-yl]-amid], $[\alpha]_D^{20}$ +92,0° (*c* 0,2; Dioxan), MS: *m/z* 997,9 ($[M + Na]^+$).

F. Sulfatierung von Biphenyl-4,4'-dicarbonsäure-bis-[[benzyl-2,6-didesoxy-6-[(S)-4,5-dihydroxy-pentanoylamino]-a-D-glucopyranosid-2-yl]-amid] wie in Bsp. 9.H. beschrieben ergab Biphenyl-4,4'-dicarbonsäure-bis-[[benzyl-2,6-didesoxy-6-[(S)-4,5-bis-hydroxysalfonyloxy-pentanoylamino]-3,4-di-O-sulfo-a-D-glucopyranosid-2-yl]-amid] Oktanatriumsalz, $[\alpha]_D^{20}$ +93,5° (*c* 0,2; Wasser), MS: *m/z* 1792 (rekonstruiertes M).

Beispiel 19

A. Reaktion von 2,3,4,5,6-Penta-O-acetyl-D-gluconsäure-(benzyl-3,4-di-O-acetyl-2-amino-2,6-didesoxy-a-D-glucopyranosid-6-yl)-amid (vgl. Bsp. 14.C.) mit Terephthalsäure wie in Bsp. 16.D. beschrieben ergab Terephthalsäure-bis-[[benzyl-3,4-di-O-acetyl-2,6-didesoxy-6-(2,3,4,5,6-penta-O-acetyl-D-gluconoylamino)-a-D-glucopyranosid-2-yl]-amid], $[\alpha]_D^{20}$ +96,5° (*c* 0,2; Chloroform), MS: *m/z* 1611,5 ($[M + H]^+$).
B. Eine Lösung von Terephthalsäure-bis-[[benzyl-3,4-di-O-acetyl-2,6-didesoxy-6-(2,3,4,5,6-penta-O-acetyl-D-gluconoylamino)-a-D-glucopyranosid-2-yl]-amid] in Methanol/ Dioxan 2:1 wurde deacetyliert wie in Bsp. 11.F. beschrieben und ergab Terephthalsäure-bis-[(benzyl-2,6-didesoxy-6-D-gluconoylamino-a-D-glucopyranosid-2-yl)-amid], MS: *m/z* 1023,3 ($[M + H]^+$).
C. Sulfatierung von Terephthalsäure-bis-[(benzyl-2,6-didesoxy-6-D-gluconoylamino-a-D-glucopyranosid-2-yl)-amid] wie in Bsp. 9.H. beschrieben ergab Terephthalsäure-bis-[[benzyl-2,6-didesoxy-3,4-di-O-salfo-6-(2,3,4,5,6-penta-O-sulfo-D-gluconoylamino)-a-D-glucopyranosid-2-yl]-amid] Tetradekanatriumsalz, $[\alpha]_D^{20}$ +79,5° (*c* 0,2; Wasser), MS: *m/z* 2452 (rekonstruiertes M).

Beispiel 20

A. Reaktion von 2,3,4,5,6-Penta-O-acetyl-D-gluconsäure-(benzyl-3,4-di-O-acetyl-2-amino-2,6-didesoxy-a-D-glucopyranosid-6-yl)-amid (vgl. Bsp. 14.C.) mit Diphenylmethan-4,4'-dicarbonsaure in Dimethylformamid ergab wie in Bsp. 16.D. beschrieben Diphenylmethan-4,4'-dicarbonsäure-bis-[[benzyl-3,4-di-O-acetyl-6-(2,3,4,5,6-penta-O-acetyl-D-gluconoylamino)-2,6-didesoxy-a-D-glucopyranosid-2-yl]-amid], $[\alpha]_D^{20}$ +100,0° (*c* 0,2; Dioxan), MS: *m/z* 1719,0 ($[M + NH_4]^+$).
B. Diphenylmethan-4,4'-dicarbonsäure-bis-[[benzyl-3,4-di-O-acetyl-6-(2,3,4,5,6-penta-O-acetyl-D-gluconoylamino)-2,6-didesoxy-a-D-glucopyranosid-2-yl]-amid] wurde deacetyliert wie in Bsp. 11.F. beschrieben und ergab Diphenylmethan-4,4'-dicarbonsäure-bis-[(benzyl-2,6-didesoxy-6-D-gluconoylamino-a-D-glucopyranosid-2-yl)-amid], MS: *m/z* 1113 ($[M + H]^+$).
C. Sulfatierung von Diphenylmethan-4,4'-dicarbonsäure-bis-[(benzyl-2,6-didesoxy-6-D-gluconoylamino-a-D-glucopyranosid-2-yl)-amid] wie in Bsp. 9.H. beschrieben ergab Diphenylmethan-4,4'-dicarbonsäure-bis-[[benzyl-2,6-didesoxy-6-(2,3,4,5,6-penta-O-sulfo-D-gluconoylamino)-3,4-di-O-sulfo-a-D-glucopyranosid-2-yl]-amid] Tetradekanatriumsalz, $[\alpha]_D^{20}$ +66,5° (*c* 0,2; Wasser), MS: *m/z* 2542 (rekonstruiertes M).

Beispiel 21

A. Reaktion von 2,3,4,5,6-Penta-O-acetyl-D-gluconsäure-(benzyl-3,4-di-O-acetyl-2-amino-2,6-didesoxy-a-D-glucopyranosid-6-yl)-amid (vgl. Bsp. 14.C.) mit Naphthalin-2,6-dicarbonsäure in Dimethylformamid ergab wie in Bsp. 16.D. beschrieben Naphthalin-2,6-dicarbonsäure-bis-[[benzyl-3,4-di-O-acetyl-6-(2,3,4,5,6-penta-O-acetyl-D-gluconoylamino)-2,6-didesoxy-a-D-glucopyranosid-2-yl]-amid], $[\alpha]_D^{20}$ +117,5° (*c* 0,2; Dioxan), MS: *m/z* 1685,5 ($[M + Na]^+$).
B. Naphthalin-2,6-dicarbonsäure-bis-[[benzyl-3,4-di-O-acetyl-6-(2,3,4,5,6-penta-O-acetyl-D-gluconoylamino)-2,6-didesoxy-a-D-glucopyranosid-2-yl]-amid] wurde deacetyliert wie in Bsp. 11.F. beschrieben und ergab Naphthalin-2,6-dicarbonsäure-bis-[(benzyl-2,6-didesoxy-6-D-gluconoylamino-a-D-glucopyranosid-2-yl)-amid], MS: *m/z* 1095,8 ($[M + Na]^+$).
C. Sulfatierung von Naphthalin-2,6-dicarbonsäure-bis-[(benzyl-2,6-didesoxy-6-D-gluconoylamino-a-D-glucopyranosid-2-yl)-amid] wie in Bsp. 9.H. beschrieben ergab Naphthalin-2,6-dicarbonsäure-bis-[[benzyl-2,6-didesoxy-6-(2,3,4,5,6-penta-O-sulfo-D-gluconoylamino)-3,4-di-O-sulfo-a-D-glucopyranosid-2-yl]-amid] Tetradekanatriumsalz, $[\alpha]_D^{20}$ +76,5° (*c* 0,2; Wasser), MS: *m/z* 2501 (rekonstruiertes M).

Beispiel 22

A. Benzyl-6-amino-2-benzyloxycarbonylamino-2,6-didesoxy-a-D-glucopyranosid (vgl. Bsp. 9.C.) und D-Glucoheptono-1,4-lacton wurden miteinander umgesetzt wie in Bsp. 14.A. beschrieben. Das Reaktionsprodukt wurde acetyliert wie in Bsp. 14.B. beschrieben und ergab 2,3,4,5,6,7-Hexa-O-acetyl-D-glycero-D-guloheptansäure-(benzyl-3,4-di-O-acetyl-2-benzyloxycarbonylamino-2,6-didesoxy-a-D-glucopyranosid-6-yl)-amid, $[\alpha]_D^{20}$ +83,0° (*c* 0,2; Dioxan), MS: *m/z* 969 ($[M + Na]^+$).
B. 2,3,4,5,6,7-Hexa-O-acetyl-D-glycero-D-guloheptansäure-(benzyl-3,4-di-O-acetyl-2-benzyloxycarbonylamino-2,6-didesoxy-a-D-glucopyranosid-6-yl)-amid wurde hydriert wie unter Bsp. 13.B. beschrieben und ergab

2,3,4,5,6,7-Hexa-O-acetyl-D-glycero-D-guloheptansäure-(benzyl-3,4-di-O-acetyl-2-amino-2,6-didesoxy-a-D-glucopyranosid-6-yl)-amid, $[\alpha]_D^{20}$ +92,0° (*c* 0,2; Dioxan), MS: *m/z* 813,3 ([M + H]$^+$).

C. Reaktion von 2,3,4,5,6,7-Hexa-O-acetyl-D-glycero-D-guloheptansäure-(benzyl-3,4-di-O-acetyl-2-amino-2,6-didesoxy-a-D-glucopyranosid-6-yl)-amid mit Naphthalin-2,6-dicarbonsäure in Dimethylformamid wie in Bsp. 16.D. beschrieben ergab Naphthalin-2,6-dicarbonsäure-bis-[[benzyl-3,4-di-O-acetyl-2,6-didesoxy-6-(2,3,4,5,6,7-hexa-O-acetyl-D-glycero-D-gulo-heptanoylamino)-a-D-glucopyranosid-6-yl]-amid], $[\alpha]_D^{20}$ +110,5° (*c* 0,2; Dioxan), MS: *m/z* 1827,4 ([M + Na]$^+$).

D. Naphthalin-2,6-dicarbonsäure-bis-[[benzyl-3,4-di-O-acetyl-2,6-didesoxy-6-(2,3,4,5,6,7-hexa-O-acetyl-D-glycero-D-gulo-heptanoylamino)-a-D-glucopyranosid-6-yl]-amid] wurde deacetyliert wie in Bsp. 11.F. beschrieben und ergab Naphthalin-2,6-dicarbonsäure-bis-[[benzyl-2,6-didesoxy-6-(D-glycero-D-gulo-heptanoylamino)-a-D-glucopyranosid-6-yl] -amid], MS: *m/z* 1157,1 ([M + Na]$^+$).

E. Sulfatierung von Naphthalin-2,6-dicarbonsäure-bis-[[benzyl-2,6-didesoxy-6-(D-glycero-D-gulo-heptanoylamino)-a-D-glucopyranosid-6-yl]-amid] wie in Bsp. 9.H. beschrieben ergab Naphthalin-2,6-dicarbonsäure-bis-[[benzyl-2,6-didesoxy-3,4-di-O-sulfo-6-(2,3,4,5,6,7-hexa-O-sulfo-D-glycero-D-guloheptanoylamino)-a-D-glucopyranosid-6-yl]-amid] Hexadekanatriumsalz, $[\alpha]_D^{20}$ +54,5° (*c* 0,2; Wasser), MS: *m/z* 2765 (rekonstruiertes M).

Beispiel 23

A. Reaktion von 2,3,4,5,6-Penta-O-acetyl-D-gluconsäure-(benzyl-3,4-di-O-acetyl-2-amino-2,6-didesoxy-a-D-glucopyranosid-6-yl)-amid (vgl. Bsp. 14.C.) mit (Z)-Stilben-4,4'-dicarbonsäure in Dimethylformamid ergab wie in Bsp. 16.D. beschrieben (Z)-4,4'-Stilben-4,4'-dicarbonsäure-bis-[[benzyl-3,4-di-O-acetyl-6-(2,3,4,5,6-penta-O-acetyl-D-gluconoylamino)-2,6-didesoxy-a-D-glucopyranosid-2-yl]-amid], $[\alpha]_D^{20}$ +105,5° (*c* 0,2; Dioxan), MS: *m/z* 1736,2 ([M + Na]$^+$).

B. (Z)-4,4'-Stilben-4,4'-dicarbonsäure-bis-[[benzyl-3,4-di-O-acetyl-6-(2,3,4,5,6-penta-O-acetyl-D-gluconoylamino)-2,6-didesoxy-a-D-glucopyranosid-2-yl]-amid] wurde deacetyliert wie in Bsp. 11.F. beschrieben und ergab (Z)-Stilben-4,4'-dicarbonsäure-bis-[(benzyl-2,6-didesoxy-6-D-gluconoylamino-a-D-glucopyranosid-2-yl)-amid], MS: *m/z* 1148,6 ([M + Na]$^+$).

C. Sulfatierung von (Z)-Stilben-4,4'-dicarbonsäure-bis-[(benzyl-2,6-didesoxy-6-D-gluconoylamino-a-D-glucopyranosid-2-yl)-amid] wie in Bsp. 9.H. beschrieben ergab (Z)-Stilben-4,4'-dicarbonsäure-bis-[[benzyl-2,6-didesoxy-6-(2,3,4,5,6-penta-O-sulfo-D-gluconoylamino)-3,4-di-O-sulfo-a-D-glucopyranosid-2-yl]-amid] Tetradekanatriumsalz, $[\alpha]_D^{20}$ +72,5° (*c* 0,2; Wasser), MS: *m/z* 2553 (rekonstruiertes M).

Beispiel 24

A. Reaktion von 2,3,4,5,6-Penta-O-acetyl-D-gluconsäure-(benzyl-3,4-di-O-acetyl-2-amino-2,6-didesoxy-a-D-glucopyranosid-6-yl)-amid (vgl. Bsp. 14.C.) mit Isophthalsäuresäure ergab wie in Bsp. 16.D. beschrieben Isophthalsäuresäure-bis-[[benzyl-3,4-di-O-acetyl-6-(2,3,4,5,6-penta-O-acetyl-D-gluconoylamino)-2,6-didesoxy-a-D-glucopyranosid-2-yl]-amid], $[\alpha]_D^{20}$ +105,0° (*c* 0,2; Dioxan), MS: *m/z* 1612,0 ([M + H]$^+$).

B. Isophthalsäuresäure-bis-[[benzyl-3,4-di-O-acetyl-6-(2,3,4,5,6-penta-O-acetyl-D-gluconoylamino)-2,6-didesoxy-a-D-glucopyranosid-2-yl]-amid] wurde deacetyliert wie in Bsp. 11.F. beschrieben und ergab Isophthalsäuresäure-bis-[(benzyl-2,6-didesoxy-6-D-gluconoylamino-a-D-glucopyranosid-2-yl)-amid], MS: *m/z* 1023,4 ([M + H]$^+$).

C. Sulfatierung von Isophthalsäuresäure-bis-[(benzyl-2,6-didesoxy-6-D-gluconoylamino-a-D-glucopyranosid-2-yl)-amid] wie in Bsp. 9.H. beschrieben ergab Isophthalsäure-bis-[[benzyl-2,6-didesoxy-6-(2,3,4,5,6-penta-O-sulfo-D-gluconoylamino)-3,4-di-O-sulfo-a-D-glucopyranosid-2-yl]-amid] Tetradekanatriumsalz, $[\alpha]_D^{20}$ +74,0° (*c* 0,2; Wasser), MS: *m/z* 2451 (rekonstruiertes M).

Beispiel 25

A. Benzyl-6-amino-2-benzyloxycarbonylamino-2,6-didesoxy-a-D-glucopyranosid (vgl. Bsp. 9.C.) und D-Gulonsäure-$\gamma$-lacton wurden umgesetzt wie in Bsp. 14.A. beschrieben. Das Rohprodukt wurde acetyliert wie in Bsp. 14.B. beschrieben und ergab 2,3,4,5,6-Penta-O-acetyl-D-gulonsäure-(benzyl-3,4-di-O-acetyl-2-benzyloxycarbonylamino-2,6-didesoxy-a-D-glucopyranosid-6-yl)-amid, $[\alpha]_D^{20}$ +88,0° (*c* 0,2; Dioxan), MS: *m/z* 897,4 ([M + Na]$^+$).

B. 2,3,4,5,6-Penta-O-acetyl-D-gulonsäure-(benzyl-3,4-di-O-acetyl-2-benzyloxycarbonylamino-2,6-didesoxy-a-D-glucopyranosid-6-yl)-amid wurde hydriert wie unter Bsp. 13.B. beschrieben und ergab 2,3,4,5,6-Penta-O-acetyl-D-gulonsäure-(benzyl-3,4-di-O-acetyl-2-amino-2,6-didesoxy-a-D-glucopyranosid-6-yl)-amid, $[\alpha]_D^{20}$ +95,0° (*c* 0,2; Dioxan), MS: *m/z* 741,4 ([M + H]$^+$).

C. Reaktion von 2,3,4,5,6-Penta-O-acetyl-D-gulonsäure-(benzyl-3,4-di-O-acetyl-2-amino-2,6-didesoxy-a-D-glucopyranosid-6-yl)-amid mit Isophthalsäuresäure ergab wie in Bsp. 16.D. beschrieben Isophthalsäuresäure-bis-[[benzyl-3,4-di-O-acetyl-6-(2,3,4,5,6-penta-O-acetyl-D-gulonoylamino)-2,6-didesoxy-a-Dglucopyranosid-2-yl]-amid], $[\alpha]_D^{20}$ +102,0° (*c* 0,2; Dioxan), MS: *m/z* 1633,1 ($[M + Na]^+$).

D. Isophthalsäuresäure-bis-[[benzyl-3,4-di-O-acetyl-6-(2,3,4,5,6-penta-O-acetyl-D-gulonoylamino)-2,6-didesoxy-a-D-glucopyranosid-2-yl]-amid] wurde deacetyliert wie in Bsp. 11.F. beschrieben und ergab Isophthalsäuresäure-bis-[(benzyl-2,6-didesoxy-6-D-gulonoylamino-a-D-glucopyranosid-2-yl)-amid], MS: *m/z* 1045,6 ($[M+ Na]^+$).

E. Sulfatierung von Isophthalsäuresäure-bis-[(benzyl-2,6-didesoxy-6-D-gulonoylamino-a-D-glucopyranosid-2-yl)-amid] wie in Bsp. 9.H. beschrieben ergab Isophthalsäuresäure-bis- [[benzyl-2,6-didesoxy-6-(2,3,4,5,6-penta-O-sulfo-D-gulonoylamino)-3,4-di-O-sulfo-a-D-glucopyranosid-2-yl]-amid] Tetradekanatriumsalz, $[\alpha]_D^{20}$ +90,5° (*c* 0,2; Wasser), MS: *m/z* 2452 (rekonstruiertes M).

Beispiel 26

A. Benzyl-6-amino-2-benzyloxycarbonylamino-2,6-didesoxy-a-D-glucopyranosid (vgl. Bsp. 9.C.) und L-Gulonsäure-γ-lacton wurden umgesetzt wie in Bsp. 14.A. beschrieben. Das Rohprodukt wurde acetyliert wie in Bsp. 14.B. beschrieben und ergab 2,3,4,5,6-Penta-O-acetyl-L-gulonsäure-(benzyl-3,4-di-O-acetyl-2-benzyloxycarbonylamino-2,6-didesoxy-a-D-glucopyranosid-6-yl)-amid, $[\alpha]_D^{20}$ +71,0° (*c* 0,2; Dioxan), MS: *m/z* 875,2 ($[M + H]^+$).

B. 2,3,4,5,6-Penta-O-acetyl-L-gulonsäure-(benzyl-3,4-di-O-acetyl-2-benzyloxycarbonylamino-2,6-didesoxy-a-D-glucopyranosid-6-yl)-amid wurde hydriert wie unter Bsp. 13.B. beschrieben und ergab 2,3,4,5,6-Penta-O-acetyl-L-gulonsäure-(benzyl-3,4-di-O-acetyl-2-amino-2,6-didesoxy-a-D-glucopyranosid-6-yl)-amid, $[\alpha]_D^{20}$ +75,5° (*c* 0,2; Dioxan), MS: *m/z* 763,6 ($[M + Na]^+$).

C. Reaktion von 2,3,4,5,6-Penta-O-acetyl-L-gulonsäure-(benzyl-3,4-di-O-acetyl-2-amino-2,6-didesoxy-a-D-glucopyranosid-6-yl)-amid mit Isophthalsäuresäure ergab wie in Bsp. 16.D. beschrieben Isophthalsäuresäure-bis-[[benzyl-3,4-di-O-acetyl-6-(2,3,4,5,6-penta-O-acetyl-L-gulonoyl-amino)-2,6-didesoxy-a-D-glucopyranosid-2-yl]-amid], $[\alpha]_D^{20}$ +91,5° (c 0,2; Dioxan), MS: *m/z* 1630,0 ($[M + NH_4]^+$).

D. Isophthalsäuresäure-bis-[[benzyl-3,4-di-O-acetyl-6-(2,3,4,5,6-penta-O-acetyl-L-gulonoylamino)-2,6-didesoxy-a-D-glucopyranosid-2-yl]-amid] wurde deacetyliert wie in Bsp. 11.F. beschrieben und ergab Isophthalsäuresäure-bis-[(benzyl-2,6-didesoxy-6-L-gulonoylamino-a-D-glucopyranosid-2-yl)-amid], MS: *m/z* 1045,9 ($[M + Na]^+$).

E. Sulfatierung von Isophthalsäuresäure-bis-[(benzyl-2,6-didesoxy-6-L-gulonoylamino-a-D-glucopyranosid-2-yl)-amid] wie in Bsp. 9.H. beschrieben ergab Isophthalsäuresäure-bis-[[benzyl-2,6-didesoxy-6-(2,3,4,5,6-penta-O-sulfo-L-gulonoylamlno)-3,4-di-O-sulfo-a-D-glucopyranosid-2-yl]-amid] Tetradekanatriumsalz, $[\alpha]_D^{20}$ +52,5° (*c* 0,2; Wasser), MS: *m/z* 2431 (rekonstruiertes M - Na).

Beispiel 27

A. Reaktion von 2,3,4,5,6,7-Hexa-O-acetyl-D-glycero-D-guloheptansäure-(benzyl-3,4-di-O-acetyl-2-amino-2,6-didesoxy-a-D-glucopyranosid-6-yl)-amid (vgl. Bsp. 22.B.) mit Isophthalsäure in Dimethylformamid wie in Bsp. 16.D. beschrieben ergab Isophthalsäure-bis-[[benzyl-3,4-di-O-acetyl-6-(2,3,4,5,6,7-hexa-O-acetyl-D-glycero-D-gulo-heptanoylamino)-2,6-didesoxy-a-D-glucopyranosid-6-yl]-amid], $[\alpha]_D^{20}$ +99,0° (*c* 0,2; Dioxan), MS: *m/z* 1773,0 ($[M + NH_4]^+$).

B. Isophthalsäure-bis-[[benzyl-3,4-di-O-acetyl-6-(2,3,4,5,6,7-hexa-O-acetyl-D-glycero-D-gulo-heptanoylamlno)-2,6-didesoxy-a-D-glucopyranosid-6-yl]-amid] wurde deacetyliert wie in Bsp. 11.F. beschrieben und ergab Isophthalsäure-bis-[[benzyl-2,6-didesoxy-6-(D-glycero-D-guloheptanoylamino)-a-D-glucopyranosid-6-yl]-amid], MS: *m/z* 1083,2 ($[M + H]^+$).

C. Sulfatierung von Isophthalsäure-bis-[[benzyl-2,6-didesoxy-6-(D-glycero-D-gulo-heptanoylamino)-a-D-glucopyranosid-6-yl]-amid] wie in Bsp. 9.H. beschrieben ergab Isophthalsäure-bis-[[benzyl-2,6-didesoxy-6-(2,3,4,5,6,7-hexa-O-sulfo-D-glycero-D-gulo-heptanoylamino)-3,4-di-O-sulfo-a-D-glucopyranosid-6-yl]-amid] Hexadekanatriumsalz, $[\alpha]_D^{20}$ +71,0° (*c* 0,2; Wasser), MS: *m/z* 2715 (rekonstruiertes M).

Beispiel 28

A. Benzyl-6-amino-2-benzyloxycarbonylamino-2,6-didesoxy-a-D-glucopyranosid (vgl. Bsp. 9.C.) wurde mit Isophthalsäure in Dimethylformamid umgesetzt wie in Bsp. 16.D. beschrieben. Das Rohprodukt wurde acetyliert wie in Bsp. 14.B. beschrieben und ergab Isophthalsäure-bis-(benzyl-3,4-di-O-acetyl-2-benzyloxycarbonylamino-2,6-didesoxy-a-D-glucopyranosid-6-yl)-amid, $[\alpha]_D^{20}$ +101,5° (*c* 0,2; Dioxan), MS: *m/z* 1125,5 ($[M + Na]^+$).

B. Isophthalsäure-bis-(benzyl-3,4-di-O-acetyl-2-benzyloxycarbonylamino-2,6-didesoxy-a-D-glucopyranosid-6-yl)-amid wurde hydriert wie unter Bsp. 13.B. beschrieben und ergab Isophthalsäure-bis-(benzyl-3,4-di-O-acetyl-2-amino-2,6-didesoxy-a-D-glucopyranosid-6-yl)-amid, $[\alpha]_D^{20}$ +92,0° (*c* 0,2; Dioxan), MS: *m/z* 813,3 ($[M + H]^+$).

C. Isophthalsäure-bis-(benzyl-3,4-di-O-acetyl-2-amino-2,6-didesoxy-a-D-glucopyranosid-6-yl)-amid und D-Gluconsäure-γ-lacton wurden umgesetzt wie in Bsp. 14.A. beschrieben. Das Rohprodukt wurde acetyliert wie in Bsp.

14.B. beschrieben und ergab Isophthalsäure-bis-[benzyl-3,4-di-O-acetyl-2,6-didesoxy-2-(2,3,4,5,6-penta-O-acetyl-D-gluconoylamino)-a-D-glucopyranosid-6-yl]-amid, $[\alpha]^{20}_{D}$ +69,4° (*c* 0,2; Dioxan), MS: *m/z* 1633,6 ($[M + Na]^+$).

D. Isophthalsäure-bis-[benzyl-3,4-di-O-acetyl-2,6-didesoxy-2-(2,3,4,5,6-penta-O-acetyl-D-gluconoylamino)-a-D-glucopyranosid-6-yl]-amid wurde deacetyliert wie in Bsp. 11.F. beschrieben und ergab Isophthalsäure-bis-(benzyl-2,6-didesoxy-2-D-gluconoylamino-a-D-glucopyranosid-6-yl)-amid, MS: *m/z* 1046,8 ($[M + Na]^+$).

E. Sulfatierung von Isophthalsäure-bis-(benzyl-2,6-didesoxy-2-D-gluconoylamino-a-D-glucopyranosid-6-yl)-amid wie in Bsp. 9.H. beschrieben ergab Isophthalsäure-bis-[benzyl-2,6-didesoxy-3,4-di-O-sulfo-2-(2,3,4,5,6-penta-O-sulfo-D-gluconoylamino)-a-D-glucopyranosid-6-yl]-amid Tetradekanatriumsalz, $[\alpha]^{20}_{D}$ +45,0° (*c* 0,2; Wasser), MS: *m/z* 2451 (rekonstruiertes M).

Beispiel 29

A. Benzyl-6-amino-2-benzyloxycarbonylamino-2,6-didesoxy-a-D-glucopyranosid (vgl. Bsp. 9.C.) wurde mit Diphenylmethan-4,4'-dicarbonsäure in Dimethylformamid umgesetzt wie in Bsp. 16.D. beschrieben und ergab Diphenylmethan-4,4'-dicarbonsäure-bis-[(benzyl-2-benzyloxycarbonylamino-2,6-didesoxy-a-D-glucopyranosid-6-yl)-amid], $[\alpha]^{20}_{D}$ +82,2° (*c* 0,2; Dioxan), MS: *m/z* 1047,6 ($[M + Na]^+$).

B. Diphenylmethan-4,4'-dicarbonsäure-bis-[(benzyl-2-benzyloxycarbonylamino-2,6-didesoxy-a-D-glucopyranosid-6-yl)-amid] wurde hydriert wie unter Bsp. 13.B. beschrieben und ergab Diphenylmethan-4,4'-dicarbonsäure-bis-[(benzyl-2-amino-2,6-didesoxy-a-Dglucopyranosid-6-yl)-amid], welches ohne weitere Reinigung in die nächste Stufe (Bsp. 29.D.) eingesetzt wurde.

C. Eine Lösung von 7,4 g 4-Fluor-3-nitro-benzoesäure in 100 ml Dimethylformamid wurde mit 16,0 g D-Glucamin versetzt und 4 Stunden bei Raumtemperatur gerührt. Nach Zugabe von 6 ml Triethylamin wurde 16 Stunden bei 40°C weitergerührt. Die Reaktionslösung wurde eingedampft. Der Rückstand wurde mit 400 ml Pyridin und 200 ml Essigsäureanhydrid 5 Stunden bei Raumtemperatur gerührt. Nach Einengen wurde der erhaltene Rückstand mit Wasser versetzt und mit 5 %iger Salzsäurelösung auf pH = 2 -3 angesäuert und mit Essigester extrahiert. Die organischen Extrakte wurden mit Eiswasser und gesättigter Kochsalzlösung gewaschen, über Magnesiumsulfat getrocknet und eingedampft. Der Rückstand wurde mit Essigester über Kieselgel chromatographiert. Die Produktfraktionen wurden eingeengt, aus Ether kristallisiert und ergaben 4-(2,3,4,5,6-Penta-O-acetyl-D-glucit-1-ylamino)-3-nitro-benzoesäure als gelbe Kristalle, $[\alpha]^{20}_{D}$ -23,0° (*c* 0,5; DMSO), MS: *m/z* 579,7 ($[M+Na]^+$).

D. Diphenylmethan-4,4'-dicarbonsäure-bis-[(benzyl-2-amino-2,6-didesoxy-a-D-glucopyranosid-6-yl)-amid] wurde mit 4-(2,3,4,5,6-Penta-O-acetyl-D-glucit-1-ylamino)-3-nitrobenzoesäure in Dimethylformamid umgesetzt wie in Bsp. 16.D. beschrieben und ergab Diphenylmethan-4,4'-dicarbonsäure-bis-[[benzyl-2,6-didesoxy-2-[4-(2,3,4,5,6-penta-O-acetyl-D-glucit-1-ylamino)-3-nitro-benzoylamino]-a-D-glucopyranosid-6-yl]-amid], $[\alpha]^{20}_{D}$ +21,4° (*c* 0,5; Dimethylsulfoxid), MS: *m/z* 1856,3 ($[M + Na]^+$).

E. Diphenylmethan-4,4'-dicarbonsäure-bis-[[benzyl-2,6-didesoxy-2-[4-(2,3,4,5,6-penta-O-acetyl-D-glucit-1-ylamino)-3-nitro-benzoylamino]-a-D-glucopyranosid-6-yl]-amid] wurde acetyliert wie in Bsp. 14.B. beschrieben. Das Rohprodukt wurde chromatographisch mit Methylenchlorid/Isopropanol über Kieselgel gereinigt und ergab Diphenylmethan-4,4'-dicarbonsäure-bis-[[benzyl-3,4-di-O-acetyl-2,6-didesoxy-2-[4-(2,3,4,5,6-penta-O-acetyl-D-glucit-1-ylamino)-3-nitro-benzoylamino]-a-D-glucopyranosid-6-yl]-amid], $[\alpha]^{20}_{D}$ +61,4° (*c* 0,5; Chloroform), MS: *m/z* 2025,6 ($[M + Na]^+$).

F. Diphenylmethan-4,4'-dicarbonsäure-bis-[[benzyl-3,4-di-O-acetyl-2,6-didesoxy-2-[4-(2,3,4,5,6-penta-O-acetyl-D-glucit-1-ylamino)-3-nitro-benzoylamino]-a-D-glucopyranosid-6-yl]-amid] wurde deacetyliert wie in Bsp. 11.F. beschrieben und ergab Diphenylmethan-4,4'-dicarbonsäure-bis-[[benzyl-2,6-didesoxy-2-(4-D-glucit-1-ylamino-3-nitro-benzoylamino)-a-D-glucopyranosid-6-yl]-amid], $[\alpha]^{20}_{D}$ +53,4° (*c* 0,5; DMSO), MS: *m/z* 1436,4 ($[M + Na]^+$).

G. Eine Suspension von 0.94 g Diphenylmethan-4,4'-dicarbonsäure-bis-[[benzyl-2,6-didesoxy-2-(4-D-glucit- 1-ylamino-3-nitro-benzoylamino)-a-D-glucopyranosid-6-yl]-amid] und 3,85 g Schwefeltrioxid-Triethylaminkomplex in 15 ml absolutem Dimethylformamid wurde 20 Stunden bei 45°C gerührt. Nach Abkühlen wurde am Hochvakuum eingeengt. Der Rückstand wurde mit einer Lösung von 3,26 g Natriumacetat in 50 ml Wasser versetzt, eingedampft, mehrmals mit Wasser versetzt und erneut eingedampft. Der so erhaltene Rückstand wurde in Wasser aufgenommen und an Sephadex® LH20 und SP Sephadex® C-25 chromatographiert. Die Produktfraktionen wurden lyophilisiert und ergaben Diphenylmethan-4,4'-dicarbonsäure-bis-[[benzyl-2,6-didesoxy-2-[4-(2,3,4,5,6-penta-O-sulfo-D-glucit-1-ylamino)-3-nitro-benzoylamino]-3,4-di-O-sulfo-a-D-glucopyranosid-6-yl]-amid] Tetradekanatriumsalz, $[\alpha]^{20}_{D}$ +64,0° (*c* 0,4; Wasser), MS: *m/z* 2842,0 (rekonstruiertes M).

Beispiel 30

A. Zu einer Lösung von 27,8 g 4-(2,3,4,5,6-Penta-O-acetyl-D-glucit-1-ylamino)-3-nitro-benzoesäure (vgl. Bsp. 29.C.) und 5,32 g N-Methylmorpholin in 150 ml absolutem Dimethylformamid wurde 8,78 g 2-Chlor-2,4-dimethoxy-1,3,5-triazin bei 0°C gegeben. Das Reaktionsgemisch wurde 2 Stunden bei dieser Temperatur gerührt und dann

mit 14,0 g Benzyl-2-amino-2-desoxy-a-D-giucopyranosid (Meyer zu Reckendorf, Chem. Ber. **107**, 869 (1974)) versetzt. Das Gemisch wurde 18 Stunden weitergerührt und dann im Vakuum konzentriert. Der verbleibende Sirup wurde durch Chromatographie an Kieselgel mit Methylenchlorid/ Isopropanol gereinigt und ergab Benzyl-2-desoxy-2-[3-nitro-4-(2,3,4,5,6-penta-O-acetyl-D-glucit-1-ylamino)-benzoylamino]-a-D-glucopyranosid, $[\alpha]_D^{20}$ +33,6° (*c* 0,5; DMSO), MS: *m/z* 808,4 ([M+H]$^+$).

B. Zu einer Lösung von 30,0 g Benzyl-2-desoxy-2-[3-nitro-4-(2,3,4,5,6-penta-O-acetyl-D-glucit-1-ylamino)-benzoylamino]-a-D-glucopyranosid in 250 ml absolutem Pyridin wurde 10 ,9 g p-Toluolsulfonylchlorid in Portionen zugegeben. Nach beendeter Zugabe wurde das Reaktionsgemisch 7 Stunden bei Raumtemperatur gerührt und dann eingeengt. Der Rückstand wurde in Essigester aufgenommen und mit Wasser extrahiert. Die organischen Phasen wurden mit verdünnter Schwefelsäure, Wasser und gesättigter Kochsalzlösung gewaschen, über Magnesiumsulfat getrocknet und eingedampft. Der Rückstand wurde mit Methylenchlorid/ Isopropanol auf Kieselgel chromatographiert und ergab Benzyl-2-desoxy-2-[3-nitro-4-(2,3,4,5,6-penta-O-acetyl-D-glucit-1-ylamino)-benzoylamino]-6-O-(p-tolylsulfonyl)-a-D-glucopyranosid, $[\alpha]_D^{20}$ +31,4° (*c* 0,5; DMSO), MS: *m/z* 984,7 ([M+K]$^+$).

C. Zu einer Lösung von 31,0 g Benzyl-2-desoxy-2-[3-nitro-4-(2,3,4,5,6-penta-O-acetyl-D-glucit-1-ylamino)-benzoylamino]-6-O-(p-tolylsulfonyl)-a-D-glucopyranosid in 250 ml absolutem Dimethylformamid wurde 6,5 g Natriumazid gegeben. Das Reaktionsgemisch wurde 6 Stunden bei 65°C gerührt und dann eingeengt. Der Rückstand wurde in Eiswasser gegossen und mit Essigester extrahiert. Die organischen Phasen wurden mit Wasser und gesättigter Kochsalzlösung gewaschen, über Magnesiumsulfat getrocknet und eingedampft. Der Rückstand wurde mit Methylenchlorid/Isopropanol auf Kieselgel chromatographiert und ergab Benzyl-6-azido-2,6-didesoxy-2-[3-nitro-4-(2,3,4,5,6-penta-O-acetyl-D-glucit-1-ylamino)-benzoylamino]-a-D-glucopyranosid, $[\alpha]_D^{20}$ +30,2° (*c* 0,5; DMSO), MS: *m/z* 855,6 ([M+Na]$^+$).

D. Zu einer Lösung von 4,16 g Benzyl-6-azido-2,6-didesoxy-2-[3-nitro-4-(2,3,4,5,6-penta-O-acetyl-D-glucit-1-ylamino)-benzoylamino]-a-D-glucopyranosid in 50 ml Tetraydrofuran und 1,8 ml Wasser wurde 2,15 g Triphenylphosphin gegeben und 20 Stunden bei Raumtemperatur gerührt. Nach Einengen wurde der Rückstand mit Essigester/ Methanol über Kieselgel chromatographiert und ergab Benzyl-6-amino-2,6-didesoxy-2-[3-nitro-4-(2,3,4,5,6-penta-O-acetyl-D-glucit-1-ylamino)-benzoylamino]-a-D-glucopyranosid, $[\alpha]_D^{20}$ +37,4° (*c* 0,5; DMSO), MS: *m/z* 829,7 ([M+Na]$^+$).

E. Zu einer Lösung von 301 mg Benzol-1,3,5-tricarbonsäure und 0,35 ml N-methylmorpholin in 5 ml absolutem Dimethylformamid wurde 615 mg 2-Chlor-2,4-dimethoxy-1,3,5-triazin bei 0°C gegeben. Das Reaktionsgemisch wurde 2 Stunden bei dieser Temperatur gerührt und dann mit 3,62 g Benzyl-6-amino-2,6-didesoxy-2-[3-nitro-4-(2,3,4,5,6-penta-O-acetyl-D-glucit-1-ylamino)-benzoylamino]-a-D-glucopyranosid versetzt. Das Reaktionsgemisch wurde 20 Stunden bei Raumtemperatur gerührt und dann eingeengt. Der Rückstand wurde mit 40 ml Acetanhydrid in 60 ml Pyridin bei Raumtemperatur acetyliert und nach 5 Stunden eingeengt, dann in Essigester aufgenommen und mit Wasser extrahiert. Die organischen Phasen wurden mit verdünnter Schwefelsäure, Wasser und gesättigter Natriumbikarbonatlösung gewaschen, über Magnesiumsulfat getrocknet und eingedampft. Der Rückstand wurde mit Methylenchlorid/ Isopropanol über Kieselgel chromatographiert und ergab Benzol-1,3,5-tricarbonsäure-tris-[[benzyl-3,4-di-O-acetyl-2,6-didesoxy-2-[4-(2,3,4,5,6-penta-O-acetyl-D-glucit-1-ylamino)-3-nitro-benzoylamino]-a-D-glucopyranosid-6-yl]-amid], $[\alpha]_D^{20}$ +65,6° (*c* 0,5; Chloroform), MS: *m/z* 2851,2 ([M+Na]$^+$).

F. Eine Lösung von 1,98 g Benzol-1,3,5-tricarbonsäure-tris-[[benzyl-3,4-di-O-acetyl-2,6-didesoxy-2-[4-(2,3,4,5,6-penta-O-acetyl-D-glucit-1-ylamino)-3-nitro-benzoylamino]-a-D-glucopyranosid-6-yl]-amid] in 30 ml Methanol und 20 ml Tetrahydrofuran wurde mit 2 ml einer 2 %igen methanolischen Natriummethanolatlösung versetzt und 6 Stunden bei Raumtemperatur gerührt. Der entstandene Niederschlag wurde abgenutscht, mit Methanol gewaschen, am Vakuum bei 60°C getrocknet und ergab Benzol-1,3,5-tricarbonsäure-tris-[[benzyl-2,6-didesoxy-2-(4-D-glucit-1-ylamino-3-nitro-benzoylamino)-a-D-glucopyranosid-6-yl]-amid], $[\alpha]_D^{20}$ +68,2° (*c* 0,5; Dimethylsulfoxid), MS: *m/z* 1968,6 ([M + Na]$^+$).

G. Sulfatierung von Benzol-1,3,5-tricarbonsäure-tris-[[benzyl-2,6-didesoxy-2-(4-D-glucit- 1-ylamino-3-nitro-benzoylamino)-a-D-glucopyranosid-6-yl]-amid] wie in Bsp. 29.G. beschrieben ergab Benzol-1,3,5-tricarbonsäure-tris-[[benzyl-2,6-didesoxy-2-[4-(2,3,4,5,6-penta-O-sulfo-D-glucit-1-ylamino)-3-nitro-benzoylamino]-3,4-di-O-sulfo-a-D-glucopyranosid-6-yl]-amid] Henikosanatriumsalz, $[\alpha]_D^{20}$ +65,0° (*c* 0,5; Wasser), MS: *m/z* 4085,5 (rekonstruiertes M).

Beispiel 31

A. Umsetzung von (Z)-Stilben-4,4'-dicarbonsäure mit Benzyl-6-amino-2,6-didesoxy-2-[3-nitro-4-(2,3,4,5,6-penta-O-acetyl-D-glucit-1-ylamino)-benzoylamino]-a-D-glucopyranosid wie unter Bsp. 30.E. beschrieben ergab (Z)-Stilben-4,4'-dicarbonsäure-bis-[[benzyl-3,4-di-O-acetyl-2,6-didesoxy-2-[4-(2,3,4,5,6-penta-O-acetyl-D-glucit-1-ylamino)-3-nitro-benzoylamino]-a-D-glucopyranosid-6-yl]-amid], $[\alpha]_D^{20}$ +74,2° (*c* 0,5; Dimethylsulfoxid), MS: *m/z* 2036,6 ([M+Na]$^+$).

B. Deacetylierung von (Z)-Stilben-4,4'-dicarbonsäure-bis-[[benzyl-3,4-di-O-acetyl-2,6-didesoxy-2-[4-(2,3,4,5,6-penta-O-acetyl-D-glucit-1-ylamino)-3-nitro-benzoylamino]-a-D-glucopyranosid-6-yl]-amid] wie unter Bsp. 30.F. beschrieben ergab (Z)-Stilben-4,4'-dicarbonsäure-bis-[[benzyl-2,6-didesoxy-2-(4-D-glucit-1-ylamino-3-nitro-benzoylamino)-a-D-glucopyranosid-6-yl]-amid], $[\alpha]^{20}_{D}$ +44,2° (*c* 0,5; Dimethylsulfoxid).

C. Sulfatierung von (Z)-Stilben-4,4'-dicarbonsäure-bis-[[benzyl-2,6-didesoxy-2-(4-D-glucit-1-ylamino-3-nitro-benzoylamlno)-a-D-glucopyranosid-6-yl]-amid] wie in Bsp. 29.G. beschrieben ergab (Z)-Stilben-4,4'-dicarbonsäure-bis-[[benzyl-2,6-didesoxy-2-[4-(2,3,4,5,6-penta-O-sulfo-D-glucit-1-ylamino)-3-nitro-benzoylamino]-3,4-di-O-sulfo-a-D-glucopyranosid-6-yl]-amid] Tetradekanatriumsalz, $[\alpha]^{20}_{D}$ +61,4° (*c* 0,5; Wasser), MS: *m/z* 2854,5 (rekonstruiertes M).

## Beispiel 32

A. Eine Lösung von 55,0 g 3-Chlor-4-methyl-benzoesäuremethylester (Stempel et al., J. Am. Chem. Soc. 73, 455 (1951)) in 1 l Tetrachlorkohlenstoff wurde mit 53,0 g N-Bromsuccinimid und 100 mg Dibenzoylperoxid versetzt und unter Belichtung mit einer 150 W Lampe 4 Stunden unter Rückfluss gekocht. Das abgekühlte Reaktionsgemisch wurde filtriert. Das Filtrat wurde eingeengt. Eine Lösung des Rückstands in 770 ml Benzol wurde mit 83,0 g Triphenylphosphin versetzt und 4 Stunden unter Rückfluss erhitzt. Nach dem Abkühlen wurden 380 ml Ether zugegeben. Das ausgefallene Phosphoniumsalz wurde abgenutscht, mit Benzol/ Ether gewaschen, bei 50°C am Vakuum getrocknet und ergab (3-Chlor-4-methoxycarbonyl-benzyl)-triphenylphosphoniumbromid, MS: *m/z* 445,4 ($M^+$).

B. Eine Suspension von 52,6 g (3-Chlor-4-methoxycarbonyl-benzyl)-triphenylphosphoniumbromid und 16,4 g 4-Formyl-benzoesäuremethylester in 700 ml Tetrahydrofuran wurde mit 115 ml einer 2 %igen Natriummethanolatlösung tropfenweise innert 30 Minuten versetzt und 1 Stunde bei Raumtemperatur weitergerührt. Das Reaktionsgemisch wurde genutscht. Das Filtrat wurde eingeengt, und der Rückstand wurde mit Essigester/Hexan/ Methylenchlorid über Kieselgel chromatographiert und ergab farblose Kristalle von (E)-2-Chlor-stilben-4,4'-dicarbonsäuredimethylester, MS: *m/z* 330.0 ($M^+$).

C. Eine Suspension von 3,0 g (E)-2-Chlor-stilben-4,4'-dicarbonsäure-dimethylester in 30 ml Methanol wurde mit 36 ml 1 m Natronlauge versetzt und 5 Stunden unter Rückfluss erhitzt. Nach dem Abkühlen wurde 37 ml 1 m Salzsäure zugetropft. Der entstandene Brei wurde genutscht; der Rückstand wurde mit Wasser gewaschen, bei 70°C unter Vakuum getrocknet und ergab (E)-2-Chlor-stilben-4,4'-dicarbonsäure, MS: *m/z* 302 ($M^+$).

D. Umsetzung von (E)-2-Chlor-stilben-4,4'-dicarbonsäure mit Benzyl-6-amino-2,6-didesoxy-2-[3-nitro-4-(2,3,4,5,6-penta-O-acetyl-D-glucit-1-ylamino)-benzoylamino]-a-D-glucopyranosid wie unter Bsp. 30.E. beschrieben ergab (E)-2-Chlor-stilben-4,4'-dicarbonsäure-bis-[[benzyl-3,4-di-O-acetyl-2,6-didesoxy-2-[4-(2,3,4,5,6-penta-O-acetyl-D-glucit-1-ylamino)-3-nitro-benzoylamino]-a-D-glucopyranosid-6-yl] -amid], $[\alpha]^{20}_{D}$ +54,6° (*c* 0,5; Chloroform), MS: *m/z* 2071,3 ($[M+Na]^+$).

E. Deacetylierung von (E)-2-Chlor-stilben-4,4'-dicarbonsäure-bis-[[benzyl-3,4-di-O-acetyL-2,6-didesoxy-2-[4-(2,3,4,5,6-penta-O-acetyl-D-glucit-1-ylamino)-3-nitro-benzoylamino]-a-D-glucopyranosid-6-yl]-amid] wie unter Bsp. 30.F. beschrieben ergab (E)-2-Chlor-stilben-4,4'-dicarbonsäure-bis-[[benzyl-2,6-didesoxy-2-(4-D-glucit-1-ylamino-3-nitro-benzoylamino)-a-D-glucopyranosid-6-yl]-amid], $[\alpha]^{20}_{D}$ +27,4° (*c* 0,5; Dimethylsulfoxid), MS: *m/z* 1481,7 ($[M+Na]^+$).

F. Sulfatierung von (E)-2-Chlor-stilben-4,4'-dicarbonsäure-bis-[[benzyl-2,6-didesoxy-2-(4-D-glucit-1-ylamino-3-nitro-benzoylamino)-a-D-glucopyranosid-6-yl]-amid] wie in Bsp. 29.G. beschrieben ergab (E)-2-Chlor-stilben-4,4'-dicarbonsäure-bis-[[benzyl-2,6-didesoxy-2-[4-(2,3,4,5,6-penta-O-sulfo-D-glucit-1-ylamino)-3-nitro-benzoylamino]-3,4-di-O-sulfo-a-D-glucopyranosid-6-yl]-amid] Tetradekanatriumsalz, $[\alpha]^{20}_{D}$ +51,6° (*c* 0,5; Wasser), MS: *m/z* 2889,5 (rekonstruiertes M).

## Beispiel 33

A. Umsetzung von Isophthalsäure mit Benzyl-6-amino-2,6-didesoxy-2-[3-nitro-4-(2,3,4,5,6-penta-O-acetyl-D-giucit-1-ylamino)-benzoylamino]-a-D-glucopyranosid wie unter Bsp. 30.E. beschrieben ergab Isophthalsäure-bis-[[benzyl-3,4-di-O-acetyl-2,6-didesoxy-2-[4-(2,3,4,5,6-penta-O-acetyl-D-glucit-1-ylamino)-3-nitro-benzoylamino]-a-D-glucopyranosid-6-yl]-amid], $[\alpha]^{20}_{D}$ +68,9° (*c* 0,5; Dimethylsulfoxid), MS: *m/z* 1929,8 ($[M+Na]^+$).

B. Deacetylierung von Isophthalsäure-bis-[[benzyl-3,4-di-O-acetyl-2,6-didesoxy-2-[4-(2,3,4,5,6-penta-O-acetyl-D-glucit-1-ylamino)-3-nitro-benzoylamino]-a-D-glucopyranosid-6-yl]-amid] wie unter Bsp. 30.F. beschrieben ergab Isophthalsäure-bis-[[benzyl-2,6-didesoxy-2-(4-D-glucit-1-ylamino-3-nitro-benzoylamino)-a-D-glucopyranosid-6-yl]-amid], $[\alpha]^{20}_{D}$ +63,6° (*c* 0,5; Dimethylsulfoxid), MS: *m/z* 1345,3 ($[M+Na]^+$).

C. Sulfatierung von Isophthalsäure-bis-[[benzyl-2,6-didesoxy-2-(4-D-glucit-1-ylamino-3-nitro-benzoylamino)-a-D-glucopyranosid-6-yl]-amid] wie in Bsp. 29.G. beschrieben ergab Isophthalsäure-bis-[[benzyl-2,6-didesoxy-2-[4-(2,3,4,5,6-penta-O-sulfo-D-glucit-1-ylamino)-3-nitro-benzoylamino]-3,4-di-O-sulfo-a-D-glucopyranosid-6-yl]-amid] Tetradekanatriumsalz, $[\alpha]^{20}_{D}$ +75,0° (*c* 0,5; Wasser), MS: *m/z* 2751,0 (rekonstruiertes M).

Beispiel 34

A. Eine Suspension von 4,8 g Benzyl-6-azido-2-benzyloxycarbonylamino-2,6-didesoxy-a-D-glucopyranosid (vgl. Bsp. 9.B.) und 1,35 g Naphthalin-1,4,5,8-tetracarbonsäuredianhydrid in 100 ml Pyridin und 3 ml Triethylamin wurde 3 Stunden auf 80°C erhitzt. Danach wurde 20 ml Essigsäureanhydrid innert 15 Minuten bei 70°C zugetropft. Nach 1 Stunde Rühren wurde das Reaktionsgemisch abgekühlt und eingeengt. Der Rückstand wurde mit Eiswasser versetzt und mit Methylenchlorid extrahiert. Die organischen Phasen wurden mit verdünnter Schwefelsäure, Eiswasser, gesättigter Natriumbikarbonatlösung und gesättigter Natriumchloridlösung gewaschen, über Magnesiumsulfat getrocknet und eingeengt. Der Rückstand wurde mit Methylenchlorid/ Isopropanol über Kieselgel chromatographiert. Die Produktfraktionen wurden aus Methylenchlorid/Essigester/ Ether kristallisiert und ergaben 2,7-Bis-(benzyl-3,4-di-O-acetyl-2-benzyloxycarbonylamino-2,6-didesoxy-a-D-glucopyranosid-6-yl)-1,2,3,6,7,8-hexahydro-benzo[lmn][3,8]phenanthrolin-1,3,6,8-tetraon, MS: *m/z* 1222,2 ($[M+NH_4]^+$).

B. Eine Lösung von 2,89 g 2,7-Bis-(benzyl-3,4-di-O-acetyl-2-benzyloxy-carbonylamino-2,6-didesoxy-a-D-glucopyranosid-6-yl)-1,2,3,6,7,8-hexahydrobenzo[lmn][3,8]phenanthrolin-1,3,6,8-tetraon in 100 ml Dimethylformamid wurde in Gegenwart von 10 % Palladium auf Kohle bei Raumtemperatur während 2 Stunden hydriert. Das Reaktionsgemisch wurde über Filterhilfe genutscht, der Rückstand wurde mit Ethanol nachgewaschen. Die Filtrate wurden auf ein Volumen von ca. 10 ml eingeengt und ohne weitere Reinigung als rohes 2,7-Bis-(benzyl-3,4-di-O-acetyl-2-amino-2,6-didesoxy-a-D-glucopyranosid-6-yl)-1,2,3,6,7,8-hexahydro-benzo[lmn][3,8]phenanthrolin-1,3,6,8-tetraon in die nächste Stufe eingesetzt.

C. Umsetzung von 4-(2,3,4,5,6-Penta-O-acetyl-D-glucit-1-ylamino)-3-nitro-benzoesäure (vgl. Bsp. 29.C.) mit 2,7-Bis-(benzyl-3,4-di-O-acetyl-2-amino-2,6-didesoxy-a-D-glucopyranosid-6-yl)-1,2,3,6,7,8-hexahydro-benzo[lmn][3,8]-phenanthrolin-1,3,6,8-tetraon wie unter Bsp. 30.E. beschrieben ergab 2,7-Bis-[benzyl-3,4-di-O-acetyl-2,6-didesoxy-2-[3-nitro-4-(2,3,4,5,6-penta-O-acetyl-D-glucit-1-ylamino)-benzoylamino]-a-D-glucopyranosid-6-yl]-1,2,3,6,7,8-hexahydro-benzo[lmn][3,8]phenanthrolin-1,3,6,8-tetraon, $[\alpha]_D^{20}$ +45,4° (*c* 0,5; Chloroform), MS: *m/z* 2038,5 ($[M+Na]^+$).

D. Deacetylierung von 2,7-Bis-[benzyl-3,4-di-O-acetyl-2,6-didesoxy-2-[3-nitro-4-(2,3,4,5,6-penta-O-acetyl-D-glucit-1-ylamino)-benzoylamino]-a-D-glucopyranosid-6-yl]-1,2,3,6,7,8-hexahydro-benzo[lmn][3,8]phenanthrolin-1,3,6,8-tetraon wie unter Bsp. 30.F. beschrieben ergab 2,7-Bis-[benzyl-2,6-didesoxy-2-(4-D-glucit-1-ylamino-3-nitro-benzoylamino)-a-D-glucopyranosid-6-yl]-1,2,3,6,7,8-hexahydro-benzo[lmn][3,8]phenanthrolin-1,3,6,8-tetraon, $[\alpha]_D^{20}$ +54,0° (*c* 0,5; Dimethylsulfoxid), MS: *m/z* 1447,1 ($[M+Na]^+$).

E. Sulfatierung von 2,7-Bis-[benzyl-2,6-didesoxy-2-(4-D-glucit-1-ylamino-3-nitro-benzoylamino)-a-D-glucopyranosid-6-yl]-1,2,3,6,7,8-hexahydro-benzo[lmn][3,8]phenanthrolin-1,3,6,8-tetraon wie in Bsp. 29.G. beschrieben ergab 2,7-Bis-[benzyl-2,6-didesoxy-2-[3-nitro-4-(2,3,4,5,6-penta-O-sulfo-D-glucit-1-ylamino)-benzoylamino]-3,4-di-O-sulfo-a-D-glucopyranosid-6-yl]-1,2,3,6,7,8-hexahydro-benzo[lmn][3,8]phenanthrolin-1,3,6,8-tetraon Tetradekanatriumsalz, $[\alpha]_D^{20}$ +65,6° (*c* 0,5; Wasser), MS: *m/z* 2855,0 (rekonstruiertes M).

Beispiel 35

A. 4-Fluor-3-nitro-benzoesäure wurde mit N-Methyl-D-Glucamin umgesetzt wie unter Beispiel 29.C. beschrieben und ergab 4-[Methyl-(2,3,4,5,6-penta-O-acetyl-D-glucit-1-yl)-amino]-3-nitro-benzoesäure, $[\alpha]_D^{20}$ +141,2° (*c* 0,5; Chloroform), MS: *m/z* 593,2 ($[M+Na]^+$).

B. Umsetzung von 4-[Methyl-(2,3,4,5,6-penta-O-acetyl-D-glucit-1-yl)-amino]-3-nitro-benzoesäure mit Benzyl-2-amino-2-desoxy-a-D-glucopyranosid wie unter Bsp. 30.A. beschrieben ergab Benzyl-2-desoxy-2-[4-[methyl-(2,3,4,5,6-penta-O-acetyl-D-glucit-1-yl)-amino]-3-nitro-benzoylamino]-a-D-glucopyranosid, $[\alpha]_D^{20}$ +108,8° (*c* 0,5; Chloroform), MS: *m/z* 822,2 ($[M+H]^+$).

C. Tosylierung von Benzyl-2-desoxy-2-[4-[methyl-(2,3,4,5,6-penta-O-acetyl-D-glucit-1-yl)-amino]-3-nitro-benzoylamino]-a-D-glucopyranosid wie unter Bsp. 30.B. beschrieben ergab Benzyl-2-desoxy-2-[4-[methyl-(2,3,4,5,6-penta-O-acetyl-D-glucit-1-yl)-amino]-3-nitro-benzoylamino]-6-O-(p-tolylsulfonyl)-a-D-glucopyranosid, $[\alpha]_D^{20}$ +78,4° (*c* 0,5; Chloroform), MS: *m/z* 976,2 ($[M+H]^+$).

D. Umsetzung von Benzyl-2-desoxy-2-[4-[methyl-(2,3,4,5,6-penta-O-acetyl-D-glucit-1-yl)-amino]-3-nitro-benzoylamino]-6-O-(p-tolylsulfonyl)-a-D-glucopyranosid wie unter Bsp. 30.C. beschrieben ergab Benzyl-6-azido-2-desoxy-2-[4-[methyl-(2,3,4,5,6-penta-O-acetyl-D-glucit-1-yl)-amino]-3-nitrobenzoylamino]-a-D-glucopyranosid, $[\alpha]_D^{20}$ +64,6° (*c* 0,5; Chloroform), MS: *m/z* 847,3 ($[M+H]^+$).

E. Umsetzung von Benzyl-6-azido-2-desoxy-2-[4-[methyl-(2,3,4,5,6-penta-O-acetyl-D-glucit-1-yl)-amino]-3-nitro-benzoylamino]-a-D-glucopyranosid wie unter Bsp. 30.D. beschrieben ergab Benzyl-6-amino-2-desoxy-2-[4-[methyl-(2,3,4,5,6-penta-O-acetyl-D-glucit-1-yl)-amino]-3-nitro-benzoylamino]-a-D-glucopyranosid, $[\alpha]_D^{20}$ +137,0° (*c* 0,5; Chloroform), MS: *m/z* 821,6 ($[M+H]^+$).

F. Umsetzung von Isophthalsäure mit Benzyl-6-amino-2-desoxy-2-[4-[methyl-(2,3,4,5,6-penta-O-acetyl-D-glucit-1-yl)-amino]-3-nitro-benzoylamino]-a-D-glucopyranosid wie unter Bsp. 30.E. beschrieben ergab Isophthalsäure-bis-[[benzyl-3,4-di-O-acetyl-2,6-didesoxy-2-[4-[methyl-(2,3,4,5,6-penta-O-acetyl-D-glucit-1-yl)-amino]-3-nitro-benzoylamino]-a-D-glucopyranosid-6-yl]-amid], $[\alpha]_D^{20}$ +147,4° (*c* 0,5; Chloroform), MS: *m/z* 1961,5 ([M+Na]$^+$).

G. Deacetylierung von Isophthalsäure-bis-[[benzyl-3,4-di-O-acetyl-2,6-didesoxy-2-[4-[methyl-(2,3,4,5,6-penta-O-acetyl-D-glucit-1-yl)-amino]-3-nitro-benzoylamino]-a-D-glucopyranosid-6-yl]-amid] wie unter Bsp. 30.F. beschrieben ergab Isophthalsäure-bis-[[benzyl-2,6-didesoxy-2-[4-[(D-glucit-1-yl)-methyl-amino]-3-nitro-benzoylamino]-a-D-glucopyranosid-6-yl]-amid], $[\alpha]_D^{20}$ +148,0° (*c* 0,5; Dimethylsulfoxid), MS: *m/z* 1373,1 ([M+Na]$^+$).

H. Sulfatierung von Isophthalsäure-bis-[[benzyl-2,6-didesoxy-2-[4-[(D-glucit-1-yl)-methyl-amino]-3-nitro-benzoylamino]-a-D-glucopyranosid-6-yl]-amid] wie in Bsp. 29.G. beschrieben ergab Isophthalsäure-bis-[[benzyl-2,6-didesoxy-2-[4-[methyl-(2,3,4,5,6-penta-O-sulfo-D-glucit-1-yl)-amino]-3-nitro-benzoylamino]-3,4-di-O-sulfo-a-D-glucopyranosid-6-yl]-amid] Tetradekanatriumsalz, $[\alpha]_D^{20}$ +71,0° (*c* 0,5; Wasser), MS: *m/z* 2781,0 (rekonstruiertes M).

Beispiel 36

A. 4-Fluor-3-nitro-benzoesäure wurde mit 1-Desoxy-1-(2-hydroxy-ethylamino)-D-glucitol umgesetzt wie unter Beispiel 29.C. beschrieben und ergab 4-[(2-Acetoxy-ethyl)-(2,3,4,5,6-penta-O-acetyl-D-glucit-1-yl)-amino]-3-nitrobenzoesäure, welches roh in der nächsten Stufe weiterverarbeitet wurde.

B. Umsetzung von 4-[(2-Acetoxy-ethyl)-(2,3,4,5,6-penta-O-acetyl-D-glucit-1-yl)-amino]-3-nitro-benzoesäure wie unter Bsp. 30.A. beschrieben ergab Benzyl-2-[4-[(2-acetoxy-ethyl)-(2,3,4,5,6-penta-O-acetyl-D-glucit-1-yl)-amino]-3-nitro-benzoylamino]-2-desoxy-a-D-glucopyranosid, $[\alpha]_D^{20}$ +103,6° (*c* 0,5; Chloroform), MS: *m/z* 894,3 ([M+H]$^+$).

C. Tosylierung von Benzyl-2-[4-[(2-acetoxy-ethyl)-(2,3,4,5,6-penta-O-acetyl-D-glucit-1-yl)-amino]-3-nitro-benzoylamino]-2-desoxy-a-D-glucopyranosid wie unter Bsp. 30.B. beschrieben ergab Benzyl-2-[4-[(2-acetoxy-ethyl)-(2,3,4,5,6-penta-O-acetyl-D-glucit-1-yl)-amino]-3-nitro-benzoylamino]-2-desoxy-6-O-(p-tolylsulfonyl)-a-D-glucopyranosid, $[\alpha]_D^{20}$ +81,4° (*c* 0,5; Chloroform), MS: *m/z* 1070,9 ([M+Na]$^+$).

D. Umsetzung von Benzyl-2-[4-[(2-acetoxy-ethyl)-(2,3,4,5,6-penta-O-acetyl-D-glucit-1-yl)-amino]-3-nitro-benzoylamino]-2-desoxy-6-O-(p-tolylsulfonyl)-a-D-glucopyranosid wie unter Bsp. 30.C. beschrieben ergab Benzyl-2-[4-[(2-acetoxy-ethyl)-(2,3,4,5,6-penta-O-acetyl-D-glucit-1-yl)-amino]-3-nitro-benzoylamino]-6-azido-2-desoxy-a-D-glucopyranosid, $[\alpha]_D^{20}$ +72,8° (*c* 0,5; Chloroform), MS: *m/z* 919,4 ([M+H]$^+$).

E. Umsetzung von Benzyl-2-[4-[(2-acetoxy-ethyl)-(2,3,4,5,6-penta-O-acetyl-D-glucit-1-yl)-amino]-3-nitro-benzoylamino]-6-azido-2-desoxy-a-D-glucopyranosid wie unter Bsp. 30.D. beschrieben ergab Benzyl-2-[4-[(2-acetoxyethyl)-(2,3,4,5,6-penta-O-acetyl-D-glucit-1-yl)-amino]-3-nitro-benzoylamino]-6-amino-2-desoxy-a-D-glucopyranosid, $[\alpha]_D^{20}$ +117,2° (*c* 0,5; Dimethylsulfoxid), MS: *m/z* 893,5 ([M+H]$^+$).

F. Umsetzung von Isophthalsäure mit Benzyl-2-[4-[(2-acetoxy-ethyl)-(2,3,4,5,6-penta-O-acetyl-D-glucit-1-yl)-amino]-3-nitro-benzoylamino]-6-amino-2-desoxy-a-D-glucopyranosid wie unter Bsp. 30.E. beschrieben ergab Isophthalsäure-bis-[[benzyl-2-[4-[(2-acetoxy-ethyl)-(2,3,4,5,6-penta-O-acetyl-D-glucit-1-yl)-amino]-3-nitro-benzoylamino]-3,4-di-O-acetyl-2,6-didesoxy-a-D-glucopyranosid-6-yl]-amid], $[\alpha]_D^{20}$ +135,0° (*c* 0,5; Chloroform), MS: *m/z* 2105,6 ([M+Na]$^+$).

G. Deacetylierung von Isophthalsäure-bis-[[benzyl-2-[4-[(2-acetoxy-ethyl)-(2,3,4,5,6-penta-O-acetyl-D-glucit-1-yl)-amino]-3-nitro-benzoylamino]-3,4-di-O-acetyl-2,6-didesoxy-a-D-glucopyranosid-6-yl]-amid] wie unter Bsp. 30.F. beschrieben ergab Isophthalsäure-bis-[[benzyl-2,6-didesoxy-2-[4-[(D-glucit-1-yl)-(2-hydroxy-ethyl)-amino]-3-nitro-benzoylamino]-a-D-glucopyranosid-6-yl]-amid], $[\alpha]_D^{20}$ +96,4° (*c* 0,5; Dimethylsulfoxid), MS: *m/z* 1433,3 ([M+Na]$^+$).

H. Sulfatierung von Isophthalsäure-bis-[[benzyl-2,6-didesoxy-2-[4-[(D-glucit-1-yl)-(2-hydroxy-ethyl)-amino]-3-nitro-benzoylamino]-a-D-glucopyranosid-6-yl]-amid] wie in Bsp. 29.G. beschrieben ergab Isophthalsäure-bis-[[benzyl-2,6-didesoxy-2-[4-[(2-hydroxysulfonyloxy-ethyl)-2,3,4,5,6-penta-O-sulfo-D-glucit-1-yl)-amino]-3-nitro-benzoylamino]-3,4-di-O-sulfo-a-D-glucopyranosid-6-yl]-amid] Hexadekanatriumsalz, $[\alpha]_D^{20}$ +73,2° (*c* 0,5; Wasser), MS: *m/z* 3045,0 (rekonstruiertes M).

Beispiel 37

A. Eine Lösung von 17,1 g 4-[Methyl-(2,3,4,5,6-penta-O-acetyl-D-glucit-1-yl)-amino]-3-nitro-benzoesäure (vgl. Bsp. 35.A.) in 515 ml Ethanol wurde in Gegenwart von 10 % Palladium auf Aktivkohle während 3 Stunden bei Raumtemperatur hydriert. Das Reaktionsgemisch wurde über Filterhilfsmittel genutscht, der Rückstand wurde mit Ethanol gewaschen. Das Filtrat wurde eingeengt und mit 250 ml Essigsäureanhydrid in 500 ml Pyridin während 16 Stunden bei Raumtempereatur acetyliert. Nach Einengen im Vakuum wurde der Rückstand während 2 Stunden in 150 ml Tetrahydrofuran, 60 ml Wasser und 10 ml Pyridin gerührt. Das Reaktionsgemisch wurde erneut eingeengt. Der Rückstand wurde mit Eiswasser versetzt und mit 2 n Salzsäure angesäuert (pH = 3). Die Lösung wurde zweimal mit Essigester extrahiert. Die organische Phase wurde mit Eiswasser und gesättigter Kochsalzlösung gewaschen, über Magnesiumsulfat getrocknet und eingeengt. Der Rückstand wurde mit Methylenchlorid/ Isopropanol

über Kieselgel chromatographiert und ergab 3-Acetylamino-4-[methyl-(2,3,4,5,6-penta-O-acetyl-D-glucit-1-yl)-amino]-benzoesäure, $[\alpha]_D^{20}$ +3.4° (*c* 0,5; Chloroform), MS: *m/z* 581,3 ($[M+H]^+$).

B. Umsetzung von 3-Acetylamino-4-[methyl-(2,3,4,5,6-penta-O-acetyl-D-glucit-1-yl)-amino]-benzoesäure mit Benzyl-2-amino-2-desoxy-a-D-glucopyranosid wie unter Bsp. 30.A. beschrieben ergab Benzyl-2-[3-acetylamino-4-[methyl-(2,3,4,5,6-penta-O-acetyl-D-glucit-1-yl)-amino]-benzoylamino]-2-desoxy-a-D-glucopyranosid, $[\alpha]_D^{20}$ +48,8° (*c* 0,5; Dimethylsulfoxid), MS: *m/z* 834,4 ($[M+H]^+$).

C. Tosylierung von Benzyl-2-[3-acetylamino-4-[methyl-(2,3,4,5,6-penta-O-acetyl-D-glucit-1-yl)-amino]-benzoylamino]-2-desoxy-a-D-glucopyranosid wie unter Bsp. 30.B. beschrieben ergab Benzyl-2- [3-acetylamino-4-[methyl-(2,3,4,5,6-penta-O-acetyl-D-glucit-1-yl)-amino]-benzoylamino]-2-desoxy-6-O-(p-tolylsulfonyl)-a-D-glucopyranosid, $[\alpha]_D^{20}$ +44,4° (*c* 0,5; Dimethylsulfoxid), MS: *m/z* 1010,6 ($[M+Na]^+$).

D. Umsetzung von Benzyl-2-[3-acetylamino-4-[methyl-(2,3,4,5,6-penta-O-acetyl-D-glucit-1-yl)-amino]-benzoylamino]-2-desoxy-6-O-(p-tolylsulfonyl)-a-D-glucopyranosid wie unter Bsp. 30.C. beschrieben ergab Benzyl-2-[3-acetylamino-4-[methyl-(2,3,4,5,6-penta-O-acetyl-D-glucit-1-yl)-amino]-benzoylamino]-6-azido-2,6-didesoxy-a-D-glucopyranosid, $[\alpha]_D^{20}$ +44,0° (*c* 0,5; Dimethylsulfoxid), MS: *m/z* 881,5 ($[M+Na]^+$).

E. Umsetzung von Benzyl-2-[3-acetylamino-4-[methyl-(2,3,4,5,6-penta-O-acetyl-D-glucit-1-yl)-amino]-benzoylamino]-6-azido-2,6-didesoxy-a-D-glucopyranosid wie unter Bsp. 30.D. beschrieben ergab Benzyl-2-[3-acetylamino-4-[methyl-(2,3,4,5,6-penta-O-acetyl-D-glucit-1-yl)-amino]-benzoylamino]-6-amino-2,6-didesoxy-a-D-glucopyranosid-a-D-glucopyranosid, $[\alpha]_D^{20}$ +58,6° (*c* 0,5; Methanol), MS: *m/z* 833,5 ($[M+H]^+$).

F. Umsetzung von Isophthalsäure mit Benzyl-2-[3-acetylamino-4-[methyl-(2,3,4,5,6-penta-O-acetyl-D-glucit-1-yl)-amino]-benzoylamino]-6-amino-2,6-didesoxy-a-D-glucopyranosid-a-D-glucopyranosid wie unter Bsp. 30.E. beschrieben ergab Isophthalsäure-bis-[[benzyl-3,4-di-O-acetyl-2-[3-acetylamino-4-[methyl-(2,3,4,5,6-penta-O-acetyl-D-glucit-1-yl)-amino]-benzoylamino]-2,6-didesoxy-a-D-glucopyranosid-6-yl]-amid], $[\alpha]_D^{20}$ +70,0° (*c* 0,5; Chloroform), MS: *m/z* 1985,6 ($[M+Na]^+$).

G. Deacetylierung von Isophthalsäure-bis-[[benzyl-3,4-di-O-acetyl-2-[3-acetylamino-4-[methyl-(2,3,4,5,6-penta-O-acetyl-D-glucit-1-yl)-amino]-benzoylamino]-2,6-didesoxy-a-D-glucopyranosid-6-yl]-amid] wie unter Bsp. 30.F. beschrieben ergab Isophthalsäure-bis-[[benzyl-2-[3-acetylamino-4-[D-glucit-1-yl)-methylamino]-benzoylamino]-2,6-didesoxy-a-D-glucopyranosid-6-yl]-amid], $[\alpha]_D^{20}$ +128,8° (*c* 0,5; Dimethylsulfoxid), MS: *m/z* 1397,2 ($[M+Na]^+$).

H. Sulfatierung von Isophthalsäure-bis-[[benzyl-2-[3-acetylamino-4-[D-glucit-1-yl)-methylamino]-benzoylamino]-2,6-didesoxy-a-D-glucopyranosid-6-yl]-amid] wie in Bsp. 29.G. beschrieben ergab Isophthalsäure-bis-[[benzyl-2-[3-acetylamino-4-[methyl-(2,3,4,5,6-penta-O-sulfo-D-glucit-1-yl)-amino]-benzoylamino]-2,6-didesoxy-3,4-di-O-sulfo-a-D-glucopyranosid-6-yl]-amid] Tetradekanatriumsalz, $[\alpha]_D^{20}$ +47,0° (*c* 0,5; Wasser), MS: *m/z* 2804,0 (rekonstruiertes M).

Beispiel 38

A. Umsetzung von 4-[(2-Acetoxy-ethyl)-(2,3,4,5,6-penta-O-acetyl-D-glucit-1-yl)-amino]-3-nitro-benzoesäure (vgl. Bsp. 36.A.) wie unter Bsp. 37.A. beschrieben ergab 4-[(2-Acetoxy-ethyl)-(2,3,4,5,6-penta-O-acetyl-D-glucit-1-yl)-amino]-3-acetylamino-benzoesäure, welche roh in die nächste Stufe eingesetzt wurde.

B. Umsetzung von 4-[(2-Acetoxy-ethyl)-(2,3,4,5,6-penta-O-acetyl-D-glucit-1-yl)-amino]-3-acetylamino-benzoesäure mit Benzyl-2-amino-2-desoxy-a-D-glucopyranosid wie unter Bsp. 30.A. beschrieben ergab Benzyl-2-[4-[(2-acetoxy-ethyl)-(2,3,4,5,6-penta-O-acetyl-D-glucit-1-yl)-amino]-3-acetylamino-benzoylamino]-2-desoxy-a-D-glucopyranosid, $[\alpha]_D^{20}$ +32,8° (*c* 0,5; Dimethylsulfoxid), MS: *m/z* 906,5 ($[M+H]^+$).

C. Tosylierung von Benzyl-2-[4-[(2-acetoxy-ethyl)-(2,3,4,5,6-penta-O-acetyl-D-glucit-1-yl)-amino]-3-acetylamino-benzoylamino]-2-desoxy-a-D-glucopyranosid wie unter Bsp. 30.B. beschrieben ergab Benzyl-2-[4-[(2-acetoxy-ethyl)-(2,3,4,5,6-penta-O-acetyl-D-glucit-1-yl)-amino]-3-acetylamino-benzoylamino]-2-desoxy-6-O-(p-tolylsulfonyl)-a-D-glucopyranosid, $[\alpha]_D^{20}$ +27,2° (*c* 0,5; Dimethylsulfoxid), MS: *m/z* 1082,5 ($[M+Na]^+$).

D. Umsetzung von Benzyl-2-[4-[(2-acetoxy-ethyl)-(2,3,4,5,6-penta-O-acetyl-D-glucit-1-yl)-amino]-3-acetylamino-benzoylamino]-2-desoxy-6-O-(p-tolylsulfonyl)-a-D-glucopyranosid wie unter Bsp. 30.C. beschrieben ergab Benzyl-2-[4-[(2-acetoxy-ethyl)-(2,3,4,5,6-penta-O-acetyl-D-glucit-1-yl)-amino]-3-acetylamino-benzoylamino]-6-azido-2,6-didesoxy-a-D-glucopyranosid, $[\alpha]_D^{20}$ +24,6° (*c* 0,5; Dimethylsulfoxid), MS: *m/z* 953,6 ($[M+Na]^+$).

E. Umsetzung von Benzyl-2-[4-[(2-acetoxy-ethyl)-(2,3,4,5,6-penta-O-acetyl-D-glucit-1-yl)-amino]-3-acetylamino-benzoylamino]-6-azido-2,6-didesoxy-a-D-glucopyranosid wie unter Bsp. 30.D. beschrieben ergab Benzyl-2-[4-[(2-acetoxy-ethyl)-(2,3,4,5,6-penta-O-acetyl-D-glucit-1-yl)-amino]-3-acetylamino-benzoylamino]-6-amino-2,6-didesoxy-a-D-glucopyranosid, $[\alpha]_D^{20}$ +42,8° (*c* 0,5; Methanol), MS: *m/z* 905,6 ($[M+H]^+$).

F. Umsetzung von Isophthalsäure mit Benzyl-2-[4-[(2-acetoxy-ethyl)-(2,3,4,5,6-penta-O-acetyl-D-glutit-1-yl)-amino]-3-acetylamino-benzoylamino]-6-amino-2,6-didesoxy-a-D-glucopyranosid wie unter Bsp. 30.E. beschrieben ergab Isophthalsäure-bis-[[benzyl-2-[4-[(2-acetoxy-ethyl)-(2,3,4,5,6-penta-O-acetyl-D-glucit-1-yl)-amino]-3-acetylamino-benzoylamino]-3,4-di-O-acetyl-2,6-didesoxy-a-D-glucopyranosid-6-yl]-amid], $[\alpha]_D^{20}$ +43,6° (*c* 0,4; Chloroform), MS: *m/z* 2129,3 ($[M+Na]^+$).

G. Deacetylierung von Isophthalsäure-bis-[[benzyl-2-[4-[(2-acetoxy-ethyl)-(2,3,4,5,6-penta-O-acetyl-D-glucit-1-yl)-amino]-3-acetylamino-benzoylamino]-3,4-di-O-acetyl-2,6-didesoxy-a-D-glucopyranosid-6-yl]-amid] wie unter Bsp. 30.F. beschrieben ergab Isophthalsäure-bis-[[benzyl-2-[3-acetylamino-4-[D-glucit-1-yl-(2-hydroxy-ethyl)-amino]-benzoylamino]-2,6-didesoxy-a-D-glucopyranosid-6-yl]-amid], $[\alpha]_D^{20}$ +88,4° (*c* 0,5; Dimethylsulfoxid), MS: *m/z* 1457,6 ($[M+Na]^+$).

H. Sulfatierung von Isophthalsäure-bis-[[benzyl-2-[3-acetylamino-4-[D-glucit-1-yl-(2-hydroxy-ethyl)-amino]-benzoylamino]-2,6-didesoxy-a-D-glucopyranosid-6-yl]-amid] wie in Bsp. 29.G. beschrieben ergab Isophthalsäure-bis-[[benzyl-2-[3-acetylamino-4-[(2,3,4,5,6-penta-O-sulfo-D-glucit-1-yl)-(2-sulfonyloxy-ethyl)-amino]-benzoylamino]-2,6-didesoxy-3,4-di-O-sulfo-a-D-glucopyranosid-6-yl]-amid] Hexadekanatriumsalz, $[\alpha]_D^{20}$ +48,8° (*c* 0,5; Wasser), MS: *m/z* 3068,2 (rekonstruiertes M).

Beispiel 39

A. Umsetzung von Isophthalsäuremonomethylester mit Benzyl-6-amino-2-benzyloxycarbonylamino-2,6-didesoxy-a-D-glucopyranosid (vgl. Bsp. 9.C.) wie unter Bsp. 30.A. beschrieben ergab N-(Benzyl-2-benzyloxycarbonylamino-2,6-didesoxy-a-D-glucopyranosid-6-yl)-isophthalamsäuremethylester, $[\alpha]_D^{20}$ +81,4° (*c* 0,5; Dimethylsulfoxid), MS: *m/z* 565,7 ($[M+H]^+$).

B. Eine Lösung von 3,8 g N-(Benzyl-2-benzyloxycarbonylamino-2,6-didesoxy-a-D-glucopyranosid-6-yl)-isophthalamsäuremethylester in 80 ml Methanol wurde mit 6,6 ml 2 m Natriumhydroxidlösung versetzt, 2,5 Stunden unter Rückfluss erhitzt und dann eingeengt. Der Rückstand wurde in 200 ml warmen Wasser aufgenommen und mit 10 ml 2 n Salzsäure angesäuert. Der farblose Niederschlag wurde abgenutscht, mit Wasser nachgewaschen, im Trokkenschrank bei 70°C getrocknet und ergab N-(Benzyl-2-benzyloxy-carbonylamino-2,6-didesoxy-a-D-glucopyranosid-6-yl)-isophthalamsäure, $[\alpha]_D^{20}$ +87,6° (*c* 0,5; Dimethylsulfoxid), MS: *m/z* 549,5 ($[M-H]^-$).

C. N-(Benzyl-2-benzyloxycarbonylamino-2,6-didesoxy-a-D-glucopyranosid-6-yl)-isophthalamsäure wurde mit 2,3,4,5,6-Penta-O-acetyl-D-gluconsäure-(benzyl-3,4-di-O-acetyl-2-amino-2,6-didesoxy-a-D-glucopyranosid-6-yl)-amid (vgl. Bsp. 14.C.) umgesetzt wie unter Bsp. 30.A. beschrieben. Nachacetylierung wie z. B. unter Bsp. 14.B. beschrieben ergab N-(Benzyl-3,4-di-O-acetyl-2-benzyloxycarbonylamino-2,6-didesoxy-a-D-glucopyranosid-6-yl)-N'-[(benzyl-3,4-di-O-acetyl-2,6-didesoxy-6-(2,3,4,5,6-penta-O-acetyl-D- gluconylamino)-a-D-glucopyranosid-2-yl]-isophthalamid, $[\alpha]_D^{20}$ +93,0° (*c* 0,5; Chloroform), MS: *m/z* 1379,3 ($[M+Na]^+$).

D. Hydrierung von N-(Benzyl-3,4-di-O-acetyl-2-benzyloxycarbonylamino-2,6-didesoxy-a-D-glucopyranosid-6-yl)-N'-[(benzyl-3,4-di-O-acetyl-2,6-didesoxy-6-(2,3,4,5,6-penta-O-acetyl-D-gluconylamino)-a-D-glucopyranosid-2-yl]-isophthalamid wie unter Bsp. 13.B. beschrieben ergab N-(Benzyl-3,4-di-O-acetyl-2-amino-2,6-didesoxy-a-D-glucopyranosid-6-yl)-N'-[(benzyl-3,4-di-O-acetyl-2,6-didesoxy-6-(2,3,4,5,6-penta-O-acetyl-D-gluconylamino)-a-D-glucopyranosid-2-yl]-isophthalamid, $[\alpha]_D^{20}$ +87,6° (*c* 0,25; Dimethylsulfoxid), MS: *m/z* 1246,9 ($[M+Na]^+$).

E. Umsetzung von 4-(2,3,4,5,6-Penta-O-acetyl-D-glucit-1-ylamino)-3-nitro-benzoesäure (vgl. Bsp. 29.C.) mit N-(Benzyl-3,4-di-O-acetyl-2-amino-2,6-didesoxy-a-D-glucopyranosid-6-yl)-N'-[(benzyl-3,4-di-O-acetyl-2,6-didesoxy-6-(2,3,4,5,6-penta-O-acetyl-D-gluconylamino)-a-D-glucopyranosid-2-yl]-isophthalamid wie unter Bsp. 30.A. beschrieben ergab N-[Benzyl-3,4-di-O-acetyl-2,6-didesoxy-2-[4-(2,3,4,5,6-penta-O-acetyl-D-glucit-1-ylamino)-3-nitro-benzoylamino]-a-D-glucopyranosid-6-yl]-N'-[(benzyl-3,4-di-O-acetyl-2,6-didesoxy-6-(2,3,4,5,6-penta-O-acetyl-D-gluconylarnino)-a-D-glucopyranosid-2-yl]-isophthalamid, $[\alpha]_D^{20}$ +81,4° (*c* 0,5; Dimethylsulfoxid), MS: *m/z* 565,7 ($[M+H]^+$).

F. Deacetylierung von N-[Benzyl-3,4-di-O-acetyl-2,6-didesoxy-2-[4-(2,3,4,5,6-penta-O-acetyl-D-glucit-1-ylamino)-3-nitro-benzoylaminol-a-D-glucopyranosid-6-yl]-N'-[(benzyl-3,4-di-O-acetyl-2,6-didesoxy-6-(2,3,4,5,6-penta-O-acetyl-D-gluconylamino)-a-D-glucopyranosid-2-yl]-isophthalamid wie unter Bsp. 30.F. beschrieben ergab N-[Benzyl-2,6-didesoxy-2-(4-D-glucit-1-ylamino)-3-nitro-benzoylamino)-a-D-glucopyranosid-6-yl]-N'-(benzyl-2,6-didesoxy-6-D-gluconylamino-a-D-glucopyranosid-2-yl)-isophthalamid, $[\alpha]_D^{20}$ +86,8° (*c* 0,5; Dimethylsulfoxid), MS: *m/z* 1195,9 ($[M+Na]^+$).

G. Sulfatierung von N-[Benzyl-2,6-didesoxy-2-(4-D-glucit-1-ylamino)-3-nitro-benzoylamino)-a-D-glucopyranosid-6-yl]-N'-(benzyl-2,6-didesoxy-6-D-gluconylamino-a-D-glucopyranosid-2-yl)-isophthalamid wie in Bsp. 29.G. beschrieben ergab N-[Benzyl-2,6-didesoxy-2-[4-(2,3,4,5,6-penta-O-sulfo-D-glucit-1-ylamino)-3-nitro-benzoylamino]-3,4-di-O-sulfo-a-D-glucopyranosid-6-yl]-N'-[(benzyl-2,6-didesoxy-6-(2,3,4,5,6-penta-O-sulfo-D- gluconylamino)-3,4-di-O-sulfo-a-D-glucopyranosid-2-yl]-isophthalamid Tetradekanatriumsalz, $[\alpha]_D^{20}$ +48,8° (*c* 0,5; Wasser), MS: *m/z* 3068,2 (rekonstruiertes M).

Beispiel A

| Tablette: | | |
|---|---|---|
| 1 | Verbindung der Formel Ia - Ic | 500 mg |
| 2 | Laktose, wasserfrei | 150 mg |
| 3 | Mikrokristalline Cellulose | 150 mg |
| 4 | Polyvinylpyrrolidon | 40 mg |
| 5 | Talk | 50 mg |
| 6 | Magnesiumstearat | 10 mg |
| | Tablettengewicht | 900 mg |

Die Bestandteile 1-4 werden gesiebt und gemischt. Diese Mischung wird mit demineralisiertem Wasser granuliert und das getrocknete Granulat mit den Bestandteilen 5 und 6 vermischt. Man verpresst zu Tabletten geeigneter Form.

Beispiel B

| Pellets: | | |
|---|---|---|
| 1 | Verbindung der Formel Ia - Ic | 500 mg |
| 2 | Mikrokristalline Cellulose | 200 mg |
| 3 | PRIMOJEL | 70 mg |
| 4 | Aromapulver | 10 mg |
| 5 | Talk | 20 mg |

Die gemischten und gesiebten Bestandteile 1-3 werden mit demineralisiertem Wasser ausreichend befeuchtet und mittels Extruder durch eine geeignete Lochscheibe gepresst. Das Extrudat wird auf einen Pelletierteller überführt, zu Kügelchen ausgerundet und anschliessend getrocknet. Man versetzt mit den gesiebten Bestandteilen 4 und 5 und füllt in Papiersachets (oder ähnliches) ab.

Beispiel C

Injektionslösung:

Zur Herstellung einer Injektionslösung werden 50 mg Verbindung der Formel Ia - Ic sowie 0,5 mg Tris-Puffer in Wasser für Injektionszwecke ad 1 ml gelöst und der pH-Wert auf 7,4 eingestellt. Die Lösung wird sterilfiltriert und nach Abfüllen in Ampullen autoklaviert.

**Patentansprüche**

1. Schwefelsäureester von Aminozuckern der allgemeinen Formel Ia, Ib, Ic

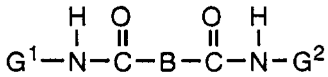

Ia

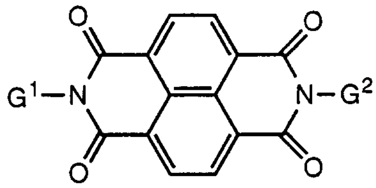

Ib

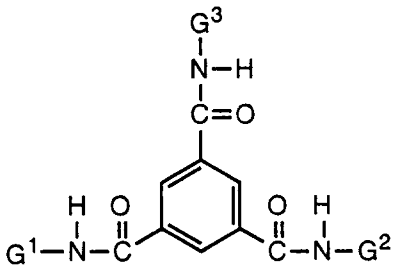

Ic

worin

B niederes Alkylen oder ein gegebenenfalls substituiertes aromatisches Ringsystem;
$G^1$, $G^2$ und $G^3$ unabhängig voneinander je einen Rest eines Glycopyranosids, einer Glycopyranose oder eines Derivates davon, wobei mindestens eine Hydroxygruppe der Reste $G^1$, $G^2$ oder $G^3$ mit Schwefelsäure verestert ist, bedeuten und pharmazeutisch anwendbare Salze davon.

2. Verbindungen gemäss Anspruch 1, worin $G^1$, $G^2$ und, falls vorhanden, $G^3$ identische Reste darstellen.

3. Verbindungen gemäss Anspruch 1 oder 2, worin das aromatische Ringsystem in Formel Ia Phenylen, Naphthylen, oder einen Rest der Formel

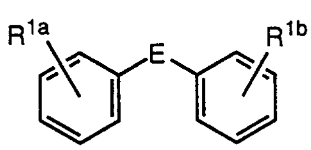

worin

E eine Kohlenstoff-Kohlenstoff-Bindung, -O-,-CO, $-CH_2-$, $-CH_2CH_2-$, -CH=CH-, -C≡C- und $R^{1a}$ und $R^{1b}$ Wasserstoff oder Halogen bedeuten.

4. Verbindungen gemäss einem der Ansprüche 1-3, worin die Reste $G^1$, $G^2$ und, falls vorhanden $G^3$ unabhängig voneinander je einen Rest der Formel a-f

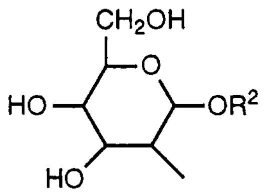 a)

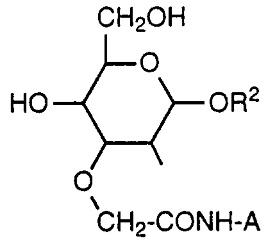 b)

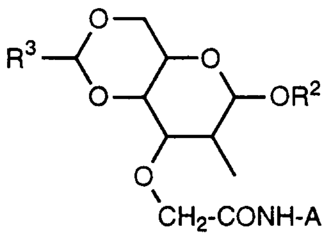 c)

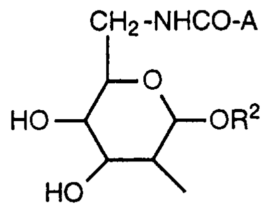 d)

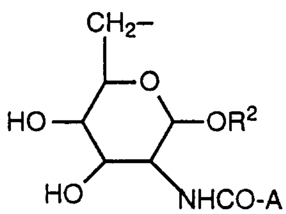 e)

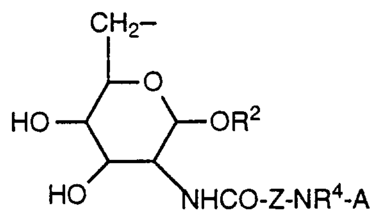 f)

wobei mindestens je eine Hydroxygruppe der Glycopyranose - oder Glycopyranosidreste a-f mit Schwefelsäure verestert ist, und
worin

$R^2$ Wasserstoff, niederes Alkyl oder Benzyl;
$R^3$ Wasserstoff, niederes Alkyl oder Phenyl;
$R^4$ Wasserstoff oder gegebenenfalls substituiertes niederes Alkyl;
Z einen gegebenenfalls substituierten Phenylenrest;
A den um die 1-Hydroxygruppe verminderten Rest eines Zuckeralkohols oder eines Derivats davon, den tris-(Hydroxymethyl)-methylrest, einen Glycopyranosid- oder Glycopyranose-Rest der oben angegebenen Formel a oder einen Rest der Formel g oder h bedeuten,

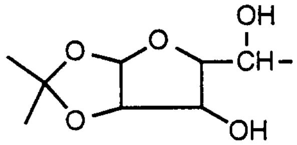 g)

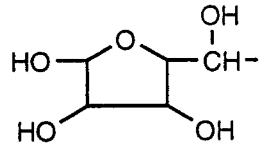 h)

wobei mindestens je eine Hydroxygruppe des Restes A mit Schwefelsäure verestert ist.

**5.** (Biphenyl-4,4'-dicarbonsäure)-bis-[[benzyl-3-O-[(benzyl-2-desoxy-2,3,4-tri-O-sulfo-a-D-glucopyranosid)-2-ylcarbamoylmethyl]-4,6-(R)-O-benzyliden-2-desoxy-a-D-glucopyranosid-2-yl]-amid] Hexanatriumsalz,
Biphenyl-4,4'-dicarbonsäure-bis-[[benzyl-2,6-didesoxy-3,4-di-O-sulfo-6-(2,3,4,5,6-penta-O-sulfo-D-gluconoylamino)-a-D-glucopyranosid-2-yl]-amid] Tetradekanatriumsalz,

Biphenyl-4,4'-dicarbonsäure-bis-[[(Z)-benzyl-2,6-didesoxy-3,4-di-O-sulfo-6-(3-desoxy-2,4,5,6-tetra-O-sulfo-D-erythro-hex-2-enonoylamino)-a-D-glucopyranosid-2-yl]-amid] Dodekanatriumsalz,

(Z)-Stilben-4,4'-dicarbonsäure-bis-[[benzyl-2,6-didesoxy-6-(2,3,4,5,6-penta-O-sulfo-D-gluconoylamino)-3,4-di-O-sulfo-a-D-glucopyranosid-2-yl]-amid] Tetradekanatriumsalz,

Isophthalsäure-bis-[[benzyl-2,6-didesoxy-6-(2,3,4,5,6-penta-O-sulfo-D-gluconoylamino)-3,4-di-O-sulfo-a-D-glucopyranosid-2-yl]-amid] Tetradekanatriumsalz,

Benzol-1,3,5-tricarbonsäure-tris-[[benzyl-2,6-didesoxy-2-[4-(2,3,4,5,6-penta-O-sulfo-D-glucit-1-ylamino)-3-nitro-benzoylamino]-3,4-di-O-sulfo-a-D-glucopyranosid-6-yl]-amid] Henikosanatriumsalz,

(E)-2-Chlor-stilben-4,4'-dicarbonsäure-bis-[[benzyl-2,6-didesoxy-2-[4-(2,3,4,5,6-penta-O-sulfo-D-glucit-1-ylamino)-3-nitro-benzoylamino]-3,4-di-O-sulfo-a-D-glucopyranosid-6-yl]-amid] Tetradekanatriumsalz,

Isophthalsäure-bis-[[benzyl-2,6-didesoxy-2-[4-(2,3,4,5,6-penta-O-sulfo-D-glucit-1-ylamino)-3-nitro-benzoylamino]-3,4-di-O-sulfo-a-D-glucopyranosid-6-yl]-amid] Tetradekanatriumsalz,

2,7-Bis-[benzyl-2,6-didesoxy-2-[3-nitro-4-(2,3,4,5,6-penta-O-sulfo-D-glucit-1-ylamino)-benzoylamino]-3,4-di-O-sulfo-a-D-glucopyranosid-6-yl]-1,2,3,6,7,8-hexahydro-benzo[lmn][3,8]phenanthrolin-1,3,6,8-tetraon Tetradekanatriumsalz,

Isophthalsäure-bis-[[benzyl-2,6-didesoxy-2-[4-[methyl-(2,3,4,5,6-penta-O-sulfo-D-glucit-1-yl)-amino]-3-nitro-benzoylamino]-3,4-di-O-sulfo-a-D-glucopyranosid-6-yl]-amid] Tetradekanatriumsalz,

Isophthalsäure-bis-[[benzyl-2,6-didesoxy-2-[4-[(2-hydroxysulfonyloxy-ethyl)-2,3,4,5,6-penta-O-sulfo-D-glucit-1-yl)-amino]-3-nitro-benzoylamino]-3,4-di-O-sulfo-a-D-glucopyranosid-6-yl]-amid] Hexadekanatriumsalz,

Isophthalsäure-bis-[[benzyl-2-[3-acetylamino-4-[methyl-(2,3,4,5,6-penta-O-sulfo-D-glucit-1-yl)-amino]-benzoylamino]-2,6-didesoxy-3,4-di-O-sulfo-a-D-glucopyranosid-6-yl]-amid] Tetradekanatriumsalz,

Isophthalsäure-bis-[[benzyl-2-[3-acetylamino-4-[(2,3,4,5,6-penta-O-sulfo-D-glucit-1-yl)-(2-sulfonyloxy-ethyl)-amino]-benzoylamino]-2,6-didesoxy-3,4-di-O-sulfo-a-D-giucopyranosid-6-yl]-amid] Hexadekanatriumsalz.

**6.** Arzneimittel, insbesondere zur Behandlung und/oder Prophylaxe von Krankheiten, die durch exzessive oder destruktive Proliferation von glatten Muskelzellen gekennzeichnet sind, sowie von arteriosklerotischen Gefässwandveränderungen, enthaltend eine Verbindung nach einem der Ansprüche 1-5 oder ein pharmazeutisch anwendbares Salz davon und einen therapeutisch inerten Träger.

**7.** Verfahren zur Herstellung von Verbindungen nach einem der Ansprüche 1-5, dadurch gekennzeichnet, dass man eine entsprechende, nicht sulfatierte Verbindung mit einem Sulfatierungsmittel umsetzt.

**8.** Verbindungen nach einem der Ansprüche 1-5 zur Anwendung als therapeutische Wirkstoffe, insbesondere zur Behandlung und/oder Prophylaxe von Krankheiten, die durch exzessive oder destruktive Proliferation von glatten Muskelzellen gekennzeichnet sind, sowie von arteriosklerotischen Gefässwandveränderungen.

**9.** Verwendung von Verbindungen nach einem der Ansprüche 1-5 oder von Salzen davon als Arzneimittel insbesondere zur Behandlung und/oder Prophylaxe von Krankheiten, die durch exzessive oder destruktive Proliferation von glatten Muskelzellen gekennzeichnet sind, sowie von arteriosklerotischen Gefässwandveränderungen bzw. zur Herstellung entsprechender Arzneimittel.

Europäisches Patentamt

# EUROPÄISCHER RECHERCHENBERICHT

Nummer der Anmeldung
EP 96 10 6537

| Kategorie | EINSCHLÄGIGE DOKUMENTE<br>Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | US 4 120 954 A (JOSEPH J.P. UND BERNSTIEN S.)<br>* das ganze Dokument *<br>--- | 1-4,6-9 | C07H13/08<br>C07H13/10<br>C07H15/18<br>A61K31/70 |
| A | US 4 431 637 A (UPESLACIS J. ET AL)<br>* das ganze Dokument *<br>--- | 1-9 | |
| A | EP 0 504 645 A (F. HOFFMANN-LA ROCHE AG)<br>* Seite 3 *<br>----- | 1,6,8,9 | |
| | | | RECHERCHIERTE SACHGEBIETE (Int.Cl.6) |
| | | | C07H<br>A61K |

Der vorliegende Recherchenbericht wurde für alle Patentansprüche erstellt

| Recherchenort | Abschlußdatum der Recherche | Prüfer |
|---|---|---|
| DEN HAAG | 29.August 1996 | Day, G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)